# EUROPEAN PATENT APPLICATION

(11) **EP 2 366 698 A1**
(43) Date of publication of application: **21.09.2011**
(21) Application number: 11170047.2
(22) Date of filing: 10.07.2007
(51) Int. Cl.: C07D 277/48, C07D 277/82, C07D 417/12, A61K 31/17, A61K 31/426, A61K 31/428, C07C 275/18, C07C 275/28

(54) **Derivatives of urea and related diamines, methods for their manufacture, and uses therefor**

(30) Priority: 10.07.2006 GB 0613674
(62) Divisional of application: 07765216.2
(71) Applicant: Galapagos SAS, 93230 Romainville (FR)
(72) Inventor: Deprez, Pierre, F-93230 Romainville (FR); Lively, Sarah, San Carlos, CA California 94070 (US); Temal-Laib, Taoues, F-93230 Romainville (FR)
(74) Representative: Lord, Hilton David

(57) **Abstract**

Compounds of formula (I): Z is >CH-, >C=CH- or >N-, R¹ and R² are optionally substituted and are the same or different, and each represents an aryl group, a heteroaryl group, or Z, R¹ and R² form a fused ring structure of formula: in which A represents a single bond, or linking group, R³ represents hydrogen, a group selected from: -AlkCOOR, -AlkNR⁷R⁸, -AlkCONR⁷R⁸, -AlkCOR⁹, -AlkSO₂NR¹⁰R^{10'}, -AlkOR1O, and -AlkS(O)ₙR¹⁰, in which Alk is alkyl, R is hydrogen or alkyl, R⁷ and R⁸ each represents H, optionally substituted alkyl, alkylaminoalkyl, or dialkylaminoalkyl, or CONR⁷R⁸ forms an optionally substituted heterocycle, and R'° and R^{10'} are hydrogen atom, optionally substituted alkyl, an alkylaminoalkyl group, an optionally substituted cycle, optionally spaced by an Alk group as defined, Q represents >C=O or >C=S, R⁵ is a hydrogen atom or an alkyl, alkoxy, hydroxyalkyl, alkylthio, or thioalkyl group, p is 1, 2 or 3, q is 0, 1 or 2, R⁶ represents an optionally substituted aryl or heteroaryl group, and salts and esters thereof, are useful as calcimimetics in therapy.

## Description

The present invention relates to urea derivatives useful in the physiological modulation of the activity of inorganic ions, particularly through their effect on inorganic ion receptors and especially on membrane calcium receptors capable of binding extracellular calcium; to processes for the preparation thereof; to their use as medicaments; to pharmaceutical compositions containing them; and to the their uses.

Extracellular calcium concentration is precisely regulated in the organism and one of the key elements of this regulation is the calcium receptor known as the Ca sensing receptor or CaSR. A receptor of this type at the surface of specific cells can detect the presence of calcium. Specific cells of the organism respond not only to chemical signals, but also to ions such as extracellular calcium ions (Ca ⁺⁺): changes in the concentration of these extracellular Ca ++ ions can modify the functional responses of these cells. These cells include parathyroid cells which secrete the parathyroid hormone known as PTH. Parathyroid cells thus have at their surface the calcium receptor (CaSR), which detects changes in extracellular calcium concentration, and initiates the functional response of this cell, which is a modulation of the secretion of the parathyroid hormone (PTH), PTH, by acting in particular on the bone cells or on the renal cells, increases the calcium level in the blood. This increase then acts as a negative control on PTH secretion. The reciprocal relationship between calcium concentration and PTH level is an essential mechanism for calcium homeostasis maintenance.

The cloning of the calcium receptor by Brown in 199a consequently demonstrated two possible signalling pathways for this G protein coupled receptor: one pathway by activation of the Gi protein (sensitive to the pertussis toxin) which stimulates phospholipase C and inhibits adenylate cyclase; the other pathway by activating the Gq protein responsible for mobilising intracellular calcium. These two signalling pathways, either independently of one another or together, can be activated so as to trigger the associated biological effect.

On its extracellular portion, the calcium receptor is a low affinity receptor which is stimulated by millimolar concentrations of agonists, in particular the calcium ion Ca²⁺. In addition, this receptor can also be activated by some divalent metals (magnesium) or trivalent metals (gadolinium, lanthanum, etc.) or else by polycationic compounds such as neomycin or spermin.

Novel compounds acting on the transmembrane portion of the receptor have been identified by Edward F. Nemeth *et al* (company NPS, USP 6,211,Z44, EP-787 122, WO 060310D3) and allow the calcium receptor to be modulated allosterically. The action of first generation and second generation compounds on the pharmacological regulation of parathyroid hormone (PTH) secretion is described, for example, by E. F. Nemeth in Current Pharmaceutical Design, 2002, 8, 2077-2087. In particular, the compound AMG073 (cinacalcet, Sensipar®, Mimpara ®) acts as an agonist of the calcium receptor and is sold for the treatment of secondary hyperparathyroidism (Idrugs, 2003, 6, 587-592 J. Iqbal, M. Zaidi, A. E. Schneider).

In the present invention, the compounds can have, in particular, an effect on PTH secretion which, without being bound by theory, is likely to result from the activation of the beta-gamma subunits of the G proteins, whether they are specifically Gi (similarly to the trivalent cation) or simultaneously Gi and Gq.

Thus, in a first aspect, the present invention provides use of a compound of formula (I): 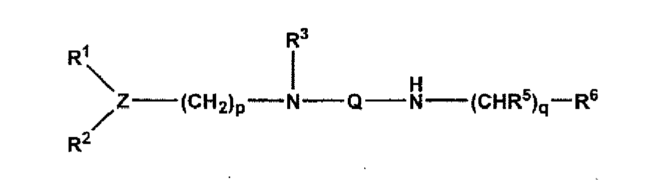 wherein:
- Z is >CH-, >C=CH- or >N-,

R¹ and R² are the same or different, and each represents an aryl group, a heteroaryl group, or Z, R¹ and R² form a fused ring structure of Formula: 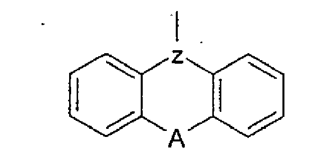 in which A represents a single bond, a methylene group, a dimethylene group, oxygen, nitrogen or sulphur, said sulphur optionally being in the sulphoxide or sulphone forms,
wherein each of R¹ and R², or said fused ring structure formed thereby, is optionally substituted by at least one substituent selected from the group c
wherein the group c consists of: halogen atoms, hydroxyl, carboxyl, linear and branched alkyl, hydroxyalkyl, haloalkyl, alkylthio, alkenyl, and alkynyl groups; linear and branched alkoxyl groups; linear and branched thioalkyl groups; hydroxycarbonylalkyl; alkylcarbonyl; alkoxycarhonylalkyl;
alkoxycarbonyl; trifluoromethyl; trifluoromethoxyl; -CN; -NO₂;
sulphonamido groups; alkylsulphonyl groups optionally in the sulphoxide or sulphone forms; wherein any alkyl component has from 1 to 6 carbon atoms, and any alkenyl or alkynyl components have from 2 to 6 carbon atoms,
and wherein, when there is more than one substituent, then each said substituent is the same or different,
RT represents hydrogen, a group selected from: -AlkCOOR, -AlkNR⁷R⁸, -AlkcONR⁷R⁸, -AlkCOR⁹, -AlkSO₂NR¹⁰R^{10'}, -AlkOR"', and -AlkS(O)ₙR¹⁰, wherein Alk is a straight or branched chain C₁₋₆ alkylene group,
n is 0, 1 or 2,
R is H or a straight or branched chain C₁₋₆ alkyl group,
RT is a linear or branched C₁₋₆ alkyl group and is optionally substituted by at least one of a phenyl group, a halogen atom, a hydroxyl group, or a C₁₋₆ alkoxy group; an alkylaminoalkyl or dialkylaminoalkyl group wherein each alkyl group contains from 1 to 6 carbon atoms; a saturated or unsaturated cycle containing 0, 1, 2, or 3 heteroatoms and having 5, b, or 7 ring atoms, said cycle being optionally substituted by at least one substituent selected from the group `b' defined below,
R¹⁰ and R^{10'} are independently a hydrogen atom, a linear or branched C₁₋₆ alkyl group optionally substituted by at least one of a phenyl group, a halogen atom, a hydroxyl group, a carboxyl group, an alkoxycarbonyl group, or a C₁₋₆ alkoxy group; an alkylaminoalkyl or dialkylaminoalkyl group wherein each alkyl group contains from 1 to 6 carbon atoms; an aminocarbonyl group; a saturated or unsaturated cycle, optionally spaced from the S or O to which the cycle is linked by an Alk group as defined, and containing 0, 1, 2, or 3 heteroatoms and having 5, 6, or 7 ring atoms, or 3-7 ring atoms when the cycle is a carbocycle, said cycle being optionally substituted by at least one substituent selected from the group '*b*' defined below,
R⁷ and R⁸, which may be the same or different, each represents: a hydrogen atom; an alkylsulphonyl group; alkylamino- or dialkylamino- sulphonyl; a linear or branched alkyl group containing from 1 to 6 carbon atoms and optionally substituted by at least one of a phenyl group, a halogen atom, a hydroxyl group, a carboxyl group, an alkoxycarbonyl group, or an alkoxy group containing from 1 to 6 carbon atoms; an alkylaminoalkyl or
- dialkylaminoalkyl group wherein each alkyl group contains from 1 to 6 carbon atoms; an amino group; an aminocarbonyl group; an alkylamino group; a saturated or unsaturated cycle, optionally spaced from the N to which the cycle is linked by an -SO₂- group, -C(O)-group, -C(O)O- group, -C(O)NH- group or an Alk group as defined, and containing 0, 1, 2, or 3 heteroatoms and having 5, 6, or 7 ring atoms, or 3-7 ring atoms when the cycle is a carbocycle, said cycle being optionally substituted by at least one substituent selected from the group '*b*'defined below,
or R⁷ and R⁸, in the group -AlkCONR⁷R⁸, together with the nitrogen atom to which they are linked, form a saturated or unsaturated heterocycle containing 0, 1 or 2 additional heteroatoms and having 5, 6, or 7 ring atoms, said heterocycle being optionally substituted by at least one substituent selected from the group 'b' defined below,
and wherein, when there is more than one substituent, said substituent is the same or different,
Q represents >C=O or >C=S,
R⁵ represents a hydrogen atom or an alkyl, alkoxy, hydroxyalkyl, alkylthio, or thioalkyl group wherein any alkyl part contains from 1 to 4 carbon atoms,
p is 1, 2 or 3,
q is 0, 1 or 2,
R⁶ represents an aryl or heteroaryl ring, two linked rings each being selected from aryl or heteroaryl rings, or a fused double or triple ring system comprising at least two rings each being selected from aryl or heteroaryl rings, and wherein said ring or rings forming R⁶ are optionally substituted by at least one substituent selected from the group a, wherein the group a consists of: halogen atoms; hydroxyl; carboxyl; aldehyde groups; aryl groups; linear and branched alkyl, alkenyl, alkynyl, hydroxyalkyl, hydroxyalkenyl, hydroxyalkynyl, haloalkyl, haloalkenyl, and haloalkynyl groups; linear and branched alkoxyl groups; linear and branched thioalkyl groups; heteroaryl groups; saturated or unsaturated heterocycyl groups; aralkoxy groups; aryloxy groups; alkoxycarbonyl; aralkoxycarbonyl; aryloxycarbonyl; heteroaralkoxy groups; heteroaryloxy groups; heteroaralkoxycarbonyl; heteroaryloxycarbonyl; hydroxycarbonylalkyl; alkoxycarbonylalkyl; aralkoxycarbonylalkyl; aryloxycarbonylalkyl; heteroaralkoxycarbonylalkyl; heteroaryloxycarbonylalkyl; perfluoroalkyl; perfluoroalkoxy; -CN; -NO₂; acyl; amino, alkylamino, aralkylamino, arylamino, dialkylamino, diaralkylamino, diarylamino, heteroaralkylamino, heteroarylamino, diheteroaralkylamino, diheteroarylamino, alkylsulphonylamino, haloalkylsulphonylamino, acylamino, and diacylamino groups; alkoxycarbonylamino, aralkoxycarbonylamino, aryloxycarbonylamino, heteroaralkoxycarbonylamino, heteroaryloxycarbonylamino, alkylcarbonylamino, heteroaralkylcarbonylamino, heteroarylcarbonylamino, alkylaminocarbonyloxy, aralkylaminocarbonyloxy, and arylaminocarbonyloxy groups; alkyl groups substituted with an amino, alkylamino, aminoalkylamino, alkylaminoalkylamino, aralkylamino, arytamino, aryloxy, arylthio, heteroaralkylamino, heteroarylamino, heteroaryloxy, heteroarylthio, heterocycyloxy, heterocycylthio, dialkylamino, diaralkylamino, diarylamino, diheteroaralkylamino, diheteroarylamino, acylamino, trifluoromethylcarbonylamino, fluoroalkyl-carbonylamino, diacylamino group; a carbamoyl group optionally substituted by an alkyl, alkylsulphonamide, sulphonamide, alkylsulphonyl, sulphonyl, aminoalkyl, or alkylaminoalkyl group; a sulphonamide group optionally substituted by an alkyl, acyl, alkoxycarbonyl, carbamoyl, alkylcarbamoyl, or carbamoyl further substituted by a carboxylic acid, aminoalkyl, or alkylaminoalkyl group; alkyl-, aralkyl-, aryl- heteroaralkyl-, and heteroaryl- amido groups; alkylthio, arylthio, aralkylthio, heteroarylthio and hetetoaralkylthio and the oxidised sulphoxide and sulphone forms thereof; sulphonyl, alkylsulphonyl, haloalkylsulphonyl, arylsulphonyl, aralkylsulphonyl, and heteroaralkylsulphonyl groups; alkylsulphonaznide, haloalkylsulphonamide, di{alkylsulphonyl)amino, aralkylsulphonamide, di{aralkylsulphonyl)amino, arylsulphonamide, di(arylsulphonyl)amino, heteroaralkylsulphonamide, di(heteroaralkylsulphonyl)amino, heteroarylsulphonamide, and di(heteroarylsulphonyl)amino; and saturated and unsaturated heterocyclyl groups, said aryl, heteroaryl and heterocyclyl groups being mono- or bi- cyclic and being optionally substituted by one or more substituents, which may be the same or different, selected from the group b,
wherein the group b consists of: keto when substituting a saturated or partially unsaturated heterocycle, halogen atoms; hydroxyl; carboxyl;
aldehyde groups; linear and branched alkyl, alkenyl, alkynyl, hydroxyalkyl, hydroxyalkenyl, hydroxyalkynyl, haloalkyl, haloalkenyl, and haloalkynyl groups; linear and branched alkoxyl groups; linear and branched thioalkyl groups; alkoxycarbonyl; hydroxycarbonylalkyl; alkoxycarbonylalkyl;
perfluoroalkyl; perfluoroalkoxy; -CN; acyl; amino, alkylamino, dialkylamino, acylamino, and diacylamino groups; alkyl groups substituted with an amino, alkylamino, dialkylamino, acylamino, or diacylamino group; CONH₂; alkylamido groups; alkylthio and the oxidised sulphoxide and sulphone forms thereof; sulphonyl, alkylsulphonyl groups; and sulphonamide, alkylsulphonamide, and di(alkylsulphonyl)amino groups wherein two groups a, where present, optionally form a fused carbocycle or heterocycle with the ring on which they are located, and are optionally substituted with a keto or a substituent selected from group b, as defined, wherein, in groups a and b, any alkyl components contain from 1 to 6 carbon atoms, and any alkenyl or alkynyl components contain from 2 to 6 carbon atoms, and are optionally substituted by at least one halogen atom or hydroxyl group, and
wherein any aryl component is optionally a heteroaryl group,
and salts and esters thereof,
in therapy.

Compounds of the invention are particularly useful as calcimimetics in therapy.

Where compounds of the invention are referred to herein, this refers equally to the novel compounds of the invention as well as to those compounds which may have been previously disclosed, but either not as therapeutic agents or not for use as a calcimimetic.

In preferred compounds of the invention, R¹ and R² each represents a monocyclic aryl group, a monocyclic heteroaryl group, or Z, R¹ and R² together form said fused ring stricture, wherein each of R¹ and R², or said fused ring structure formed thereby, is optionally substituted by at least one substituent selected from the group 'c' as defined. Preferably, R¹ and R² each represent a phenyl, pyridinyl, or thienyl radical, or said fused ring structure formed thereby, is optionally substituted as defined.

Preferred substituents of R¹ and R², or said fused ring structure formed thereby, are selected from the group *c*', consisting of: fluorine and chlorine atoms, hydroxyl, linear and branched alkyl, alkylthio, hydroxyalkyl, and fluoroalkyl groups; linear and branched alkoxyl groups; trifluoromethyl; trifluoromethoxyl; -CN; alkylcarbonyl groups; alkylsulphonyl groups, and any alkyl component has from 1 to 4 carbon atoms,
and wherein, when there is more than one substituent, then each said substituent is the same or different.

More preferably, the substituents on R¹ and R², are selected from the group consisting of: fluorine and chlorine atoms, hydroxyl groups, linear or branched alkoxy groups containing from 1 to 5 carbon atoms, linear or branched alkyl groups containing from 1 to 5 carbon atoms, trifluoromethyl and trifluoromethoxy groups, and -CN groups,
and wherein, when there is more than one substituent, then each said substituent is the same or different.

Particularly preferred substituents on R¹ and R² are selected from hydrogen and the group consisting of substituents *a* ": chlorine atoms; hydroxyl groups; carboxyl groups; linear and branched alkyl, hydroxyalkyl,; linear and branched alkoxyl groups; alkoxycarbonyl groups; hydroxycarbonylalkyl groups; alkoxycarbonylalkyl groups; trifluoromethyl groups; trifluoromethoxy groups; -CN groups; amino, alkylamino, and dialkylamino groups; alkoxycarbonylamino, alkylcarbonylamino groups; alkylaminocarbonyloxy groups; alkyl groups substituted with an amino, alkylamino, or dialkylamino group; CONH₂; alkylcarbonylalkyl; alkylthio; sulphonyl and alkylsulphonyl groups; sulphonamide, alkylsulphonamide, and di(alkylsulphonyl)amino groups; trifluoromethylsulphoxide; trifluoromethylsulphonyl groups; trifluoromethylsulphonamide, and di(trifluoromethyl-suiphonyl)amino groups; and phenyl, phenoxyl, pyrrolidinyl, piperidinyl, piperazinyl, morpholinyl, and thiomorpholinyl groups optionally substituted by one or more substituents, which may be the same or different, selected from the group b as defined above,
and wherein any pyrrolidinyl, piperidinyl, piperazinyl, morpholinyl, and thiomorpholinyl groups are not further substituted.

Each of R¹ and R² is preferably an, optionally substituted, phenyl, pyridinyl, or thienyl group. Particularly preferably, R¹ and R², or Z, R¹ and R² together forming said fused ring structure, are unsubstituted. More preferably, R¹ and R² are preferably each phenyl.

R⁶ is preferably an aryl or heteroaryl group selected from: fluorenyl, phenyl, _ naphthyl, monocyclic heteroaryls, and bicyclic heteroaryls, and is optionally substituted as defined. More preferably, R⁶ is selected from the group consisting of: phenyl, naphthyl, benzothiazolyl, fluorenyl, benzazolyl, benzoxazolyl, thienyl, thiazolyl, isothiazolyl, furyl, oxazolyl, isoxazolyl, imidazolyl, triazolyl, indolyl, pyrrolyl, quinolyl, pyridinyl, pyrazolyl, pyrazinyl, pyrimidinyl, pyridazinyl, pyrrolyl, furanyl, 1,2,3-triazolyl, 1,2,4-triazoiyl, tetrazolyl, 1,2,4-triazinyl, 1,3,5-triazinyl, benzofuranyl, benzothiazyl, benzimidazolyl, indazolyl, tetraquinolinyl, tetrahydroisoquinolinyl, isoquinolinyl, indolyl, carbazolyl, indolinyl, alpha- or beta-carbolinyl, and benzothienyl groups.
When R⁶ is substituted, then this is preferably by at least one substituent selected from substituents a': fluorine atoms; chlorine atoms; hydroxyl groups; carboxyl groups; aldehyde groups; linear and branched alkyl, hydroxyalkyl, and fluoroalkyl groups; linear and branched alkoxyl groups; linear and branched thioalkyl groups; alkoxycarbonyl groups; benzylcarbonyl groups; hydroxycarbonylalkyl groups; alkoxycarbonylalkyl groups; trifluoromethyl groups; trifluoromethoxy groups; -CN groups; amino, alkylamino, dialkylamino, acylamino, and diacylamino groups; alkoxycarbonylamino, alkylcarbonylamino groups; alkylaminocarbonyloxy groups; alkyl groups substituted with - an amino, alkylamino, dialkylamino, acylamino, or diacylamino group; CONH₂; alkylamido groups; alkylthio; alkylsulphoxide; sulphonyl, and alkylsulphonyl groups; sulphonamide, alkylsulphonamide, and di(alkylsulphonyl)amino groups; trifluoromethylsulphoxide; trifluoromethylsulphonyl groups; trifluoromethylsulphonamide, and di(trifluoromethylsulphonyl)amino groups; alkylcarbonylalkyl; phenyl, phenoxy, phenylthio, and benzyl groups; and saturated monocyclic heterocyclyl groups, said aryl and heterocyclyl groups being optionally substituted by one or more substituents, which may be the same or different, selected from the group *b* as defined above. More preferably, the substituent(s) are selected from substituents *a*" as defined above.

Substituents b are preferably selected from substituents b' consisting of: chlorine atoms; hydroxyl groups; linear and branched alkyl, hydroxyalkyl, and alkoxyl groups; trifluoromethyl groups; trifluoromethoxy groups; -CN groups; amino, alkylamino, and dialkylamino groups; sulphonyl, alkylsulphonyl groups; and sulphonamide, alkylsulphonamide, and di(alkylsulphonyl)amino groups.

In one preferred group of compounds, R³ represents a group -AlkCONR⁷R⁸, wherein R⁷and R⁸, together with the nitrogen atom to which they are linked, form a five-, six-, or seven- membered heterocyclic group, preferably a six-membered group. Preferably, the heterocyclic group is pyrrolidinyl, pyrrolinyl, morpholinyl, piperidinyl, piperazinyl, or homopiperazinyl, although other groups are illustrated below. The heterocyclic group may be substituted by at least one substituent **'c'** and, in one preferred embodiment, the heterocyclic group is piperazinyl and the substituent is attached to the available nitrogen atom, being the nitrogen in the ring, rather than the nitrogen to which R⁷ and R⁸ are attached. Preferred substituents on any ring are alkyl, and substituted carbonyl, and preferred substituted carbonyl groups are butoxycarbonyl, aminocarbonyl and alkylcarbonyl.

In one preferred embodiment, the heterocyclic ring comprises an unsubstituted nitrogen atom therein.

Any heterocyclic group may be substituted by an alkyl group, or may be unsubstituted.

In another preferred grouping, R³ represents a group -AlkCONR⁷R⁸, and one of R⁷ and R⁸ represents a hydrogen atom and the other represents a cycle.

Preferred cycles are six-membered cycles, such as cyclohexyl, phenyl, piperidinyl and piperazinyl. Other preferred cycles are five-membered cycles, preferably cyclopentyl or pyrrolidinyl.

Other preferred compounds include those wherein R³ represents a group -AlkCONR⁷R⁸ or -AlkNR⁷R⁸, and one of R⁷ and R⁸ represents a hydrogen atom and the other represents a hydrogen atom or an optionally substituted alkyl group. The alkyl group may be substituted by an aromatic group, preferably phenyl.

In one preferred class of compounds, one of R⁷ and R⁸ represents an alkyl group substituted by one or two substituents selected from: alkoxy, carboxyl, amino, alkylamino, dialkylamino, and aromatic groups. The aromatic group is preferably a phenyl or pyridinyl group.

In another preferred class, R³ represents a group -AlkCONR⁷R⁸ and one of R⁷ and R⁸ is a sulphonyl optionally substituted by an alkyl, amino, alkylamino or dialkylamino group.

There is also provided the class of compounds in which R³ represents a group -AlkCONR⁷R⁸ and one of R⁷ and R⁸ is a sulphonyl substituted by an aryl group optionally substituted by a substituent selected from substituents b.

There is further provided those compounds wherein R³ represents a group -AlkCONR⁷R⁸ and one of R⁷ and R⁸ is a carbonyl group substituted by an optionally substituted alkyl group or heterocyclic group which is itself optionally substituted with a substituent selected from substituents *b*.

In other preferred compounds, R³ is -AlkCOOR and R is H, or R is an alkyl group, preferably ethyl or terk-butyl.

R³ may also represent -AlkCOR⁹ and a preferred group of compounds is where R⁹ is a saturated heterocycle, such as a piperidinyl group. In one preferred group of compounds, the saturated heterocycle comprises an unsubstituted nitrogen atom.

R³ may also represent -AlkCOR⁹ and a preferred group of compounds is where k⁹ is an alkyl group substituted by phenyl group.

R³ may also represent -AlkOR¹⁰ or -AlkS(O)ₙR¹⁰, and a preferred group of compounds is where R¹⁰ is hydrogen or carbamoyl.

R³ may also represent -AlkOR¹⁰ or -AlkS(O)ₙR¹⁰, and a preferred group of compounds is where R¹⁰ is a C₁₋₄ alkyl group.

The group -AlkS(O)ₙR¹⁰ may simply be AlkSH or AlkSAlk, for example.

R³ may further represent -AlkSO₂NR¹⁰R¹⁰ and a preferred group of compounds is where R¹⁰ and R¹⁰ are independently hydrogen or a C₁₋₄ alkyl group.

In general, it is preferred that Alk represents a propylene group or, more genrally, C₁-₄-alkylene.

Generally, R⁷ and R⁸ may, for example, each represent a methyl or ethyl group. In the group -AlkCONR⁷R⁸, they may form, together with the nitrogen atom to which they are linked, for example, a morpholinyl, thiomorpholinyl, piperazinyl, hoznopiperazinyl, pyrrolidinyl, imidazolyl, or piperidinyl group, optionally substituted by at least one substituent which may be selected, for example, from the group consisting of: chlorine atoms, hydroxyl groups, trifluoromethyl groups, alkoxy groups, hydroxyalkyl groups, and alkyl groups. Preferred compounds in this group are where R⁷ and R⁸, together with the nitrogen atom to which they are linked, may form a morpholinyl group optionally substituted by at least one substituent selected from the group consisting of: trifluoromethyl groups and alkyl groups. More preferably, R⁷ and R⁸, in this group, together with the nitrogen atom to which they are linked, form a morpholinyl group or thiomorpholinyl group, particularly preferably a morpholinyl group.

Z may be >CH-, >C=CH-, or >N-, and p is preferably 2 when Z is >C- or >N-, and 1 when Z is >C=CH-.

Preferably, q is 0.

R⁵ is preferably a methyl group, and is more preferably hydrogen.

In one preferred embodiment, Q is >C=O. In this embodiment, it is preferred that Z is >N- and R⁴ is H.

In another preferred embodiment, Q is >C=S.

In another preferred group, Q is >C=O, and q is 1 or 0, preferably 0.

In a further preferred group, Q is >C=O, and q is 0.

In the above preferred groups, R¹ and R² are preferably each unsubsituted phenyl, and Z is N or >CH-.

It is generally preferred that any alkyl, alkenyl or alkynyl component has no more than 4 carbon atoms.

It will be appreciated that compounds of formula (I) may be in any racemic, enantiomeric, or diastereoisomeric form. Salts include addition salts with inorganic and organic acids or bases.

The substituents R¹ and R² are the same or different, and there is no particular preference for whether they are the same or different, although more preferred groups are as defined above. There is no particular preference for the nature of the aryl group or heteroaryl group, although it is generally preferred that they be monocyclic and 5- or 6-membered. A general preference is that both be unsubstituted phenyl.

In the compounds of the present invention, where a sulphur atom is present, other than at position Q, then it may be present in the sulphoxide (SO) or sulphone (SOz) forms, where desired.

In general, carboxyl groups are in the form -COOH, and branched alkyl may take the form of singly or multiply branched alkyl, such as t-butyl or 4-methylpentyl, for example. Alkyl groups preferably contain from 1 to 6 carbons, and more preferably from 1 to 4 carbon atoms. Methyl and ethyl are particularly preferred as substituents. Similar considerations apply to hydroxyalkyl, haloalkyl, alkylthio, alkenyl, and alkynyl groups. Hydroxyalkyl may be substituted by one or more hydroxyl groups, but preferably one. Thioalkyl groups typically take the form HS-Alk-, where Alk indicates an alkyl group. Hydroxycarbonylalkyl typically take the form HOOC-AIk-. Alkylcarbonyl groups take the form Alk-CO-, while alkoxycarbonylalkyl groups take the form AlkOCOAlk-. Alkoxycarbonyl groups take the form AI.kOCO-. Alkylthio groups take the form Alk-Sand are optionally in the sulphoxide (Alk-SO-) or sulphone (Alk-SO₂-) forms. Any alkyl component preferably has from 1 to 6 carbon atoms, so that alkoxycarbonylalkyl may be hexyl-5-pentanoate or methylmethanoate for example. Alkenyl and alkynyl components have from 2 to 6 carbon atoms, and take the form of an alkyl group possessing at least one double or triple bond between adjacent carbons. It is preferred that there is only one such unsaturated bond per alkenyl or alkynyl substituent.

Where multiple substituents are selected from a common group, such as substituents a, *b* or c, then each substituent is the same or different.

R⁷ and R&, when representing alkyl, are preferably methyl or ethyl, and it is further preferred that these are unsubstituted or substituted with one or more fluorine atoms. Similar considerations apply when R⁷ and R⁸ represent alkylaminoalkyl or dialkylaminoalkyl groups.

When R⁷ and R⁸ form a heterocycle, it is preferred that this is saturated and contains 5 or 6 ring atoms, said heterocycle being optionally substituted by at least one substituent. selected from the group 'c' as defined.

When R⁷ and R⁸ represent an unsaturated heterocycle, the additional heteroatoms, if any, may typically be selected from oxygen, sulphur and nitrogen. Exemplary unsaturated heterocycles include, imidazole, pyrazole, indazole, benzimidazole, purine, aza-benzimidazole, triazole, pyrrole, indole, isoindazole, and azaindole.

More generally, it is preferred that, when R⁷ and R⁸, together with the nitrogen atom to which they are linked, form a heterocycle, then the heterocycle is saturated. Preferred saturated heterocycles are morpholinyl, thiomorpholinyl, piperazinyl, homopiperazinyl, and piperidinyl groups, preferably morpholinyl and thiomorpholinyl, and particularly morpholinyl.

In one aspect, R⁶ is a substituted or unsubstituted thiazolyl or benzothiazolyl group.

In general, R⁶ represents an aryl or heteroaryl group. There is no particular restriction on the nature of the aryl or heteroaryl group, but it is generally preferred that such a group is monocyclic or bicyclic, preferably containing 5, 6, 9 or 10 ring atoms.

In one embodiment, R⁶ is unsubstituted.

In the group a, and elsewhere, hydroxyalkenyl, hydroxyalkynyl groups are as defined above for alkenyl and alkynyl, and have one or more hydroxyl groups present, preferably one. Similarly, haloalkyl, haloalkenyl, and haloalkynyl groups have one or more halogen atoms present thereon, preferably selected from iodine, bromine, chlorine and fluorine, preferably chlorine or fluorine. Perhalo substituents are preferably perfluoro substituents, preferably trifluoromethyl. Where an alkyl group is specified herein, then this may include haloalkyl, particularly fluoroalkyl, and especially trifluoromethyl groups, although unsubstituted alkyl are genrally preferred over halo-substituted alkyls. The most preferred haloalkyl group is trifluoromethyl. Linear and branched alkoxyl groups and linear and branched thioalkyl groups are as defined above for linear and branched alkyl groups. Aralkoxy groups take the form Ar-AlkO-, while aryloxy groups take the form Ar0-, where Ar is an aryl or heteroaryl group. It will be understood that similar considerations apply to aralkoxycarbonyl and aryloxycarbonyl, and other groups specifying aralkoxy and aryloxy.

Acyl groups are those consisting of a carboxylic acid residue linked via the -CO-moiety. Alkyl-, aralkyl-, and aryl- amido groups have the appropriate groups linked via the nitrogen, such as Alk-CONH-. Amido takes the form of -CONH-, so that alkylamido takes the form alkyl-CONH-, for example, while aralkylamido takes the form aryl-alkyl-CONH-.

Sulphonamide, alkylsulphonamide, di(alkylsulphon.yl)arnino, aralkylsulphonamide, di(aralkylsulphonyl)amino, arylsulphonamide, and di(arylsulphonyl)amino are of the form sulphonyl or disulphonyl substituted on nitrogen, such as Alk-SO₂-NH-.

Alkoxycarbonylamino groups take the form Alk-O-CONH-, and aralkoxycarbonylamino, aryloxycarbonylamino, alkylcarbonylamino, aralkylcarbonylamino, and arylcarbonylamino groups should be construed accordingly. Alkylaminocarbonyloxy groups take the form Alk-NHCOO-, and aralkylaminocarbonyloxy and arylaminocarbonyloxy groups should be construed accordingly.

The present invention relates in particular to the novel compounds of formula (I), and especially to those compounds exemplified in the accompanying Examples hereinbelow.

Preferred compounds are:
3-[3-tert-butoxycarbonylmethyl-3-(3,3-diphenyl-propyl)-ureido]-benzoic acid methyl ester;
3-[3-(3,3-diphenyl-propyl)-3-(3-ethoxycarbonyl-propyl)-ureido]-benzoic acid methyl ester;
3-[3-(3-carboxy-propyl)-3-(3,3-diphenyl-propyl)-ureido]-benzoic acid;
3-[3-carboxymethyl-3-(3,3-diphenyl-propyl)-ureido]-benzoic acid;
(R)-1-(3,3-diphenylpropyl)-3-(4-(4-(methylsulfonamido)phenyl)thiazol-2-yl)-1-(4-oxo-4-(pipeiidin-3-ylami.no)butyl)urea;
1-(4-amino-4-oxobutyl)-1-(3,3-diphenylpropyl)-3-(4-(4-(methylsulfonarnido)-phenyl)thiazol-2-yl)urea;
3-(1-(3,3-diphenylpropyl)- 3-(4-phenylthiazol-2-yl)ureido)-2-methylpropanoic acid;
1-(3,3-diphenylpropyl)-3-(4-(4-(methylsulfonamido)phenyl)thiazol-2-yl)-1-(4-oxo-4-(piperidin-4-ylamino)butyl)urea;
(R)-3-(benzo[d]thiazol-2-yl)-1-(3,3-diphenylpropyl)-1-(4-oxo-4-(piperidin-3-ylamino)butyl)urea;
4-(3-(5-chloro-4-(4-(methylsulfonyl)phenyl)thiazol-2-yl)-1-(3,3-diphenylpropyl)-ureido)butanoic acid;
4-(3-(5-chloro-4-(4-(methylsulfonamido)phenyl)thiazol-2-yl)-1-(3,3-diphenylprapyl)ureido)butanoic acid;
(S)-3-(benzo[d]thiazol-2-yl)-1-(3,3-diphenylpropyl)-1-(4-oxo-4-(piperidin-3-ylamina)butyl)urea;
3-(benzo[d]thiazol-2-yl)-1-(3,3-diphenylpropyl)-1-(4-oxo-4-(piperidin-4-ylamino)butyl)urea;
(R)-I-(3,3-diphenylpropyl)- 1-(4-oxo-4-(piperidin-3-ylamino)butyl)-3-(4-phenylthiazol-2-yl)urea; _
1-(3-amino-3-oxopropyl)-1-(3,3-diphenylpropyl)-3-(4-(4-(methylsulfonamido)-phenyl)thiazol-2-yl)urea;
(S)-1-(3,3-diphenylpropyt)-1-(4-oxo-4-(pipendin-3-ylammo)butyl)-3-(4-phenytthiazol-2-yl)urea;
4-(1-(3,3-diphenylpropyl)-3-(4-(4-(methylsulfonyl)phenyl)thiazol-2-yl)ureido)butanoic acid;
1-(4-amino-4-oxobutyl)-3-(benzo[d]thiazol-2-yl)-1-(3,3-diphenylpropyl)urea;
1-(4-amino-4-oxobutyl)-3-(5-chloro-4-phenylthiazol-2-yl)-1-(3,3-diphenylpropyl)-urea;
3-(benzo[d]thiazol-2-yl)-1-(3,3-diphenylpropyl)-1-(4-oxo-4-(piperazin-1-yl)butyl)urea;
4-(3-(5-chloro-4-phenylthiazol-2-yl)-1-(3,3-diphenylpropyl)ureido)butanoic acid;
1-(4-(4-acetylpiperazin-1-yl)-4-oxobutyl)-1-(3,3-diphenylpropyl)-3-(4-phenylthiazol-2-yl)urea;
3-(benzo[d]thiazol-2-yl)-1-(3,3-diphenylpropyl)-1-(4-(ethylamino)-4-oxobutyl)urea;
3-(benzo[d]thiazol-2-yl)-1-(4-(be.nzylam-mo)-4-oxobutyl)-1-(3,3-diphenylpropyl)urea;
3-(benzo[d]thiazol-2-yl)-1-(4-(4-(dimethylcarbamoyl)piperazin-1-yl)-4-oxobutyl)-1-(3,3 - diphenylpropyl)urea;
1-(3,3-diphenylpropyl)-1-(4-oxo-4-(piperazin-1-yl)butyl)-3-(4-phenylthiazal-2-yl)urea;
3-(benzo[d]thiazol-2-yl)-1-(4-(dimethylamino)-4-oxobutyl)-1-(3,3-diphenylpropyl)urea; (S)-1-(3,3-diphenylpropyl)-1-(4-(3-methylpiperazm-1-yl)-4-oxobutyl)-3-(4-phenylthiazot-2-yl)urea;
(R)-1-(3,3-diphenylpropyl)-1-(4-(2-methylpiperazin-1-yl)-4-oxobutyl)-3 -(4-phenylthiazol-2-yl)urea;
4-(3-(5-chloro-4-(4-cyanophenyl)thiazol-2-yl)-1-(3,3-diphenylpropyl)ureido)butanoic acid;
(S)-1-(3,3-diphenylpropyl)-1-(3-oxo-3-(piperidin-3-ylamino)propyl)-3-(4-phenylthiazol-2-yl)urea;
3-(benzo[d]thiazol-2-yl)-1-(3,3-diphenylpropyl)-1-(4-morpholino-4-oxobutyl)urea; tert-butyl 4-(4-(3-(benzo[d]thiazol-2-yl)-1-(3,3-diphenylpropyl)ureido)-butanoy!)piperazine-1 -carboxylate;
tert-butyl 4-(4-(1-(3,3-diphenylpropyl)-3-(4-phenylthiazol-2-yl)ureido)-butanoyl)piperazine-1-carboxylate;
3-(benzo[d]thiazol-2-yl)-1-(4-(cyclahexylarnino)-4-oxobutyl)-1-(3,3-diphenylpropyl)urea; 3-(3-(5-chloro-4-(4-(methylsulfonamido)phenyl)thiazol-2-yl)-1-(3,3-diphenylpropyl)ureido)propanoic acid;
(R)-1 -(3,3-diphenylpropyl)-1-(4-(3-methylpiperazm-1-yl)-4-oxobutyl)-3-(4-phenylthiazol-2-yl)urea;
(S)-1-(3,3-diphenylpropyl)-1-(4-(2-methylpiperazin-1-yl)-4-oxobutyl)-3-(4-phenylthiazol-2-yl)urea;
tert-butyl 4-(3-(benzo [d]thiazo!-2-yl)-1 -(3,3 -dipbenylpropyl)ureido)butano ate; (R)-1-(3,3-diphenylpropyl)-1-(3-oxo-3-(piperidin-3-ylamino)propyl)-3-(4-phenylthiazol-2-yl)urea;
3-(benzo[d]thiazol-2-yl)-1-(3,3-diphenylpropyl)-1-(3-morpholino-3-oxopropyl)urea;
4-(3-(benzo[d]thiazol-2-yl)-1-(3,3-diphenylpropyl)ureido)butanoic acid;
3-(benzo[d]thiazol-2-yl)-1-(3,3-diphenylpropyt)-1-(4-oxo-4-(pipendm-1-yl)butyl)-urea;
3-(benzo[d]thiazol-2-yl)-1-(3,3-diphenylpropyl)-1-(3-oxo-3-(piperidin-1-yl)propyl)urea;
3-(benzo[d]thiazol-2-yl)-1-(3-dimethylamino)-3-oxopropyl)-1-(3,3-diphenyl-propyl)urea;
3-(benzo[d]thiazol-2-yl)-1-(3,3-diphenylpropyl)-1-(4-(4-methylpiperazin-1-yl)-4-oxobutyl)urea;
1-(3-amino-3-oxopropyl)-3-(benzo[d]thiazol-2-yl)-1-(3,3-diphenylpropyl)urea;
1-(3-arnino-3-oxopropyl)-3-(5-chloro-4-phenylthiazol-2-yl)-1-(3,3-diphenylpropyl)urea;
4-(1-(3,3-diphenylpropyl)-3-(4-phenylthiazol-2-yl)ureido)butanoic acid;
3-(benzo[d]thiazol-2-yl)-1-(3-(cyclohexylamino)-3-oxopropyl)-1-(3,3-diphenylpropyl)urea;
3-(1-(3,3-diphenylpropyl)-3-(4-(4-(methylsulfonamido)phenyl)thiaol-2-yl)ureido)propanoic acid;
3-(3-(benzo[d]thiazol-2-yl)-1-(3,3-diphenylpropyl)ureido)propanoic acid; (S)-3-(benzo[d]thiazol-2-yl)-1-(3,3-diphenylpropyl)-1-(3-(3-methylpiperazin-1-yl)-3 oxoprvpyl)urea;
ethyl 4-(3-(benzo[d]thiazol-2-yl)-1-(3,3-diphenylpropyl)ureido)butanoate;
3-(benzo[dlthiazol-2-yl)-1-(3-(benzylamino)-3-oxopropyl)-1-(3,3-diphenylpropyl)urea;
3-(benxo[d]thiazol-2-yl)-1-(3,3-diphenytpropyt)-1-(4-(4-isopropylpiperazin-1-yl)-4-oxobutyl)urea;
3-(1-(3,3-diphenylpropyl)-3-(4-(4-(methylsulfonyl)phenyl)thiazol-2-yl)ureido)propanoic acid;
(R)-3-(benzo[d]thiazoi-2-yl)-1-(3,3-dipbenytpropyl)-1-(3-(2-methylpiperaxm-1-yl)-3-oxopropyl)urea;
3-(3-(5-chloro-4-phenylthiazol-2-yl)-1-(3,3-diphenylpropyl)ureido)propanoic acid;
3-(3-(benzo[d]thiazol-2-yl)-1-(3,3-diphenylpropyl)ureido)-2-methylpropanoic acid; (S)-3-(benzo[d]thiazol-2-yl)-l-(3,3-diphetiylpropyl)-1-(3-(2-methylpiperazin-1-yl)-3-oxopropyl)urea;
4-(1-(3,3-diphenylpropyl)-3-(4-(4-sulfonamido)phenyl)thiazol-2-yl)ureido)butanoic acid; (R)-3-(benzo[d]thiazol-2-yl)-1-(3,3-diphenylpropyl)-1-(3-(3-methylpiperazin-1-yl)-3-oxopropyl)urea;
3-(1-(3,3-diphenylpropyl)-3-(4-phenylthiazol-2-yl)ureido)propanoic acrid
3-(benzo[d]thiazol-2-yl)-1-(3,3-diphenylpropyl)-1-(4-(piperidine-4-carboxamido)butyl)urea;
3-(benzo[d]thiazol-2-yl)-1-(3,3-diphenylpropyl)-1-(4-(piperidine-2-carboxamido)butyl)urea;
3-(benzo[d]thiazol-2-yl)-1-(3,3-diphenylpropyl)-1-(2-(piperidine-3-carboxamido)ethyl)urea;
1-(3,3-diphenylpropyl)-3-(4-(4-(methylsulfonamido)phenyl)thiazol-2-yl)-1-(2-(piperidine-3-carboxamido)ethyl)urea;
3-(5-chloro-4-(4-(methylsulfonamido)phenyl)thiazol-2-yl)-1-(3,3-diphenylpropyl)-1-(2-(piperidine-3-carboxamido)ethyl)urea;
2-(3-(5-chloro-4-(4-(methylsulfonamido)phenyl)thiazol-2-yl)-1-(3,3-diphenylpropyl)-ureido)ethyl carbamate;
3-(5-chloro-4-(4-(methylsulfonamido)phenyl)thiazol-2-yl)-1-(3,3-diphenylpropyl)-1-(2-hydroxyethyl)urea;
3-(5-chloro-4-(4-(methylsulfonamido)phenyl)thiazol-2-yl)-1-(3,3-diphenylpropyl)-1-(3-hydroxypropyl)urea;
1-(2-ammoethyl)-3-(5-chloro-4-(4-(methylsulfonamido)phenyl)thiazol-2-yl)-1-(3,3-diphenylpropyl)urea;
2-[{[(5-chloro-4-{4-[(methylsutfbnyl)amino]phenyl}-1,3-thiazol-2-yl)amino]carbonyl)(3,3-diphenylpropyl)amino]ethanesulfonamide;
3-(5-chloro-4-(4-(methylsulfonamido)phenyl)thiazol-2-yl)-1-(3,3-diphenylpropyl)-1-(2-(methylthio)ethyl)urea;
3-(5-chloro-4-(4-(methylsulfonamido)phenyl)thiazol-2-yl)-1-(3,3-diphenylpropyl)-1-(3-(methylthio)propyl)urea;
3 -(5-chioro-4-(4-(methylsulfonamido)phenyl)thiazol-2-yl)-1-(3,3-diphenylpropyl)-1-(2-(methylsulfonamido)ethyl)urea;
(R)-3-(5-chloro-4-(4-(methylsulfonamido)phenyl)thiazol-2-yl)-1-(3,3-dipheaylpropyl)-1-(2-(piperidin-3-ylamino)ethyl)urea;
(8)-3-(5-chloro-4-(4-(methylsulfonamido)phenyl)thiazol-2-yl)-1-(3,3-diphenylpropyl)-1-(2-(piperidin-3-ylamino)ethyl)urea;
(S)-3-(5-chloro-4-phenylthiazol-2-yl)-1-(3,3-diphenylpropyl)-1-(2-(6-oxopiperidin-3-ylamino)ethyl)urea;
3-(benzo[d]thiazol-2-yl)-1-(3,3-diphenylpropyl)-1-(2-(piperidin-4-ylarnino)ethyl)urea; (R)-3-(benzo[d]thiazol-2-yl)-1-(3,3-diphenylpropyl)-1-(2-(6-oxopiperidin-3-ylamina)ethyl)-urea;
(R)-3-(betizo[d]thiazol-2-yt)-1-(3,3-diphenylpropyl)-1-(2-(pipendm-3-ylammo)ethyl)urea; (R)-3-(benzo[d]thiazol-2-yl)-1-(3,3'diphenylpropyl)-1-(2-(6-oxopiperidin-3-ylamino)ethyl)-urea;
(R)-3-(5-chloro-4-phenylthiazol-2-yl)-1-(3,3-diphenylpropyl)-1-(2-(6-oxopiperidin-3 - ylamino)ethyl)urea;
(2-[3-Benzothiazol-2-yl-1-(3,3-diphenyl-propyl)-ureido]-ethyl)-carbamic acid tert-butyl ester;
4-({2-[3-Benzothiazol-2-yl-1-(3,3-diphenyl-propyl)-ureido]-ethylamino}-methyl)-piperidine-1-carboxylic acid benzyl ester;
3-Benzothiazol-2-yl-1-[2-(3-dimethylamino-2,2-dimethyl-propylamino)-ethyl]-1-(3,3 - diphenyl-propyl)-urea trihydrachloride;
(2-[3-Benzothiazol-2-yl-1-(3,3-diphenyl-propyl)-ureido]-ethylamino)-acetic acid ethyl ester;
2-({2-[3-Benzothiazol-2-yl-1-(3,3-diphenyl-propyl)-ureido]-ethylamino}-methyl)-cyclopropanecarboxylic acid ethyl ester;
3-Benzothiazol-2-yl-1-[2-(2,2-dimethoxy-ethylamino)-ethyl]-1-(3,3-diphenyl-propyl)-urea;
3-Benzothiazol-2-yl-1-(3,3-diphenyt-propyl)-1-{2-[(6-methoxy-pyridin-3-ylmethyl)-amino]-ethyl}-urea;
3-Benzothiazol-2-yl-1-(3,3-diphenyl-propyl)-1-[2-(tetrahydro-thiopyran-4-ylamino)-ethyl]-urea;
3-Benzothiazol-2-yl-1-(3,3-diphenyl-propyl)-1-[2-(tetrahydro-pyran-4-ylamino)-ethyl]-urea;
3-Benzothiazol-2-yl-1-(3,3-diphenyl-propyl)-1-[2-(2-methyl-tetrahydro-furan-3-ylamino)-ethyl]-urea;
(4-{2-[3-Benzothiazol-2-yl-1-(3,3-dipbenyl-propyl)-ureido]-ethylamino}-piperidin-1-yl)-acetic acid tert-butyl ester;
4-{2-(3-Benzothiazol-2-yl-1-(3,3-diphenyl-propyl)-ureido]-ethylamino}-piperidine-1-carboxylic acid tert-butyl ester;
3-Benzothiazol-2-yl-1-(3,3-diphenyl-propyl)-1-(2-(piperidin-4-ylamino)-ethyl]-urea; {2-[3-Benzothiazol-2-yl-1-(3,3-diphenyl-propyl)-ureido]-ethylammo}-acetic acid;
(4-{2-[3-Benzothiazol-2-yl-1-(3,3-diphenyl-propyl)-ureido]-ethylamino}-piperidin-1-yl)-acetic acid;
3-Benzothiazol-2-yl-1-[2-(cyclohexyl-methyl-amino)-ethyl]-1-(3,3-diphenyl-propyl)-urea; 1-[2-(Cyclohexyl-methyl-amino)-ethyl]-1-(3,3-diphenyl-propyl)-3-(4-phenyl-thiazol-2-yl)-urea;
4-{2-[3-Benzothiazol-2-yl-1-(3,3-diphenyl-propyl)-ureido]-ethylcarbamoyl}-piperidine-1-carboxylic acid tert-butyl ester;
Piperidine-4-carboxylic acid {2-[3-benzothiazol-2-yl-1-(3,3-diphenyl-propyl)-ureido]-ethyl}-amide;
*N*-(2-[3-Benzothiazol-2-yl-1-(3,3-diphenyl-propyl)-ureido]-ethyl}-methanesulfonarnide; Propane-1-sulfonic acid (2-[3-benzotWazol-2-yl-l-(3,3-diphenyl-propyl)-ureido]-ethyl)-amide;
*N*-{2-[3-Benzothiazol-2-yl-1-(3,3-diphenyl-propyl)-ureido]-ethyl}-4-cyanobenzenesulfonamide;
*N*-(4-{2-[3-Benzothiazol-2-yl-1-(3,3-diphenylpropyl)-ureido]-ethylsulfamoyl}-phenyl)-acetamide;
2-{2-[3-Benzothiazol-2-yl-1-(3,3-diphenyl-propyl)-ureido]-ethylsulfamoyl}-benzoic acid methyl ester;
*N*-{2-[3-Benzothiazol-2-yl-1-(3,3-diphenylpropyl)-ureido]-ethyl}-4-methoxybenzene-sulfonamide;
*N*-{2-[3-Benzothiazol-2-yl-1-(3,3-diphenylpropyl)-ureido]-ethyl}-4-trifluoromethyl-benzenesulfonamide;
N, N-dimethylamino-sulfonyl-{ 2-[3-benzothiazol-2-yl-l-(3,3-diphenyl-propyl)-ureido]-ethyl}-amide; and
3-Benzothiazol-2-yl-1-(3,3-diphenylpropyl)-1-(2-hydroxy-ethyl)urea.

Addition salts with inorganic or organic acids of the compounds of formula (I) can optionally be salts formed between a molecule of formula (I) and one, two or three acid molecules. These salts may be, for example, salts formed with hydrochloric, hydrobromic, hydroiodic, nitric, sulphuric, phosphoric, propionic, acetic, trifluoroacetic, formic, benzoic, maleic, fumaric, succinic, tartaric, citric, oxalic, glyoxylic, aspartic or ascorbic acids, alkylmonosulphonic acids such as, for example, methanesulphonic acid, ethanesulphonic acid, propanesulphonic acid, alkyldisulphonic acids such as, for example, methanedisulphonic acid, alpha-, beta-ethane disulphonic acid, arylmonosulphonic acids such as benzenesulphonic acid and aryl disulphonic acids.

Stereoisomerism can be defined broadly as isomerism of compounds having the same general formulae, but of which the different groups are disposed differently in space such as, in particular, in monosubstituted cyclohexanes of which the substituent can be in the axial or equatorial position, and the various possible rotational configurations of ethane derivatives. However, there is another type of stereoisomerism due to the different spatial arrangements of substituents fixed either on double bonds or on rings, which is often called geometric isomerism or cis-trans isomerism. The term stereoisomers is used in its broadest sense in the present application and therefore relates to all of the above-mentioned compounds.

There is further provided the use of a compound as defined in any of the accompanying claims in the manufacture of a medicament for the treatment or the prevention of diseases or disorders linked to abnormal physiological behaviour of inorganic ion receptors and in particular of the calcium receptor. Preferably, the calcium receptor is expressed in the parathyroid, the thyroid, the bone cells, the renal cells, the lung, the brain, the pituitary gland, the hypothalamus, the gastrointestinal cells, the pancreas cells, the skin cells, the cells of the central or peripheral nervous system and/or the smooth muscle cells. ,

The present invention further provides use of a compound as defined in any of the accompanying claims in the manufacture of a medicament for the prevention or treatment of: cancers, in particular of the parathyroid and the digestive tract; neurodegenerative diseases; bone and articular metabolism diseases, in particular osteoporosis, osteopaenia and Paget's disease, rheumatoid arthritis and osteoarthritis; abnormal calcium homeostasis; hyperplasia and parathyroid adenoma; intestinal malabsorption; biliary lithiasis and renal lithiasis; hyperparathyroidism, preferably where said hyperparathyroidism is observed in the event of renal insufficiency; ionised serum calcium level reduction during the treatment of hypercalcaemia; and, cardiovascular diseases and more particularly hypertension.

The present invention relates in particular to the compounds of formula (I), and especially to those compounds exemplified in the accompanying Examples.

The above-described compounds can, if desired, be subjected to salification reactions, for example using an inorganic or organic acid or an inorganic or organic base, by conventional methods known to the person skilled in the art.

The optically active forms of the above-described compounds may be prepared by resolving the racemic forms by conventional methods known to the person skilled in the art.

Illustrations of reactions of the type defined above are given in the preparation of the examples described hereinafter.

The above reactions are further illustrated in the accompanying, non-limiting Examples.

The products of formula (I) as defined above and their addition salts with acids or bases have beneficial pharmacological properties.

The products of the present invention can thus act on an inorganic ion, especially calcium, receptor and thus modulate one or more activities of the receptor.

Products of the present application which act on calcium receptors may thus be used, in particular, for the treatment or prevention of diseases or disorders linked with abnormal physiological behaviour of inorganic ion receptors and, in particular, of calcium receptors such as membrane calcium receptors capable of binding extracellular calcium (Ca sensing receptor CaSR).

It will be appreciated that mention of calcium receptors and CaSR herein includes reference to other inorganic ion receptors unless otherwise indicated or apparent from the context. It will be noted that the preferred target receptor of the present invention is the calcium receptor, and especially CaSR.

The products of the present invention as defined above are capable of modulating the activity of the calcium receptor. The products of the present invention can thus act as agonists or antagonists of the calcium receptor.

While the compounds of the invention are believed to exert their effects by interacting with the calcium sensing receptor (CaSR), the mechanism of action by which the compounds act is not a limiting embodiment of the invention. For example, compounds of the invention may interact with calcium sensing receptors other than CaSR.

Thus, the products of the present invention are of particular use in regulating the serum levels of PTH and extracellular Ca⁺⁺. Preferred products of the present invention possess agonistic properties toward the calcium receptor and can therefore be used, in particular, to participate in a reduction of the serum levels in the parathyroid hormone known as PTH: these products could thus be useful, in particular, for the treatment of diseases such as hyperparathyroidism. Similarly, abnormalities in calcium homeostasis can be treated with these compounds, in particular hypercalcaemia. Still in the region of the parathyroid, the compounds of formula (I) as defined can treat hyperplasia and parathyroid adenoma.

Another preferred class of products of formula (I) as defined above has properties which enable them to reduce bone resorption which depends directly on the fluctuation of circulating PTH levels: these products could be useful, in particular, for the treatment of diseases such as osteoporosis, osteopaenia Paget's disease and the reconstruction of fractures. They can also be used in the treatment and prophylaxis of polyarthritis and osteoarthritis.

It will be appreciated that reference to treatment herein includes reference to all applicable forms of treatment and prophylaxis.

With regard to digestion, the products of the present invention may also be used for the treatment of motor disorders (such as diarrhoea or constipation), functional digestive disorders, ulcerous diseases, sarcoidosis, familial adenomatous polyposis, polyps of the intestine and colon, cancer of the colon and intestinal malabsorption.

The presence of the calcium receptor in various cells of the nervous system (in particular the pituitary gland and hypothalamus) indicates that the products of the present invention can thus be used for the treatment or prevention of diseases or disorders such as, in particular: inappropriate antidiuretic hormone secretion (ADH syndrome), convulsions, stroke, cranial traumatism, diseases of the spinal marrow, neurodegenerative diseases (such as Alzheimer's disease, Parkinson's disease and Huntington's chorea), dementia, migraine, cerebral hypoxia, abnormalities in growth hormone secretion, psychiatric diseases (such as depression, anxiety, obsessive behaviour disorder, schizophrenia, post-traumatic stress, and neuroleptic malignant syndrome).

The products of formula (I) of the present invention may also possess therapeutic properties in regard of the following: thrombopaenia, platelet hypo- or hyper-coagulability, arterial hypertension, cardiac insufficiency, prevention or attenuation of renal toxicity of aminosides, renal lithiasis, pancreas insufficiency, diabetes, psoriasis, breast adenoma and cancer, cirrhosis, biliary lithiasis, and obesity.

The present invention further provides medicaments comprising compounds of formula (I), in any and all possible racemic, enantiomeric and diastereoisomeric isomeric forms, as well as the pharmaceutically acceptable addition salts thereof with inorganic and organic acids or inorganic or organic bases.

It is especially preferred that the compounds of formula (I) as defined above are used in the treatment and prophylaxis of diseases needing control of PTH hormone levels in the plasma.

It is especially preferred that the compounds of formula (I) as defined above are used in the treatment and prophylaxis of hypercalcaemia or hyperparathyroidism. Such products are particularly useful for the treatment or prevention of hyperparathyroidism.

In a preferred aspect the present invention provides medicaments comprising a compound of formula (I), and/or an addition salt thereof.

Preferred compounds are those listed above and as described in the accompanying Examples, especially when present as the active ingredient of a medicament.

The invention also relates to pharmaceutical compositions containing at least one of the medicaments defined above as the active ingredient.

The invention further relates to the use of the compounds of formula (I) as defined, above and/or their pharmaceutically acceptable salts:
- for the manufacture of medicaments for the treatment or prevention of diseases or disorders linked to abnormal physiological behaviour of inorganic ion receptors and in particular of the calcium receptor, characterised in that the calcium receptor is expressed in at least one of the parathyroid, the thyroid, the bone cells, the renal cells, the lung, the brain, the pituitary gland, the hypothalamus, the gastrointestinal cells, the pancreas cells, the skin cells, the cells of the central or peripheral nervous system and the smooth muscle cells,
- for the manufacture of medicaments for the prevention or treatment of cancers, in particular of the parathyroid and/or the digestive tract,
- for the manufacture of medicaments for the prevention or treatment of neurodegenerative diseases,
- for the manufacture of medicaments for the prevention or treatment of bone and articular metabolism diseases, in particular osteoporosis, osteopaenia and Paget's disease, rheumatoid arthritis and/or osteoarthritis,
- for the manufacture of medicaments for the prevention or treatment of abnormal calcium homeostasis,
- for the manufacture of medicaments for the prevention or treatment of hyperplasia and/or parathyroid adenoma,
- for the manufacture of medicaments for the prevention or treatment of intestinal malabsorption,
- for the manufacture of medicaments for the prevention or treatment of biliary lithiasis and/or renal lithiasis,
- for the manufacture of medicaments for the prevention or treatment of hyperparathyroidism, characterised in that secondary hyperparathyroidism is observed in the event of renal insufficiency,
- for the manufacture of medicaments for the prevention or treatment of ionised serum calcium level reduction during the treatment of hypercalcaemia,
- for the manufacture of medicaments for the prevention or treatment of cardiovascular diseases.

In one aspect, the invention provides a method of inhibiting, decreasing or preventing vascular calcification in an individual. The method comprises administering to the individual a therapeutically effective amount of the calcimimetic compound of the invention. In one aspect, administration of the compound of the invention retards or reverses the formation, growth or deposition of extracellular matrix hydroxyapatite crystal deposits. In another aspect of the invention, administration of the compound of the invention prevents the formation, growth or deposition of extracellular matrix hydroxyapatite crystal deposits.

In one aspect, the compounds of the invention may be used to prevent or treat atherosclerotic calcification and medial calcification and other conditions characterized by vascular calcification. In one aspect, vascular calcification may be associated with chronic renal insufficiency or end-stage renal disease. In another aspect, vascular calcification may be associated with pre- or post-dialysis or uremia. In a further aspect, vascular calcification may be associated with diabetes mellitus I or II. In yet another aspect, vascular calcification may be associated with a cardiovascular disorder.

In one aspect, administration of an effective amount of the compounds of the invention can reduce serum PTH without causing aortic calcification. In another aspect, administration of the compounds of the invention can reduce serum creatinine level or can prevent increase of serum creatinine level. In another aspect, administration of the compounds of the invention can attenuates parathyroid (PT) hyperplasia.

The compounds of the invention may be administered alone or in combination with other drugs for treating vascular calcification, such as vitamin D sterols and/or RENAGEL®. Vitamin D sterols can include calcitriol, alfacalcidol, doxercalciferol, maxacalcitol or paricalcitol. In one aspect, the compounds of the invention can be administered before or after administration of vitamin D sterols. In another aspect, the compounds of the invention can be co-administered with vitamin D sterols. The methods of the invention can be practised to attenuate the mineralising effect of calcitriol on vascular tissue. In one aspect, the methods of the invention can be used to reverse the effect of calcitriol of increasing the serum levels of calcium, phosphorus and Ca x P product thereby preventing or inhibiting vascular calcification. In another aspect, the compounds of the invention of the invention can be used to stabilise or decrease serum creatinine levels. In one aspect, in addition to creatinine level increase due to a disease, a further increase in creatinine level can be due to treatment with vitamin D sterols such as calcitriol. In addition, the compounds of the invention may be administered in conjunction with surgical and non-surgical treatments. In one aspect, the methods of the invention can be practised in injunction with dialysis.

In one aspect, the compounds of the invention can be used for treating abnormal intestinal motility disorders such as diarrhoea. The methods of the invention comprise administering to the individual a therapeutically effective amount of the compounds of Formula 1.

As used herein, the term "diarrhoea" refers to a condition of three or more unformed stools in a 24-hour period of volume more than 200 g per day. In one aspect, diarrhoea can be osmotic, i.e., resulting if the osmotic pressure of intestinal contents is higher than that of the serum. This condition may result from malabsorption of fat (e.g., in celiac disease) or of lactose (e.g., in intestinal lactase deficiency), or it can happen due to the use of certain laxatives (e.g., lactulose, magnesium hydroxide) or artificial sweeteners (e.g., sorbitol, mannitol). In another aspect, diarrhoea can be secretory, *i.e*., occurring when there is a net secretion of water into the lumen. This may occur with bacterial toxins (such as those produced, *e.g*., by E.coli and Vibrio cholerae), or with hormones, such as vasoactive intestinal polypeptide, which is produced by rare islet cell tumors (pancreatic cholera). Both osmotic and secretory diarrhoeas result from abnormalities in the small intestine such that the flow of water through the ileocecal area overcomes the absorptive capacity of the colon.

In a further aspect, diarrhoea can be exudative diarrhoea, i.e., resulting from direct damage to the small or large intestinal mucosa. This type of diarrhoea can be caused by infectious or inflammatory disorders of the gut. In one aspect, exudative diarrhoea can be associated with chemotherapy, radiation treatment, inflammation or toxic traumatic injury. In another aspect, exudative diarrhoea can be associated with a gastrointestinal or abdominal surgery.

In another aspect, diarrhoea can be due to acceleration of intestinal transit (rapid transit diarrhoea). Such condition may occur because the rapid flow-through impairs the ability of the gut to absorb water.

In one aspect, the invention provides the compounds and compositions for treating abnormal gastric fluid secretion / absorption disorders in conjunction with treating underlying causes of, for example, diarrhoea or with other treatment methods. In one aspect, calcimimetics can be administered to a subject before, after or concurrently with oral rehydration therapy. For example, oral rehydration therapy may contain the following ingredients: sodium, potassium, chloride, bicarbonate, titrate and glucose. In another aspect, the compounds of the invention can be administered to a subject before, after or concurrently with an antimotility agent, such as loperamide (Imodium), diphenoxylate, or bismuth subsalicylate (Pepto-Bismol). In another aspect, calcimimetics can be administered with antibiotics (e.g., trimethoprim-sulfamethoxazole (Bactrim DS), ciprofloxacin (Cipro), norfloxacin (Noroxin), ofloxacin (Floxin), doxycycline (Vibramycin), erythromycin). In one aspect, the compounds of the invention can be administered together with calcium or polyamines such as spermine, spermidine, putrescine, and ornithine metabolites or amino acids such of L-tryptophan, L-phenylalanine. In another aspect, the compounds of the invention can be administered together with sodium and glucose. In addition, the compounds of the invention may be administered in conjunction with surgical and non-surgical treatments.

The invention further provides methods for modulating intestinal fluid secretion and absorption. In one aspect, the purpose can be to increase fluid absorption and/or decrease fluid secretion in a subject and thus the methods of the invention can comprise administering an effective amount of a pharmaceutical composition comprising a compound of the invention.

The invention provides methods of modulation the absorption or secretion of a drug, poison or nutrient in the intestinal tract of a subject, comprising administering an effective amount of a pharmaceutical composition comprising a compound of the invention together with a pharmaceutically acceptable carrier to the subject. In one aspect, the invention provides methods of treatment of a malassimilation or a malabsorption of a subject, comprising administering an effective amount of a pharmaceutical composition comprising a compound of Formula I together with a pharmaceutically acceptable carrier to the subj ect.

As used herein the term "malassimilation" encompasses impaired processes of food digestions and absorption occurring in one of two ways (1) through intraluminal disorders (maldigestion of food) and (2) through intramural disorders (malabsorption of food).

Methods of the invention comprising administering a pharmaceutical composition of the invention can also be practised to treat malnutrition in a subject. For example, a subject can be malnourished if the subject is grossly underweight (weight for height is below 80% of the standard), grossly overweight (weight for height above 120°/a of the standard), if the subject unintentionally lost 10% or more of body weight, has a gastrointestinal tract surgery, experienced nutrient losses (e.g., from diarrhoea, dialysis, vomiting), has increased metabolic needs (e.g., due to pregnancy, lactation, increased physical activity, fever, injury), is an alcoholic or chronic drug user (antibiotics, antidepressants, diuretics), has medical conditions which interfere with nutrient intake, absorption, metabolism, or utilisation, has poor dentition (particularly in the elderly subjects), or has mouth sores due to herpes, HN or chemotherapy. In another aspect, the subject can be malnourished due to dietary risk factors (fez, loss of appetite, inadequate food or nutrient intake, lack of variety of foods, fad, weight-loss diets, inadequate fibre, excessive fat, sodium, sugar, excess alcohol, eats too few fruits, vegetables) or due to social risk factors (*e.g*., chronic ill heath, poverty, inadequate money to buy food, low socioeconomic status, immobility or inability to purchase, store, or cook food, social isolation, eats alone most of the time, substance abuser, conditions which limit subject's ability to eat). Further, the methods of the invention can be practised when a subject has limited access to nutrients such as during survival following environmental disasters, survival at sea, marooning and deep-sea living or space travel.

The products of formula (I) and their pharmaceutically acceptable salts may be administered to animals, preferably to mammals and, in particular, to humans, as therapeutic or prophylactic medicaments.

They may be administered as they are or in a mixture with one or more compounds of formula (1) or else in the form of a pharmaceutical composition containing as the active compound an effective dose of at least one product of formula (I) and/or their pharmaceutically acceptable salts and common pharmaceutically inert excipients and/or additives.

These pharmaceutical compositions can be administered buccally, enterally or parenterally or topically to the skin and mucous membranes or by intravenous or intramuscular injection.

The medicaments may therefore be administered orally, for example in the form of pills, tablets, coated tablets, gel-coated tablets, granules, hard and soft capsules, solutions, syrups, emulsions, suspensions or aerosol mixtures.

The medicaments may however be effectively administered rectally, for example in the form of suppositories, or as pessaries, or parenterally, for example in the form of injectable solutions or infusion, microcapsules or implants, percutaneously, for example in the form of an ointment, solutions, pigments or colorants, transdermally (patches) or by other methods, for example in the form of an aerosol or nasal spray.

The medicaments according to the present invention may therefore be formulated as pharmaceutical compositions containing one or more products of formula (I) as defined above.

Pharmaceutical compositions of this type can therefore constitute the form in which the products of formula (I) as defined above are used in the therapeutic application thereof.

The pharmaceutical compositions according to the invention are prepared by conventional methods, pharmaceutically inert organic or inorganic excipients being added to the compounds of formula (I) and/or their pharmaceutically acceptable salts.

These compositions may therefore be solid or liquid and may have any pharmaceutical forms commonly employed in human medicine, for example, simple tablets or dragees, pills, tablets, hard capsules, droplets, granules, injectable preparations, ointments, creams or gels; they are prepared by conventional methods.

Excipients such as lactose, cornstarch or derivatives thereof, talc, stearic acid or the salts thereof, for example, may be used for producing pills, tablets, coated tablets and hard gelatin capsules.

Suitable vehicles for soft gelatin capsules or suppositories include, for example, fats, semi-solid or liquid polyol waxes and natural or modified oils, etc. Appropriate vehicles for the preparation of solutions, for example injectable solutions, emulsions or syrups include, for example, water, alcohols, glycerol, polyols, sucrose, invert sugars, glucose, vegetable oils, etc. Suitable vehicles for microcapsules or implants include, for example, glyoxylic and lactic acid copolymers. The pharmaceutical preparations normally contain from 0.5 % to 90 % by weight of products of formula (I) and/or the physiologically acceptable salts thereof.

The active principle may be incorporated in excipients which are normally used in these pharmaceutical compositions, such as talc, gum arabic, lactose, starch, magnesium stearate, cocoa butter, aqueous or non-aqueous vehicles, fats of animal or vegetable origin, paraffin derivatives, glycols, various wetting agents, dispersants or emulsifiers and preservatives.

In addition to the active principles and excipients, the pharmaceutical compositions may contain additives such as, for example, diluents, disintegrating agents, binders, lubricants, wetting agents, stabilisers, emulsifiers, preservatives, sweeteners, colorants, flavourings or aromatising agents, thickeners, buffers and also solvents or solubilisers or retarding agents and also salts to modify osmotic pressure, coating agents or antioxidants.

They can also contain two or more products of formula (I) and/or their pharmaceutically acceptable salts as defined above. Moreover, in addition to at least one or more products of formula (I) and/or their pharmaceutically acceptable salts, they can contain at least one or more other active principle which can be used therapeutically or prophylactically.

Pharmaceutical compositions of this type contain as active compound an effective dose of at least one product of formula (I) and/or its pharmaceutically acceptable salts as well as one or more pharmaceutically acceptable excipients and/or vehicles and optionally one or more conventional additives.

The present invention thus extends to pharmaceutical compositions containing at least one of the medicaments as defined above as the active ingredient.

When using the products of formula (I), the doses can vary within wide limits and will be determined by the skilled physician, taking into account such factors as the age, weight and sex of the patient. Other factors to be taken into consideration include the compound employed, the nature and severity of the disease to be treated, whether the condition is serious or chronic, and whether a prophylactic treatment is being employed.

The pharmaceutical compositions normally contain from 0.2 to 500 mg, preferably from 1 to 200 g of compound of formula (I) and/or their pharmaceutically acceptable salts.

In the case of oral administration, the daily dose varies generally from 0.05 to 10 mg/kg and preferably from ,0. 1 to 8 mg/kg, in particular from 0.1 to 6 mg/kg. For an adult, for example, a daily dose varying from 5 to 500 mg could be considered.

In the case of intravenous administration, the daily dose varies approximately from 0.05 to 6 mg/kg and preferably from 0.1 to 5 mg/kg.

The daily dose may be divided into a plurality of portions, for example 2, 3 or 4 portions, in particular if a large amount of active ingredient is to be administered. It may possibly be necessary to administer the various doses in an increasing or decreasing manner, depending on the behaviour in an individual case. These doses may be applied multiple times per day, once a day, once every other day, or any other regimen deemed appropriate by the skilled physician. Apart from the use of the products of formula (1) as defined above as medicaments, their use as a vehicle or support for active compounds for transporting these active compounds specifically toward a site of action can also be envisaged (Drug targeting, see Targeted Drug Delivery, R.C. Juliano, Handbook of Experimental Pharmacology, Vol. 100, Ed. Born, G.V.R. et al, Springer Verlag). The active compounds which may be transported are, in particular, those used for the treatment or prevention of the above-mentioned diseases.

The pharmaceutical compositions according to the present invention thus containing compounds of formula (I) and/or their pharmaceutically acceptable salts can thus be used, in particular, for the treatment or prevention of diseases necessitating the administration of products which are agonists or antagonists of inorganic ion receptors such as, in particular, calcium receptors.

The present invention accordingly relates, in particular, to the use of the products of formula (I) as defined above and/or their pharmaceutically acceptable salts for the manufacture of medicaments for the treatment or prevention of diseases or disorders linked to abnormal physiological behaviour of inorganic ion receptors and in particular of calcium receptors.

The pharmaceutical compositions according to the present invention can thus be used as medicaments for the above-mentioned therapeutic applications.

The present invention further relates to processes for the preparation of compounds of formula (I), as defined above, and the salts and/or isomers thereof.

The experimental section hereinafter provides examples of the preparation of compounds of formula (1). These Examples illustrate the invention without limiting it.

### General

All reagents were obtained commercially unless otherwise noted.

Unless run in aqueous media, all reactions were performed using oven-dried glassware under an atmosphere of dry nitrogen.

Air- and moisture-sensitive liquids and solutions were transferred *via* syringe or stainless steel cannula.

Organic solutions were concentrated under reduced pressure (ca. 15-30 mm Hg) by rotary evaporation.

Anhydrous solvents were purchased from VWR or Aldrich and used as received. Chromatographic purification of products was accomplished using forced-flow chromatography on EMD Chemicals Inc. silica gel 60 (40-63 ppm) or the Teledyne Isco Combiflash Companion equipped with Teledyne Isco Redi-Sep normal phase disposable flash columns (silica 35-70 µm).

Thin layer chromatography was performed on Analtech, Inc. thin layer chromatography plates bearing silica gel HLF (250 microns, catalog #47521). Visualization of the developed chromatogram was accomplished by fluorescence quenching and by staining with ethanolic anisaldehyde, aqueous potassium permanganate, or aqueous ceric ammonium molybdate (CAM) solution.

Reverse phase HPLC was performed with an Agilent 1200 Series HPLC using a Phenomenex Gemini 10µ C18 110A preparative column (250x 30 mem) with UV detection of product (X = 214 nm and 254 nm). The products were eluted using a solvent gradient (solvent A = 0.1% TFA/H₂O; solvent B = 0.1% TFA/CH₃CN).

NMR spectra were acquired on Bruker 400 and S00 NMR instruments operating at 400 and 500 MHz, respectively, for ¹H NMR, and were referenced internally according to residual proton solvent signals. Data for ¹H NMR are recorded as follows: chemical shift (5, ppm), multiplicity (s, singlet; d, doublet, t, triplet; q, quartet; sept, septet; m, multiple), coupling constant (Hz), integration. Mass spectra were obtained on an Agilent 1100 Series HPLC equipped with a Shiseido Co., Ltd. Capcell Pak 3µ analytical column and Agilent 6140 Quadrupole LC/MS detector. Samples were eluted in positive mode using a solvent gradient (solvent A = 0.1°/p HCO₂H/H₂O; solvent B = 0.1% HCO₂H/CH₃CN) and negative mode using a solvent gradient (solvent A = 5mM NH₄O₂CH in 95:5H₂O/CH₃CN; solvent B = 100% CH₃CN).

### Preparation of thioureas of formula (II), ureas of formula (III):

As mentioned hereinafter, this may be achieved in several ways:

In the accompanying schemes, the substituent labels R₁ and R₁ correspond to substituents R¹ and R² of Formula (I), R₂ corresponds to R⁶, and R₃ corresponds to R⁹.

### Preparation of amines of formula (IV):

This may be achieved by the method of synthesis described below:

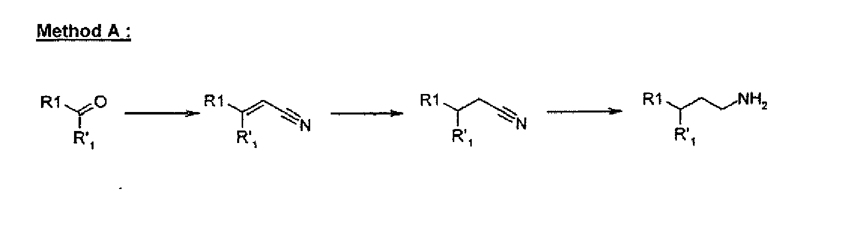

### Method A: 'synthesis of substituted 3.3-propylamines'

The synthesis of 2-(9H-fluoren-9-yl)-ethylarrune in which R1, R'1 = fluorenyl is described by way of example.

### Stage a):

### Synthesis of fluoren-9-ylidene-acetonitrile:

528 mg ofNaH (in a 55-65% suspension in oil)(13.2 mmol, 2.2 eq) in suspension in 20 mL of DME are introduced into a 1 a0 mL Woulff bottle equipped with a straight condenser. 1.94 mL of diethyl cyanomethylphosphonate (12 mmol, 2 e^{q}) in solution in 5 mL of DME are then added dropwise. After the release of gas, the reaction medium is heated under reflux for 1 S min, then 1.08 g of fluoren-9-one (6 mmol, 1 eq) in solution in 5 mL of DME are added dropwise.

After refluxing for 2 hours, the reaction is stopped by addition of 40 mL of an aqueous ammonium chloride solution.

The medium is taken up with ethyl acetate, and the aqueous phase is extracted with ethyl acetate. The organic phases are collected, dried over MgS₀4, filtered and concentrated. The fluoren-9-ylidene-acetonitrile is purified by chromatography over silica gel (CH₂CI₂/heptane elution gradient: 80/20) and obtained in a yield of 50 %.

### Stags

### Synthesis of (9H-fluoren-9-yl)-acetonitrile:

610 mg (3 mmol, 1 e^{q}) of fluoren-9-ylidene-acetonitrile in solution in 40 mL of methanol and 10 mL of ethyl acetate, then 225 mg of palladium hydroxide over coal are introduced into a 250 mL flask under a nitrogen atmosphere. The reaction medium is purged then placed under a hydrogen atmosphere (skin flask) and stirred for 6 hours while stirring. The catalyst is removed by filtration over Clarcel. The solvent is evaporated and the expected product is obtained in a yield of 73 %.

### Stage c):

### Synthesis of 2-(9H-fluoren-9-yl)-ethylamine:

6.3 mL (6.3 mmol, 2.7 eq) of a 1 M solution of LiAlH₄ in THF are dissolved in 20 mL of THF in a 250 mL flask under argon. The reaction medium is cooled to -78 °C and 478 mg (2.3 mmol, 1 eq) of (9H-fluoren-9-yl)-acetonitrile in solution in 20 niL of THF are added dropwise. The temperature is allowed to rise progressively to ambient temperature.

Stirring is continued for 5 hours, then the medium is hydrolysed at 0 °C by addition of 30 mL of a sodium and potassium tartrate solution.

The THF is evaporated and the aqueous phase is extracted with ethyl acetate. After drying over Mg2SO4 and evaporation of the solvent, the crude product is subjected to chromatography over silica gel (elution gradient: CH₂Cl₂ - CH₂Cl₂/MeOH: 9/1 to CH₂CI₂/MeOH/NI-₄0H: 91110.5). The 2-(9H-fluoren-9-yl)-ethylamine is obtained in a yield of 12 %.

### Amines of formula (IV) obtained by method A:

### Stage a):

| **Structure** | **MS** | **NMR (CDCl13)** |
|---|---|---|
| 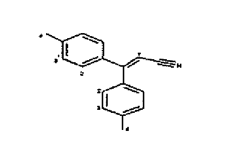 | MS/El 233: [M]⁺ 218: [M:]⁺ - CH₃ 203: [M :]⁺ - 2 (CH₃) | 7.27 (d, 2H, aromatic H), 7.12 (d, 2H, aromatic H), 6.85 (d, 2H, aromatic H), 6.79 (d, 2H, aromatic H), 5.76 (s, 1H, H alkene), 2.23 (s, 6H, 2xCH₃) |
| 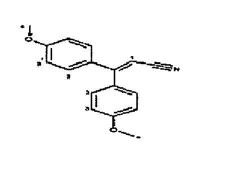 | Electrospray 266: [MH]⁺ | 3.84 and 3.86 (s and s, 2 x 3H, H₄ and H_{4'}). 5.54 (s, 1H, H₁), 6.88 and 6.95 (d and d, 2 x 2H, H₃ and H_{3'}) 7.25 and 7.40 (d and d, 2 x 2H, H₂ and H_{2'}). |
| 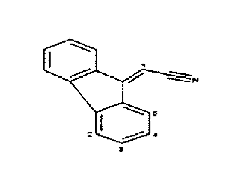 | MS/E1 203: [M]⁺ 176: [M :]⁺ - HCN | 6.89 (s, 1H, H₁), 7.34 (m, 1H, H.), 7.46 (m, 1 H, Har), 7.51 (m, 1 H, He 7.56 (m, I H, Hₐᵣ), 7.84 (d, 1 H, Har), 7.89 (d, 1H, Hₐᵣ), 794 (d, 1H, Hₐᵣ), 8.06 (d, 1H, Hₐᵣ) - |
| 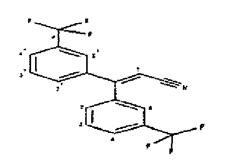 | MS/EI341: [M]⁺ 322: [M :]⁺ - F 272: [M :]⁺ - CF₃ 252: [M:]⁺ -CF₃ -HF | 5.88 (s, 1H, H₁), 7.42 (m, 1H, Hₐᵣ), 7.46 (m, 1 H, Har), 7.54 (m, 2H, Hₐᵣ), 7.62 to 7.81 (m, 4H, Hₐᵣ). |

### Stage b):

| **Structure** | **MS** | **NMR (CDCl3)** |
|---|---|---|
| 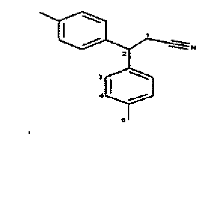 | MS/EI 235: [M]⁺ 207: [M:]⁺- HCN 195: [M]⁺ - (CH₂CH₂NH₂) | 2.25 (s, 6H, H₅), 3.26 (d, 2H, H₁), 4.32 (t, 1H, H₂), 7.11 (d, 4H, Hₐᵣ), 7.21 (d, 4H, Hₐᵣ). |
| 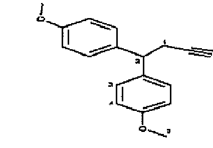 | MS/EI 267: [M]⁺ | 3.23 (d, 2H, H₁), 3.71 (s, 6H, H₅), 4.30 (t, 1H, H₂), 6.80 (d, 4H, H₄), 7.23 (d, 4H, H₃). |
| 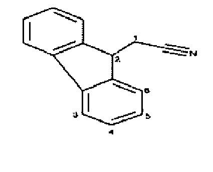 | MS/EI 205: [M]⁺ 165: [M]⁺ - (CH₂CH₂NH₂) | 3.30 (masked, 1H, H₂), 4.25 (d, 2H, H₂), 7.37 (m, 4H, H₄ or H₅), 7.44 (m, 4H, H₄ or H₅), 7.72 (d, 2H, H₃ or H₆), 7.90 (d, 2H, H₃ orH₆). |
| 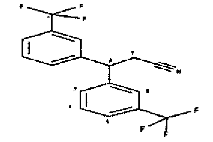 | MS/EI 343: [M]⁺ | 7.71 (d, 4H, aromatic H), 7.52 (d, 4H, aromatic H), 4.30 (t, 1H, CH), 3.42 (d, 2H, CH₂) |

### Stage c):

| **Structure** | **MS** | **NMR (CDCl3)** |
|---|---|---|
| 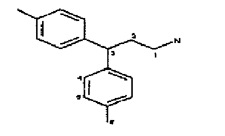 | MS/EI 239: [M]⁺ 222: [M]⁺ - NH₃ 195: [M]⁺ - (CH₂CH₂NH₂) | 2.18 (m, 2H, H₂), 2.29 (s, 6H, H₆), 2.67 (t, H₁), 3.94 (t, 1H, H₃), 7.07 (d, 4H, Hₐᵣ), (d, 4H, Hₐᵣ). |
| 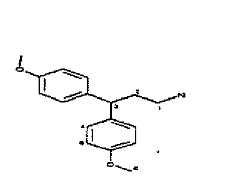 | Electrospray 271: [MH]⁺ 255: [MH]⁺ - NH₃ 195: [MH]⁺- (CH₂CH₂NH₂) | 2.04 (m, 2H, H₂), 2.45 (t, 2H, H₁), 3.94 (t. 1H, H₃), 3.70 (s, 6H, H₆), 6.82 (d, 4H, H₅), 7.16 (d, 4H, H₄). |
| 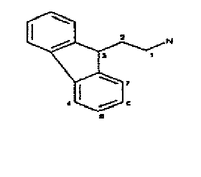 | MS/EI 209: [M]⁺ 191: [M]⁺ - NH₃ 178: [M]⁺ - (CH₂NH₂) 165: [M]⁺ - (CH₂CH₂NH₂) | 2.28 (m, 2H, H₂), 2.60 (m, 2H, H₁), 4.07 (t, 1H, H₃), 7.31 (m, 4H, H₅ or H₆), 7.37 (m, 4H, H₅ or H₆), 7.52 (d, 2H, H₄ or H₇), 7.75 (d, 2H, H₅ or H₇). |
| 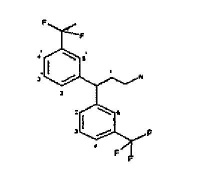 | MS/EI 347: [M]⁺ 330: [M]⁺ - NH₃ 318: [M]⁺ - CH₃NH₂ 303: [M]⁺ - CH₃CH₂NH₂ | 7.81 (d, 4H, aromatic H), 7.56 (d, 4H, aromatic H), 4.01 (t, 1H, CH), 2.35 (t, 2H, CH₂-N), 2.12 (m, 2H, CH2) |

### Preparation of ureas of formula (III):

This may be achieved by the method of synthesis described below:

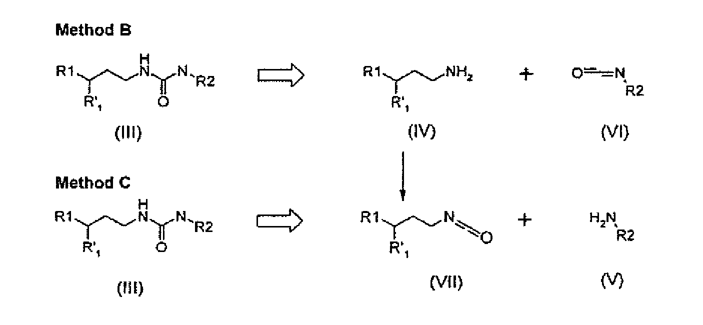

### EXAMPLES

### Method B: synthesis of ureas of formula (III) starting from commercial isocyanates of formula (VI)

The amine (0.5 mmol, 1 eq) of formula (IV) in solution in 6 mL of dichloromethane then the isocyanate (0.5 mmol, 1 eq) of formula (VI) were introduced into a 100 mL flask under N₂.

After 2 hours of stirring, the reaction mixture was taken up by 60mL of dichloromethane and washed with 20 mL of water then 20mL of brine. The organic phase was then dried over MgSO₄ and concentrated in a rotary evaporator.

The product was recrystallised in diethyl ether or subjected to chromatography over silica gel with an eluant: CH₂Cl₂/AcOEt - 90/10. The expected ureas were obtained in an average yield of 79%.

### Ureas of formula (III) obtained by method B:

| **Structure** | **MS** (**Electrospray)** | **NMR (300 MHz, CDCl3)** |
|---|---|---|
| | | |
| **EXAMPLE 1:** 1-(3,3-diphenyl-propyl)-3-(4-nitro-phenyl)-urea: | | |
| | | |
| 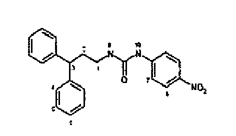 | 376: [MH]⁺ 398: [M + Na]⁺ 212: [(Ph₂CH-(CH₂)₂NH]⁺ | 2.20 (q, 2H, H₂), 2.95 (q, 2H, H₁), 4.09 (t, 1H, H₃), 7.03 to 7.37 (m, 10H, Hₐᵣ), 7.10 (t, 1H, H₉), 7.61 (d, 2H, H₇), 8.10 (d, 2H, H₈), 10.20 (sl, 1H, H₁₀). |
| | | |
| **EXAMPLE 2:** 1-Biphenyl-4-yl-3-(3.3-diphenyl-propyl)-urea | | |
| | | |
| 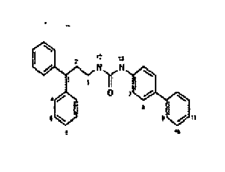 | 407: [MH]⁺ | 2.22 (q, 2H, H₂), 3.00 (q, 2H, H₁), 4.01 (t, 1H, H₃), 6.25 (t, 1H, H₁₂), 7.18 (m, 2H, Hₐᵣ), 7.31 (m, 9H, Hₐᵣ), 7.42 (m, 2H, H₁₀), 7.46 and 7.53 (m, 4H, H₇ and H₈), 8.55 (s, 1H, H₁₃). |
| | | |
| **EXAMPLE 3:** 1-(3,3-diphenyl-propyl)-3-(3-nitro-phenyl)-urea | | |
| | | |
| 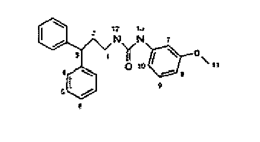 | 361: [MH]⁺ | 2.28 (m, 2H, H₂), 3.22 (m, 2H, H₁, 3.78 (s, 3H, H₁₁), 3.96 (t, 1H, H₃), 4.73 (t, 1H, H₁₂), 6.22 (s, 1H, H₁₃), 6.63 (dd, 1H, H₈ or H₁₀), 6.72 (dd, 1H, H₈ or H₁₀), 6.92 (s, 1H, H₇), 7.12 to 7.30 (m, 11H, Hₐᵣ). |
| | | |
| **EXAMPLE 4:** 1-(3,3-di-p-totyl-propyl)-3-(3-methoxy-phenyl)-urea | | |
| | | |
| 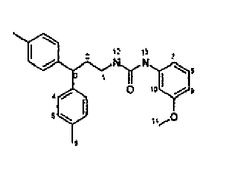 | 389: [MH]⁺ 411: [M+Na]⁺ 799: [2M + Na]⁺ | 2.13 (m, 2H, H₂), 2.23 (s, 6H, H₆), 2.94 (m, 2H, H₁), 3.69 (s, 3H, H₁₁), 3.89 (t, 1H, H₃), 6.16 (m, 1H, H₈), 6.45 (m, 1H, H₉), 6.83 (m, 1H, H₇), 7.08 (m, 5H, Hₐᵣ and H₁₂), 7.11 (m, 1H, H₁₀), 7.16 (d, 4H, Hₐᵣ), 8.41 (s, 1H, H₁₃). |
| | | |
| **EXAMPLE 5:** 3-[3-(3,3-di-p-tolyl-propyl)-ureido]-benzoic acid methyl ester | | |
| | | |
| 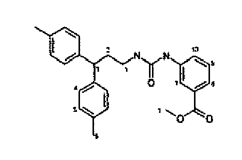 | 417: [MH]⁺ 439: [M + Na]⁺ | 2.12 (m, 2H, H₂), 2.25 (s, 6H, H₆), 3.00 (m, 2H, H₁), 3.82 (s, 3H, H₁₁), 3.98 (t, 1H, H₃), 6.19 (m, 1H, H₈), 6.45 (m, 1H, H₉)_{,} 6.88 (m, 1H, H₇), 7.09 (m, 4H, Hₐᵣ), 7.14 (m, 1H, H₁₀), 7.16 (d, 4H, Hₐᵣ). |
| | | |
| **EXAMPLE 6:** 3-{3-[3,3-bis-(4-methoxy-phenyl)-propyl]-ureido}-benzoic acid methyl ester | | |
| | | |
| 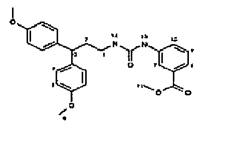 | 449: [MH]⁺ | 2.12 (q, 2H, H₂), 2.97 (m, 2H, H₁), 3.70 (s, 6H, H₆), 3.88 (m, 4H, H₃ and H₁₁), 6.26 (t, 1H, H₁₂), 6.84 (d, 4H, H₅), 7.20 (d, 4H, H₄), 7.3 5 (m, 1H, H₉), 7.47 (d, 1H, H₈ or H₁₀), 7.56 (d, 1H, H₈ or H₁₀), 8.00 (s, 1H, H₇), 8.73 (s, 1H, H₁₃). |
| | | |
| **EXAMPLE 7:** 3-{3-[2-(9H-fluoren-9-yl)-ethyl]-ureido}-benzoic acid methyl ester | | |
| | | |
| 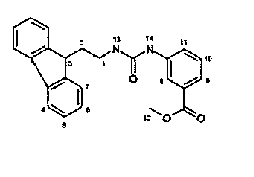 | 387: [MH]⁺ 409: [M + Na]⁺ 355: [MH]⁺ -CH₃OH 210: [MH]⁺ - (CO-NH-Ph-CO₂Me) | 2.10 (q, 2H, H₂), 3.06 (m, 2H, H₁), 3.82 (s, 3H, H₁₂), 4.05 (t, 1H, H₃), 6.33 (t, 1H, H₁₃), 7.33 (m, 3H, H₆ and H₁₀), 7.40 (m, 2H, H₅), 7.47 (d, 1H, H₉ or H₁₁), 7.55 (d, 1H, H₉ or H₁₁), 7.63 (d, 2H, H₇), 7.87 (d, 1H, H₄), 8.10 (s, 1H, H₈), 8.78 (s, 1H, H₁₄). |
| | | |
| **EXAMPLE 8:** 3-{3-[3,3-bis-(3-trifluoromethyl-phenyl)-propyl]-ureido}-benzoic acid methyl ester | | |
| | | |
| 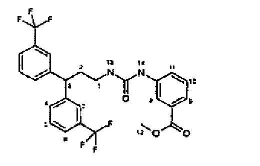 | 525: [MH]⁺ 1049: [2M + H]⁺ | 2.31 (q, 2H, H₂), 3.00 (m, 2H, H₁), 3.83 (s, 3H, H₁₂), 4.33 (t, 1H, H₃), 6.31 (t, 1H, H₁₃), 7.35 (m, 1H, H₁₁), 7.46 (m, 1H, H₁₀), 7.56 (m, 5H, Hₐᵣ), 7.71 (m, 4H, Hₐᵣ), 8.09 (s, 1H, H₈), 8.76 (s, 1H, H₁₄). |
| | | |
| **EXAMPLE 9:** 4-[3-(3,3-diphenyl-propyl)-ureido]-benzoic acid ethyl ester | | |
| | | |
| 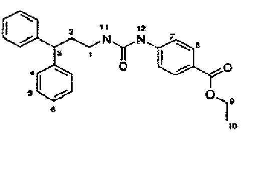 | 403: [MH]⁺ 425: [M + Na]⁺ 805: [2M + H]⁺ 827: [2M + Na]⁺ 7.30 | 1.38 (t, 3H, H₁₀), 2.32 (q, 2H, H₂), 3.25 (q, 2H, H1), 3.98 (t, 1H, H₃), 4.35 (q, 2H, H₉), 4.65 (t, 1H, H₁₁), 6.35 (s, 1H, H₁₂), 7.13 to 7.30 (m, 10H, Hₐᵣ), 7.32 (d, 2H, H₇), 7.95 (d, 2H, H₈). |
| | | |
| **EXAMPLE 10:** 1-(2-chloro-phenyl)-3-(3,3-diphenyl-propyl)-urea | | |
| | | |
| 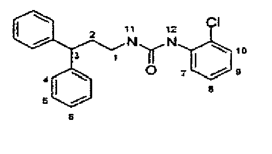 | 365: [MH]⁺ 387: [M + Na]⁺ 751: [2M + Na]⁺ 212: [(Ph₂CH-(CH₂)₂-NH]⁺ | 2.35 (q, 2H, H₂), 3.29 (q, 2H, H₁), 4.02 (t, 1H, H₃), 4.48 (t, 1H, H₁₁), 6.50 (sl, 1H, H₁₂), 6.98 (m, 1H, H₈), 7.13 to 7.35 (m, 12H, Hₐᵣ), 8.06 (d, 1H, H₁₀). |
| | | |
| **EXAMPLE 11:** 1-(3,3-diphenyl-propyl)-3-naphthalen-1-yl-urea | | |
| | | |
| 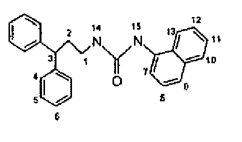 | 381: [MH]⁺ 403: [M + Na]⁺ 761: [2M + H]⁺ 783: [2M + Na]+ 212: [(Ph₂CH-(CH₂)₂-NH]⁺ | 2.22 (q, 2H, H₂), 3.18 (q, 2H, H₁), 3.83 (t, 1H, H₃), 4.48 (t, 1H, H₁₄), 6.28 (sl, 1H, H₁₅), 7.15 to 7.30 (m, 10H, H₄ to H₆), 7.42 to 7.80 (m, 3H, H₇ to H₉), 7.52 and 7.56 (m, 2H, H₁₁ and H₁₂), 7.90 and 8.05 (m, 2H, H₁₀ and H₁₃). |
| | | |
| **EXAMPLE 12:** 1-(4-chloro-phenyl)-3-(3,3-diphenyl-propyl)-urea | | |
| | | |
| 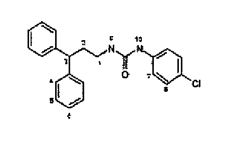 | 365: [MH]⁺ 387: [M + Na]⁺ 212: [(Ph₂CH-(CH₂)₂-NH]⁺ | (q,2H, H₂), 2.98 (q, 2H, H₁), 3.99 (t, 1H, H₃), 6.24 (t, 1H, H₉), 7.16 (m, 2H, Hₐᵣ), 7.23 (m, 2H, H₇ or H₈), 7.25 to 7.35 (m, 8H, Hₐᵣ), 7.40 (d, 2H, H₇ or H₈), 8.56 (s, 1H, H₁₀). |
| | | |
| **EXAMPLE 13:** 1-(4-bromo-phenyl)-3-(3,3-diphenyl-propyl)-urea | | |
| | | |
| 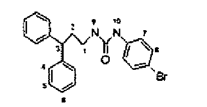 | 409: [MH]⁺ | 2.20 (q, 2H, H₂), 2.97 (q, 2H, H₁), 3.99 (t, 1H, H₃), 6.26 (t, 1H, H₉), 7.12 to 7.34 (m, 10H, Hₐᵣ), 7.36 (m, 4H, H₇ and H₈), 8.60 (s, 1H, H₁₀). |
| | | |
| **EXAMPLE 14:** 1-(3-chloro-phenyl)-3-(3,3-diphenyl-propyl)-urea | | |
| | | |
| 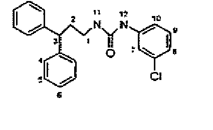 | 365: [MH]⁺ | 2.20 (q, 2H, H₂), 2.98 (q, 2H, H₁, 3.99 (t, 1H, H₃), 6.30 (t, 1H, H₁₁), 6.91 (d, 2H, Hₐᵣ), 7.15 to 7.32 (m, 12H, Hₐᵣ), 7.65 (s, 1H, H₇), 8.66 (s, 1H, H₁₂). |
| | | |
| **EXAMPLE 15:** 1-(3,3-diphenyl-propyl)-3-(4-phenoxy-phenyl)-urea | | |
| | | |
| 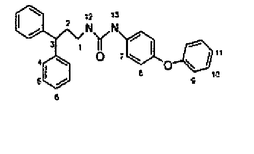 | 423 : [MH]⁺ 445: [M + Na]⁺ | 2.20 (q, 2H, H₂), 2.98 (q, 2H, H₁), 4.00 (t, 1H, H₃), 6.18(t, 1H, H₁₂), 6.91 (m, 4H, H₈ and H₉), 6.96 (m, 1H, H₁₁), 7.17 (m, 2 H, H₁₀), 7.38 (d, 2H, H₇), 7.25 to 7.40 (m, 10H, Hₐᵣ), 8.43 (s, 1H, H₁₃). |
| | | |
| **EXAMPLE 16:** 1-(3,3-diphenyl-propyl)-3-(4-methoxy-benzyl)-urea | | |
| | | |
| 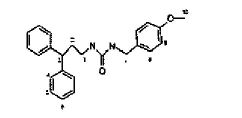 | 375: [MH]⁺ 397: [M + Na]⁺ 771: [2M + Na]⁺ | 2.16 (m, 2H, H₂), 2.90 (m, 2H, H₁), 3.72 (s, 3H, H₁₀), 3.95 (t, 1H, H₃), 4.10 (d, 2H, H₇), 5.94 (t, 1H, H_{mob}), 6.20 (t, 1H, H_{mob}), 6.85 to 7.27 (m, 14H, Hₐᵣ). |
| | | |
| **EXAMPLE 17:** (R) - 1-(3,3-diphenyl-propyl)-3-(1-phenyl-ethyl)-urea | | |
| | | |
| 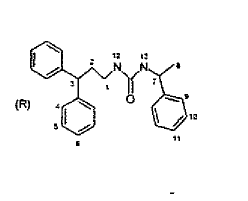 | 359: [MH]⁺ 381: [M + Na]⁺ 212: [(Ph₂CH-(CH₂)₂-NH]⁺ 255: [MH]⁺ -(CH(CH₃)Ph) | 1.29 (d, 3H, H₈), 2.10 (m, 2H, H₂), 2.85 (m, 2H, H₁), 3.93 (t, 1H, H₃), 4.71 (m, 1H, H₇), 5.84 (t, 1H, H₁₂), 6.28 (d, 1H, H₁₃), 7.15 to 7.50 (m, 15 H, Hₐᵣ). |
| | | |
| **EXAMPLE 18:** 1-(3,3-diphenyl-propyl)-3-(2-methylsulphanyl-phenyl)-urea | | |
| | | |
| 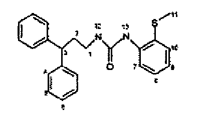 | 377: [MH]⁺ 399: [M + Na]⁺ 212: [(Ph₂CH-(CH₂)₂-NH]⁺ | 2.20 (m, 2H, H₂), 2.40 (s, 1H, H₁₁), 2.97 (m, 2H, H₁), 4.00 (t, 1H, H₃), 6.20 (t, 1H, H₁₂), 7.16 (m, 4H, Hₐᵣ), 7.30 (m, 10H, Hₐᵣ), 8.45 (s, 1 H, H₁₃). |

### EXAMPLE 19

### 1-(3,3-diphenyl-propyl)-3-(4-methoxy-phenyl)-urea

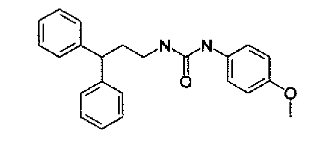

**¹H NMR (400 MHz, CDCl₃)** δ.30 to 7.14 (m, 10H, aromatic H), 7.11 (d, 2H, aromatic H), 6.86 (d, 2H, aromatic H), 6.00 (sl, 1H, NH-Ph-OMe), 4.53 (tl, 1H, CO-NH-CH₂), 3.93 (t, 1H, CH), 3.80 (s, 3H, OCH₃), 3.20 (ql, 2H, CH₂N), 2.27 (ql, 2H, CH₂) **MS (EI):** 360⁺ [M]⁺

### EXAMPLE 20

### 1-(3,3-diphenyl-p ropyl)-3- p-tolyl-urea

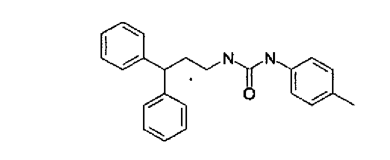

**¹H NMR (400 MHz, CDCl₃)** δ.31 to 7.04 (m, 14H, aromatic H), 6.20 (sl, 1H, NH), 3.94 (t, 1H, CH), 3.21 (m. 2H, CH₂N), 2.32 (s, 3H, CH₃), 2.27 (m, 2H, CH₂) **MS (Electrospray):** 345⁺ [M+H]⁺

### EXAMPLE 21

### 1-(2-chloro-benzyl)-3-(3,3-diphenyl-propyl)-urea

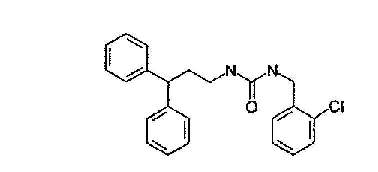

**¹H NMR (300 MHz, CDCl₃)** δ 7.43 to 7.12 (m, 14H, aromatic H), 4.40 (sl, 2H, NH-CH₂ - Ph), 3.94 (tl, 1H, CH), 3.14 (tl, 2H, CH₂CH₂N), 2.26 (ql, 2H, CH₂)
**MS (Electrospray):** 379⁺ and 381⁺ [M+H]⁺, 757⁺ [2M+H]⁺

### EXAMPLE 22

### 3-[3-(3,3-diphenyl-propyl)-ureido]-benzoic acid methyl ester

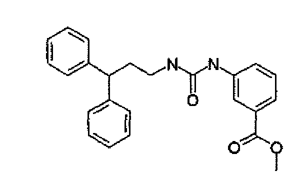

**¹H NMR (300 MHz, CDCl₃)** δ 7.81 (dd, 1H, aromatic H), 7.73 (ddd, 1H, aromatic H), 7.61 (ddd, 1H, aromatic H), 7.36 (t, 1H, aromatic H), (7.31 to 7.13 (m, 10H, aromatic H), 6.38 (sl, 1H, NH), 3.97 (t, 1H, CH), 3.89 (s, 3H, OCH₃), 3.25 (m, 2H, CH₂N), 2.31 (m, 2H, CH₂)
**MS (EI (70eV)):** 388⁺ [M]⁺

### ureas of formula (III) synthesised using method B with 2,2-diphenyl-ethylamine:

### EXAMPLE 23

### 1-(2,2-diphenyl-ethyl)-3-(4-methoxy-phenyl)-urea

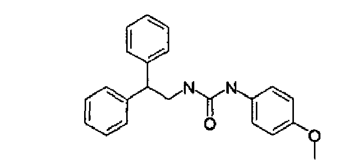

**¹H NMR (300 MHz, CDCl₃)** δ 7.34 to 7.17 (m, 10H, aromatic H), 6.88 (d, 2H, aromatic H), 6.73 (d, 2H, aromatic H), 5.94(s, 1H, CO-NH-Ph), 4.54 (t1, 1H, CH₂-NH , 4.21 (t, 1H, CH), 3.85 (dd, 2H, CH₂N), 3.77 (s, 3H, OCH₃)
**MS (EI):** 346⁺ [M]⁺

### EXAMPLE 24

### 1-(2,2-diphenyl-ethyl)-3-(3-methoxy-phenyl)-urea

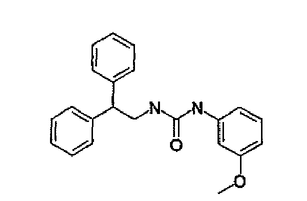

**H NMR (300 MHz, CDCl₃)** δ 7.34 to 7.18 (m, 10H, aromatic H), 7.10 (t, 1H, aromatic H), 6.81 (dd, 1H, aromatic H), 6.60 (dd, 1H, aromatic H), 6.56(dd, 1H, aromatic H), 6-14 (s1, 1H, CO-NH-Ph), 4.73 (tl, 1H, CH2-NH), 4.23 (t, 1H, CH), 3.89 (dd, 2H, CH₂N), 3.73
(s, 3H, OCH₃)
**MS (EI):** 346⁺ [M]⁺

### EXAMPLE 25

### 1-(2-chloro-benzyl)-3-(2,2-diphenyl-ethyl)-urea

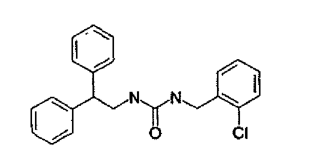

**H NMR (300 MHz, CDCl₃)** δ 7.37 to 7.12 (m, 14H, aromatic H), 4.38 (sl, 2H, NH-CH₂-Ph), 4.17 (tl, 1H, CH), 3.83 (d, 2H, CH₂CH)
**MS (Electrospray):** 365⁺ [M+H]⁺, 387⁺ [M+Na]⁺

### EXAMPLE 26

### 3-[3-(2,2-diphenyl-ethyl)-ureido]-benzoic acid methyl ester

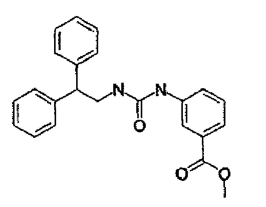

**H NMR (300 MHz, CDCl₃)** δ 7.78 (sl, 1H, aromatic H), 7.70 (dd, 1H, aromatic H), 7.49 (dd, 1H, aromatic H), 7.37 to 7.16 (m, 11H, aromatic H), 6.40 (sl, 1H, NH), 4.23 (tl, 1H, CH), 3.90 (d, 2H, CH₂-CH), 3.88 (s, 3H, OCH₃)
**MS (Electrospray):** 375⁺ [M+H]⁺ 397⁺ [M+Na]⁺, 749⁺ [2M+H]⁺

### Method C: synthesis of ureas of formula (III) after intermediate formation of the isocyanate of_formula (VII)

### The synthesis of ureas of formula (III) after intermediate formation of 3,3'-diphenylpropyl isocyanate of formula (VII) in which R1= phenyl and R'1= phenyl was described by way of example.

2.11 g of 3,3'-diphenylpropylamine (10 mmol, 1 eq) in solution in 60 mL of toluene, 100 mg of activated carbon and 905 µL of diphosgene (7.5 mmol, 0.75 eq) were introduced into a 250 mL flask under N₂.

The reaction mixture was heated under reflux for 5 hours. After confirmation of the formation of the isocyanate by IR (V_{N=C=O} = 2280 cm⁻¹), the crude reaction product was filtered over Clarcel and concentrated to dryness.

The medium was then taken up by dichloromethane and subjected to simultaneous synthesis:
0.5 mmol (1 eq) of amine of formula (V) was introduced into test tubes, then 0.75 mmol (1.5 eq) of 3,3'-diphenylpropyl isocyanate of formula (VII) in solution in 8 mL of CH₂Cl₂. The reaction medium was stirred for 1 night. After washing with water, the organic phases were dried over magnesium sulphate and evaporated.

The products were recrystallised in diethyl ether or subjected to chromatography over silica gel with an eluant: CH₂Cl₂/AcOEt - 90/10.

The average yield over two stages was 48 %.

### Ureas of formula (III) obtained by method C:

| **Structure** | **MS (Electrospray)** | **NMR (300 MHz, CDCl3)** |
|---|---|---|
| | | |
| **EXAMPLE 27:** 1-(3,3-diphenyl-propyl)-3-quinolin-2-yl-urea | | |
| | | |
| 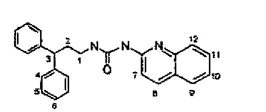 | 382: [MH]⁺ | 2.36 (m, 2H, H₂), 3.18 (m, 2H, H₁), 4.08 (t, 1H, H₃), 7.18 (m, 2H, H₆), 7.30 (m, 4H, H₅), 7.36 (m, 4H, H₅), 7.42 to 7.82 (m, 6H, Hₐᵣ). |
| | | |
| **EXAMPLE 28:** 1-(3,3-diphenyl-propyl)-3-(4-morpholin-4-yl-phenyl)-urea | | |
| | | |
| 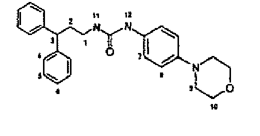 | 416: [MH]⁺ | 2.19 (m, 2H, H₂), 2.96 (m, 2H, H₁), 2.98 (t, 4H, H₉), 3.71 (t, 4H, H₁₀), 3.99 (t, 1H, H₃), 6.05 (t, 1H, H₁₁ 6.81 (d, 2H, H₈), 7.22 (d, 2H, H₇), 7.25 to 7.35 (m, 10H, Hₐᵣ), 8.13 (s, 1H, H₁₂). |
| | | |
| **EXAMPLE 29:** 1-(3,3-diphenyl-propyl)-3-(5-methyl-1H-pyrazol-3-yl)-urea | | |
| | | |
| 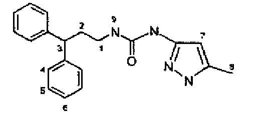 335: | 335: [MH]⁺ | 2.03 (s, 3H, H₈), 2.28 (m, 2H, H₂), 3.09 (m, 2H, H₁), 3.98 (t, 1H, H₃), 5.13 (s, 1H, H₇), 6.28 (s, 1H, H_{mob}), 7.16 (m, 2H, H₆), 7.28 (m, 4H, H₅), 7.32 (m, 4H, H₄), 7.95 (t, 1H, H₉). |
| | | |
| **EXAMPLE 30:** 3-[3-(3,3-diphenyl-propyl)-ureido]-thiophene-2-carboxylic acid methyl ester | | |
| | | |
| 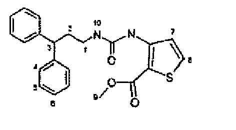 | 395: [MH]⁺ 417:[M+Na]⁺ | 2.21 (m, 2H, H₂), 2.98 (m, 2H, H₁), 3.81 (s, 3H, H₉), 4.03 (t, 1H, H₃), 7.17 (m, 2H, H₆), 7.25 to 7.35 (m, 8H, H₄ H₅), 7.71 (t, 1H, H₁₀), 7.75 (d, 1H, Hₐᵣ), 7.91 (d, 1H, Hₐᵣ). |
| | | |
| **EXAMPLE 31:** 1-(3,3-diphenyl-propyl)-3-(9H-fluoren-2-yl)-urea | | |
| | | |
| 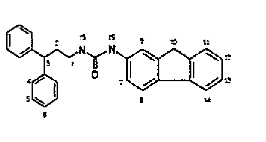 | 419: [MH]⁺ 441:[M+Na]⁺ | 2.22 (m, 2H, H₂), 3.00 (m, 2H, H₁), 3.84 (s, 2H, 4.01 (t, 1H, H₃), 6.23 (t, 1H, H₁₅), 7.18 (m, 2H, H₆), 7.21 (m, 2H, H₁₂ H₁₃), 7.30 (m, 9H, Hₐᵣ), 7.51 (m, 1H, Hₐᵣ), 7.70 (m, 1H, Hₐᵣ), 7.72 (s, 1H, H₉), 7.75 (m, 1H, Hₐᵣ), 8.51 (s, 1H, H₁₆). |
| | | |
| **EXAMPLE 32:** 1-(3,3-diphenyl-propyl)-3-(2-phenylsulphanyl-phenyl)-urea | | |
| | | |
| 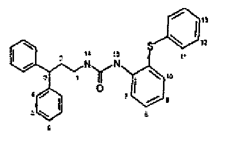 | 439: [MH]⁺ 877: [2M + H]⁺ 200: [NH-Ph-S-Ph]⁺ | 2.15 (m, 2H, H₂), 2.92 (m, 2H, H₁), 3.95 (t, 1H, H₃), 6.98 (m, 1H, Hₐᵣ), 7.09 (m, 2H, H₁₁), 7.16 (m, 4H, Hₐᵣ and H₁₄), 7.27 (m, 10H, Hₐᵣ), 7.35 (m, 1H, Hₐᵣ), 7.44 (d, 1H, Hₐᵣ), 8.10 (d, 1H, H₁₅), 8.16 (d, 1H, Hₐᵣ). |
| | | |
| **EXAMPLE 33:** 1-(4-chloro-3-trifluoromethyl-phenyl)-3-(3,3-diphenyl-propyl)-urea | | |
| | | |
| 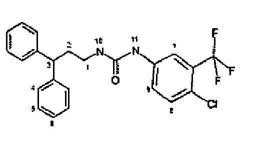 | 433: [MH]⁺ | 2.11 (m, 2H, H₂), 2.99 (m, 2H, H₁), 3.99 (t, 1H, H₃), 6.41 (t, 1H, H₁₀), 7.17 (m; 2H, H₆), 7.28 (m, 4H, H₅), 7.32 (m, 4H, H₄), 7.52 (d, 1H, H₈), 7.56 (d, 1H, H₉), 8.03 (s, 1H, H₇), 8.96 (s, 1H, H₁₁). |
| | | |
| **EXAMPLE 34:** 1-(2-chloro-pyridin-3-yl)-3-(3,3-diphenyl-propyl)-urea | | |
| | | |
| 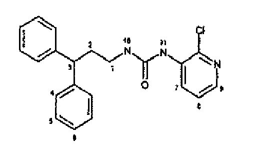 | 366: [MH]⁺ 330: [MH]⁺ - HCl 731: [2M + H]⁺ | 2.22 (m, 2H, H₂), 3.00 (m, 2H, H₁), 4.02 (t, 1H, H₃), 7.17 (m, 3H, H₆ and H₁₀), 7.29 (m, 4H, H₅), 7.32 (m, 5H, H₄ and H₈), 7.95 (d, 1H, H₉), 8.12 (s, 1H, H₁₁), 8.49 (d, 1H, H₇). |
| | | |
| **EXAMPLE 35:** 1-(3,3-diphenyl-propyl)-3-pyridin-2-yl-urea | | |
| | | |
| 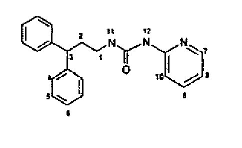 | 332: [MH]⁺ 212: [(Ph₂CH-(CH₂)₂-NH]⁺ | 2.25 (m, 2H, H₂), 3.07 (m, 2H, H₁), 4.01 (t, 1H, H₃), 6.91 (m, 1H, H₈), 7.17 (t, 2H, H₆), 7.29 (m, 5H, H₅ and H₁₀), 7.34 (m, 4H, H₄), 7.65 (m, 1H, H₉), 8.19 (d, 1H, H₇), 8.30 (t, 1H, H₁₁), 9.17 (s, 1H, H₁₂). |
| | | |
| **EXAMPLE 36:** 1 -biphenyl-3-yl-3-(3 ,3 -diphenyl-propyl)-urea | | |
| | | |
| 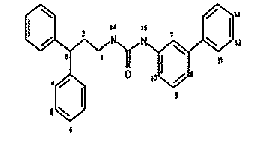 | 407: [MH]⁺ | 2.22 (q, 2H, H₂), 3.00 (m, 2H, H₁), 4.01 (t, 1H, H₃), 6.28 (t, 1H, H₁₄), 7.17 to 7.40 (m, 14H, Hₐᵣ), 7.45 (m, 2H, H₁₂), 7.58 (d, 2 H, H₁₁). 7.72 (s, 1H, H₇), 8.56 (s, 1H, H₁₅). |
| | | |
| **EXAMPLE 37:** 4-chloro-3-[3-(3,3-diphenyl-propyl)-ureido]-benzoic acid methyl ester | | |
| | | |
| 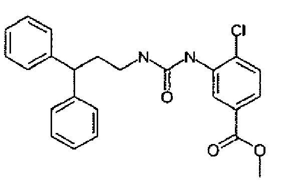 | 423: [MH]⁺ 445: [M+Na]⁺ 845: [2M+H]⁺ | 8.80 (s, 1H, aromatic H), 8.35 to 8.25 (m, 10H, aromatic H), 8.16 (s, 1H, NH), 7.55 (m, 1H, aromatic H), 7.50 (m, 1H, aromatic H), 7.17 (t, 1H, NH), 4.03 (t, 1H, CH), 3.83 (s, 3H, OCH₃), 3.01 (q, 2H, CH₂N), 2.22 (q, 2H, CH₂) |
| | | |
| **EXAMPLE 38:** 2-chloro-5-[3-(3,3-diphenyl-propyl)-ureido]-benzoic acid methyl ester | | |
| | | |
| 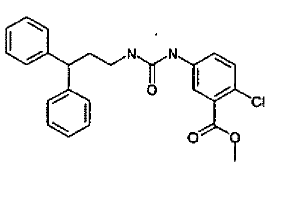 | 423: [MH]⁺ 445: [M+Na]⁺ 845: [2M+H]⁺ | 8.82 (s, 1H, NH), 7.93 (s, 1H, aromatic H), 7.48 (m, 1H, aromatic H), 7.38 (m, 1H, aromatic H), 7.29 (m, 8H, aromatic H), 7.16 (m, 2H, aromatic H), 6.34 (t, 1H, NH), 3.97 (t, 1H, CH), 3.83 (s, 3H, OCH₃), 2.96 (q, 2H, CH₂N), 2.20 (q, 2H, CH₂) |
| | | |
| **EXAMPLE 39:** 3-[3-(3,3-diphenyl-propyl)-ureido]-4-methoxy-benzoic acid methyl ester | | |
| | | |
| 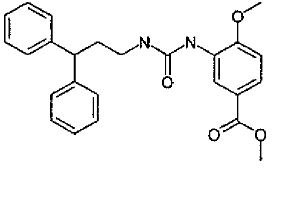 | 419: [MH]⁺ 441: [M+Na]⁺ 837: [2M+H]⁺ . | 8.72 (s, 1H, aromatic H), 7.52 (m, 1H, aromatic. H), 7.30 (m, 8H, aromatic H), 7.16 (m, 2H, aromatic H), 7.04 (m, 1H, aromatic H), 4.00 (t, 1H, CH), 3.90 (s, 3H, OCH₃), 3.72 (s, 3H, OCH₃), 2.96 (q, 2H, CH₂N), 2.20 (m, 2H, CH₂) |
| | | |
| **EXAMPLE 40**: 3-[3-(3,3-diphenyl-propyl)-ureido]-4-methyl-benzoic acid methyl ester | | |
| | | |
| 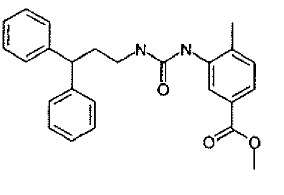 | 403: [MH]⁺ 425: [M+Na]⁺ 805: [2M+H]⁺ | 2.22 (m, 5H, CH₂ and CH₃), 3.00 (q, 2H, CH₂N), 3.80 (s, 3H, OCH₃), 4.00 (t, 1H, CH), 7.16 (m, 2H, aromatic H), 7.25 (m, 1H, aromatic H), 7.30 (m, 8H, aromatic H), 7.45 (m, 1H, aromatic H), 8.50 (s, 1H, aromatic H), |
| | | |
| **EXAMPLE 41**: 5-[3-(3,3-diphenyl-propyt)-ureido]-2-hydroxy-benzoic acid methyl ester | | |
| | | |
| 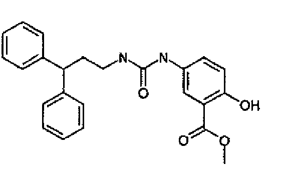 | 405: [MH]⁺ 427: [M+Na]⁺ | 2.20 (q, 2H, CH2), 2.97 (q, 2H, CH₂N), 3.87 (s, 3H, OCH₃), 3.98 (t, 1H, CH), 6.16 (t, 1H, NH), 6.87 (m, 1H, aromatic H), 7.17 to 7.30 (m, 10H, aromatic H), 7.41 (m, 1H, aromatic H), 7.93 (m, 1H, aromatic H), 8.41 (s, 1H, NH), 10.10 (sl, 1H, OH) |
| | | |
| **EXAMPLE 42:** 4-amino-3-[3-(3,3-diphenyl-propyl)-ureido]-benzoic acid methyl ester | | |
| | | |
| 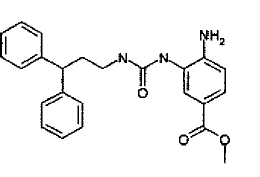 | 404: [MH]⁺ | 2.20 (m, 2H, CH₂), 2.98 (q, 2H, CH₂N), 3.73 (s, 3H, OCH₃), 4.01 (t, 1H, CH), 5.54 (sl, 2H, NH₂), 6.26 (t, 1H, NH), 6.69 (m, 1H, aromatic H), 7.16 to 7.30 (m, 10H, aromatic H), 7.42 (m, 1H, aromatic H), 7.59 (s, 1H, NH), 7.89 (s, 1H, aromatic H) |
| | | |
| **EXAMPLE 43:** 2-chloro-3-[3-(3,3-diphenyl-propyl)-ureido]-benzoic acid methyl ester | | |
| | | |
| 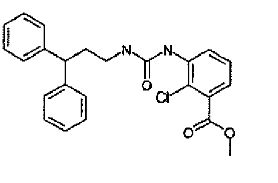 | 423: [MH]⁺ | 2.22 (q, 2H, CH₂), 2.99 (q, 2H, CH₂N), 3.84 (s,! 3H, OCH₃), 4.00 (t, 1H, CH), 7.18 (t, 1H, NH), 7.17 to 7.29 (m, 11H, aromatic H), 7.34 (m, 1H, aromatic H), 8.15 (s, 1H, NH), 8.30 (m, 1H, aromatic H) |

### Preparation of thioureas of formula (III):

This may be achieved by one of the two methods of synthesis described below:

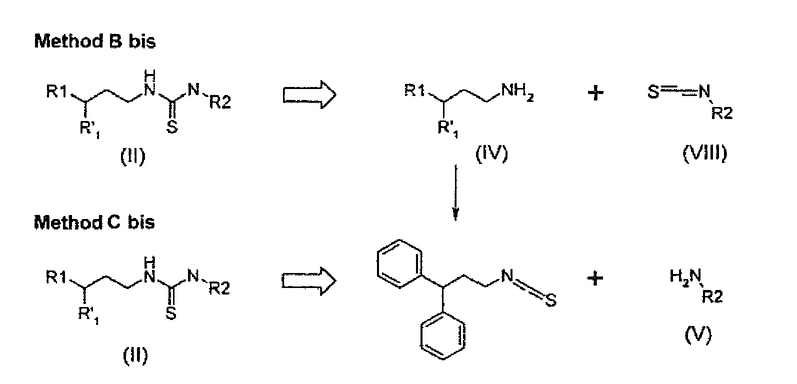

### Method B bis: synthesis of thioureas of formula an starting from commercial aromatic isothiocvanates of formula (VIII)

Same method as method B, replacing the isocyanate of formula (VI) with an isothiocyanate of formula (VIII).

After extraction, the products obtained were recrystallised in methanol or diethyl ether and the expected thioureas were obtained in an average yield of 94 %.

### Thioureas of formula (II) obtained by method B bis:

| **Structure** | **MS (Electrospray)** | **NMR (300 MHz, CDC13)** |
|---|---|---|
| | | |
| **EXAMPLE 44**: 1 -(4-chloro-3-trifluoromethyl-phenyl)-3-(3,3 -diphenyl-propyl)-thiourea | | |
| | | |
| 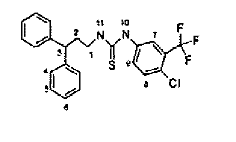 | 449: [MH]⁺ | 2.44 (q, 2H, H₂), 3.69 (m, 2H, H₁), 3.97 (t, 1H, H₃), 5.73 (sl, 1H, H₁₀),7.13 to 7.39 (m, 11H, Har), 7.46 (d, 1H, H₇), 7.52 (d, 1H, H₉). |
| | | |
| **EXAMPLE 45**: 1-(3,3-diph(-,nyi-propyl)-3-(4-methoxy-phenyl)-thiourea | | |
| | | |
| 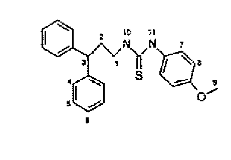 | 377: [MH]⁺ 399: [M + Na]⁺ 775: [2M + Na]⁺ | 2.30 (q, 2H, H₂), 3.35 (masked, 2H, H₁), 3_{.}74 (s, 3H, H₉), 3.98 (t, 1H, H₃), 6.88 (d, 2H, H₈), 7.18 (m, 4H, 144), 7.30 (m, 8H. H₅ to H₇), 7.54 (sl, 1H, H₁₀), 9.26 (sl, 1H, H₁₁). |
| | | |
| **EXAMPLE 46**: 1-(3,3-diphenyl-propyl)-3-(4-methoxy-benzyl)-thiourea | | |
| | | |
| 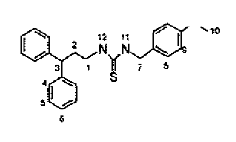 | 391: [MH]⁺ 413: [M + Na]⁺ | 2.30 (q, 2H, H₂), 3.35 (t, 2H, H₁), 3.74 (s. 3H, H₁₀), 3.98 (t, 1H, H₃), 4.21 (s, 2H. H₇), 6.88 (d, 2H, H₉), 7.18 (m, 4H, H₄), 7.30 (m, 8H, Hₐᵣ), 7.54 (sl, 1H, H₁₂), 9.26 (sl, 1H, H₁₁) |
| | | |
| **EXAMPLE 47**: 3-[3-(3,3-diphenyl-propyl)-thioureido]-benzoic acid methyl ester | | |
| | | |
| 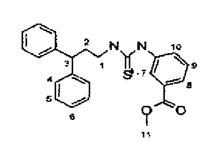 | 405: [MH]⁺ | 2.34 (q, 2H, H₂), 3.36 (t, 2H, H₁), 3.85 (s, 3H, H₁₁), 4.03 (t, 1H, H₃), 7.17 (m, 2H, Har), 7.30 (m, 8H, Hₐᵣ), 7.44 (t, 1H, H₉), 7.67 (m, 2H, H₈ and H₁₀), 7.96 (sl, 1H, H_{mob}), 8.07 (s, 1H, H₇), 9.67 (sl, 1H, H_{mob}) |

### Method C bis: synthesis of thioureas of formula an obtained after intermediate formation of 3,3'-diphenylpropyl isothiocyanate

### The synthesis of [3-(3,3-diphenyl-propyl)-thioureido]-benzoic acid ethyl ester was described by way of example.

422 mg (2 mmol, 1 eq) of 3,3'-diphenylpropylamine in solution in 20 mL of dimethylformamide, then 1.2 mL of CS₂ (20 mmol, 10 eq) and 836µL (6 mmol, 3 eq) of triethylamine were introduced into a 100 mL flask under a nitrogen atmosphere. 884 mg (2 mmol, 1 eq) of BOP were then added to the reaction medium, which was left with stirring for 3 hours at ambient temperature.

The mixture was concentrated to dryness to remove the excess CS₂, then taken up with 50 mL of water. The aqueous phase was extracted with 150 mL of ethyl acetate, and the organic phase was dried over MgSO₄ then concentrated to dryness.

The intermediate 3,3'-diphenylpropyl isothiocyanate obtained was dissolved in 15 mL of dimethylformamide under a nitrogen atmosphere. 2.2 mmol (1.1 eq) of 3-aminobenzoic acid ethyl ester of formula (V) were added and the mixture was left with stirring for 3 hours.

The reaction medium was taken up with 50 mL of water, and the aqueous phase was extracted with 150 mL of ethyl acetate. The organic phase was washed with 40 mL of HCl (1 M) then 40 mL of water, dried over MgS0₄ and concentrated in a rotary evaporator. The product was recrystallised in diethyl ether.

The 3-[3-(3,3-diphenyl-propyl)-thioureido]-benzole acid ethyl ester was obtained in a yield of 40 % in two stages.

### Thiourea of formula (II) obtained by method C bis:

### EXAMPLE 48

### 3-[3-(3,3-diphenyl-propyl)-thioureido]-benzoic acid ethyl ester

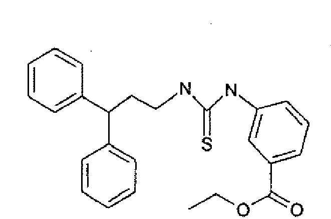

¹H NMR (400 MHz, CDC1₃) δ8.05 (s, 1H, aromatic H), 7.65 (m, 2H, aromatic H), 7.44 (m, 1H, aromatic H), 7.33 to 7.13 (m, 10H, aromatic H), 4.30 (q, 2H, CH₂), 4.02 (t, 1H, CH), 3.29 (q, 2H, CH₂), 2.32 (q, 2H, CH₂), 1.37 (t, 3H, CH₃) MS: 419⁺ [M+H]⁺, 441⁺ [M+Na]⁺, 859⁺ [2M+Na]⁺

### Method D: Synthesis of carboxylic acid analogues, synthesis of ureas and thioureas of formula (III, II, I) by saponification

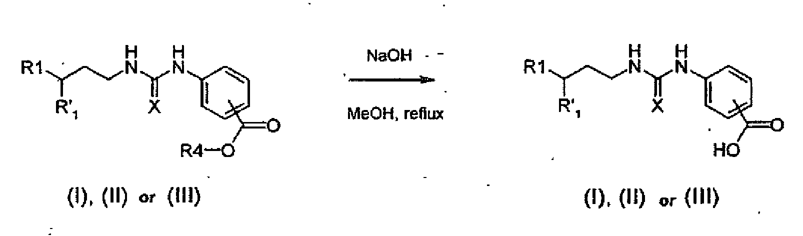

X is O or S, and R4 is H or Me

0.1 mmol of ester (1 eq) in solution in 2 mL of methanol then 1 mmol of 1 M sodium hydroxide solution (10 eq) were introduced into a 10 mL flask equipped with a straight condenser.

The reaction mixture was heated under reflux for 1 hour. It was subsequently concentrated to dryness and taken up with water then 1 N hydrochloric acid. The aqueous phase was extracted with ethyl acetate, then the organic phase was dried over MgSO₄ and concentrated. The expected acids were obtained in an average yield of 80 %.

### Ureas and thioureas of formula (III, II, I) obtained by method D:

| **Structure** | **MS (Electrospray** | **RMN (dmso, 300MHz)** |
|---|---|---|
| | | |
| **EXAMPLE 49**: 4-[3-(3,3-diphenyl-propyl)-ureido]-benzoic acid | | |
| | | |
| 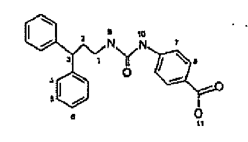 | 375: [MH]⁺ 397: [M + Na]⁺ | 2.10 (era, 2H, H₂), 3.0 (m, 2H, H₁), 4.0 (t, 1H, H₃), 6.38 (t, 1H, H₉), 7.15 to 7.30 (m, 10H, H₄ to H₆), 7.47 to 7.80 (m, 4H, H₇ et H₈), 8.84 (s, 1H, H₁₀), 12.5 (sl, 1H, H₁₁). |
| | | |
| **EXAMPLE 50**: 3-[3-(3,3-diphenyl-propyl)-thioureido]-benzoic acid | | |
| | | |
| 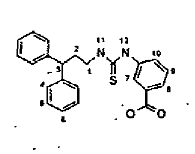 | 391: [MH]⁺ 2.33 | (m, 2H, H₂), 3.37 (m, 2H, H₁), 4.03 (t, 1H, H₃), 7.11 to 7.36 (m, 10H, H₄ to H₆), 7.42 (m, 1H, H₉), 7.65 (m, 2H, H₈ et H₁₀), 7.94 (t, 1H, H₁₁), 8.01 (s, 1H, H₇), 9.65 (s, 1H, H₁₂) |
| | | |
| **COMPARATIVE EXAMPLE 51**: 3-[N'-(3,3-diphenyl-propyl)-guanidino]-benzoic acid | | |
| | | |
| 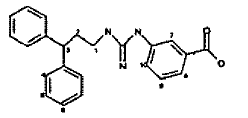 374: | 374: [MH]⁺ | 2.34 (q, 2H, H₂), 3.12 (t, 2H, H₁), 4.10 (t, 1H, H₃), 7.18 to 7.31 (m, 11H, Hₐᵣ), 7.41 (m, 1H, H₁₀), 7.77 (s, 1H, H₇). |
| | | |
| **COMPARATIVE EXAMPLE 52**: 3-[N'-(3,3-diphenyl-propyl)-N"-methyl-guanidino]-benzoic acid | | |
| | | |
| 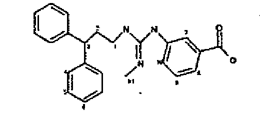 388: | 388: [MH]⁺ | 2.08 (m, 2H, H₂), 2.65 (s, 3H, H₁₁), 3.03 (t, 2H, H₁), 4.01 (t, 1H, H₃), 7.10 to 7.35 (m, 11H, Har). 7.43 (m, 1H, H₈), 7.52 (d, 1H, H₁₀), 7.64 (m, 1H, H₉). |
| | | |
| **EXAMPLE 53**: 3-[3-(3,3-di-p-tolyl-propyl)-ureido]-benzoic acid | | |
| | | |
| 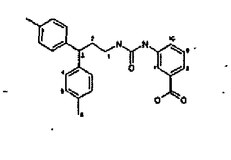 | 503: [MH]⁺ | 2.10 (m, 2H, H₂). 2.26 (s, 6H, H₆), 3.03 (m, 2H, H₁), 4.00 (t, 1H, H₃), 6.19 (m, 1H, H₈), 6.46 (m, 1H, H₉), 6.89 (m, 1H. H₇), 7.00 (m, 4H, Hₐᵣ), 7.16 (m, 5H, H₁₀ et Har). |
| | | |
| **EXAMPLE 54**: 3-{3-[3,3-Bis-(4-methoxy-phenyl)-propyl]-ureido}-benzoic acid | | |
| | | |
| 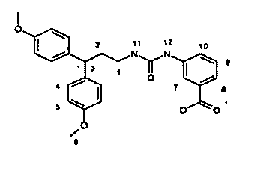 | 435: [MH]⁺ 457: [M + Na]⁺ | 2.12 (q, 2H, H₂), 2.90 (m, 2H, H₁), 3.60 (s, 6H, H₆), 3.88 (t, 1H, H₃), 6.23 (t, 1H, H₁₁), 6.83 (d, 4H, H₅), 7.18 (d, 4H, H₄), 7.27 (m, 1H, H₉), 7.44 (d, 1H, H₈ or H₁₀), 7.56 (d, 1H, H₈ or H₁₀), 7.97 (s, 1H, H₇), 8.62 (s, 1H, H₁₂). |
| | | |
| **EXAMPLE 55**: 3-{3-[3,3-Bis-(3-trifluoromethyl-phenyl)-propyl]-ureido}-benzoic acid | | |
| | | |
| 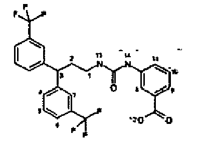 | 511: [MR]⁺ 533: [M +Na]⁺ | 2.31 (m, 2H, H₂), 2.99 (m, 2H, H₁), 4.32 (t, 1H, H₃), 6.26 (t, 1H, H₁₃), 7.31 (t, 1H. H₁₀), 7.45 (d, 1H, H₁₁), 7.55 to 7.73 (m, 7H, Hₐᵣ), 8.01 (5. ,1H, H₈), 8.67 (s, 2H, H₇), i2.78 (sl, 1H, H₁₂). |
| | | |
| **EXAMPLE 56**: 3-[3-(3,3-diphenyl-propyl)-ureido]-benzoic acid | | |
| | | |
| 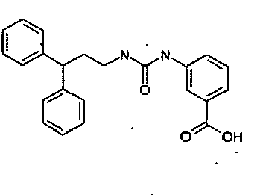 | 375: [MH]⁺ 397: [M + Na]⁺ 416: [MH]⁺ + CH₃CN 749: [2M + H]⁺ | 12.83 (sl, 1H, CO₂H), 8.69 (sl, 1H, NH-Ph), 8.26 (tl, 1H NH-CH₂), 8.03 (sl, 1H, aromatic H), 7.57 (dt, 1H, aromatic H), 7.46 (dl, 1H, aromatic H), 7.39 to 7.10 (m, 11H, aromatic H), 4.00 (tl, 1H, CH), 2.99 (m, 2H, CH₂N), 2.20 (m, 2H, CH2) |

### Method E: Synthesis of a primary alcohol analogue, synthesis of a urea of formula (III) by reduction of ester

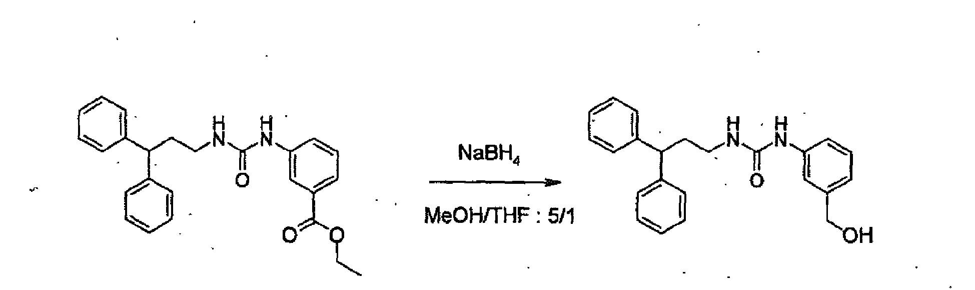

150 mg (0.375 mmol, 1 eq) of 3-[3-(3,3-diphenyl-propyl)-ureido]-benzoic acid ethyl ester in solution in 3 mL of THF then 70.9 mg (1.$75 mmol, 5 eq) of NaBH₄ were introduced into a 20 mL Woulff bottle under N₂. The mixture was brought to reflux and 0.6 mL, of methanol was added dropwise. After 18 hours of reflux, the crude product was concentrated to dryness then taken up with 50 mL of ethyl acetate. After washing with a brine solution, the organic phase was dried over MgS0₄ and concentrated. The product was obtained by purification over a silica column (CH₂Cl₂/MeOH elution: 9/1) with a yield of 21 °r° (m= 29 rng).

### Urea of formula (III) obtained by method E:

| **Structure** | **MS .** | **- NMR (CDCI3)** |
|---|---|---|
| | | |
| **EXAMPLE 57:** 1-(3,3-diphenyl-propyl)-3-(3-hydroxymethyl-phenyl)-urea | | |
| | | |
| 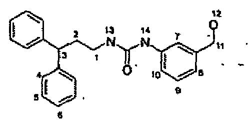 | 361: [MH]⁺ 343: [MH]⁺ -H₂O 383: [M + Na]⁺ 721: [2M + H]⁺ | 2.20 (m, 2H, H₂), 2.98 (m, 2H, H₁), 4.0 (t, 1H H₃), 4.42 (d, 2H, H₁₁), 5.10 (t, 1H, H₁₂), 6.17 (t, 1H, H₁₃), 6.80 to 7.35 (m, 14H, Hₐᵣ), 8.40 (s, 1H. H₁₄). |

### Method F: Synthesis of a brominated derivative, synthesis of a urea of formula (III) by bromination of urea.

### The synthesis of 1-(2-bromo-5-methoxy-phenyl)-3-(3,3-diphenyl-propyl)-urea starting from 1-(3,3-diphenyl-propyl)-3-(3-methoxy-phenyl)-urea was described by way of example.

750 mg (2.08 mmol, 1 eq) of 1-(3,3-diphenyl-propyl)-3-(3-methoxy-phenyl)-urea in solution in 20 mL of acetonitrile (insoluble reagent) were introduced under a nitrogen atmosphere. Then, 389 mg (2.11 mmol, 1.05 eq) of N-bromosuceinimide were added in one batch, and the reaction medium dissolves. The mixture was stirred for 6 hours at ambient temperature. The medium precipitates after one and a half hours of reaction. The acetonitrile was concentrated. The white solid was taken up in diethyl ether and filtered. The solid obtained was purified over a silica column after solid deposition (cyclohexane/ethyl acetate elution: 4/1 to 1/1, m = 600 mg, R= 66%).

### Urea of formula III obtained by method F:

### EXAMPLE 58

### 1-(2-bromo-5-methoxy-phenyl)-3-(3,3-diphenyl-propyl)-urea

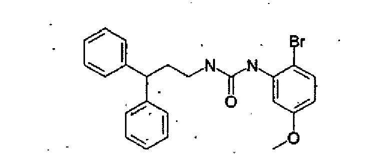

**H NMR (300 MHz, CDCl₃)** δ 37 (d, 1H, aromatic H), 7.31 to 7.13 (m, 10H; aromatic H), 7.22 (masked s, 1H, aromatic H), 6.50 (dd, 1H aromatic H), 6.20 (sl, 1H, CO-NH-Ph), 4.59 (sl, 1H, NH); 3.96 (t, 1H, CH), 3.86 (s, 3H, OCH₃), 3.24 (tl, 2H, CH₂-N), 2.30 (q, 2H, CH₂-CH) .
MS (Electrospray): 439⁺ [M+H]⁺, 480⁺ [M+CH₃CN]⁺, 877⁺ [2M+H]⁺

### 2 ureas synthesised by method C :

### EXAMPLE 59

### 1-(3,3-diphenyl-propyl)-3-(3-oxazol-5-yl-phenyl)-urea

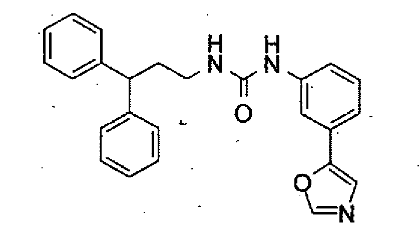

**MS (Electrospray)** : 398⁺ [M+H]⁺
**Rf** : 0.27 (Heptane/AeOEt: 1/1)

### EXAMPLE 60

### 1-benzothiazol-2-yl-3-(3,3-diphenyl-propyl)-urea

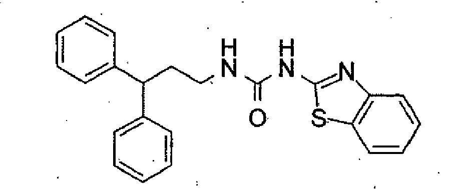

**MS (Electrospray)** : 388⁺ [M+H]⁺ **Rf** : 0.27 (Heptane/ACOEt : 1/1)

### Protocol for Preparation of Selected Compounds of the Invention

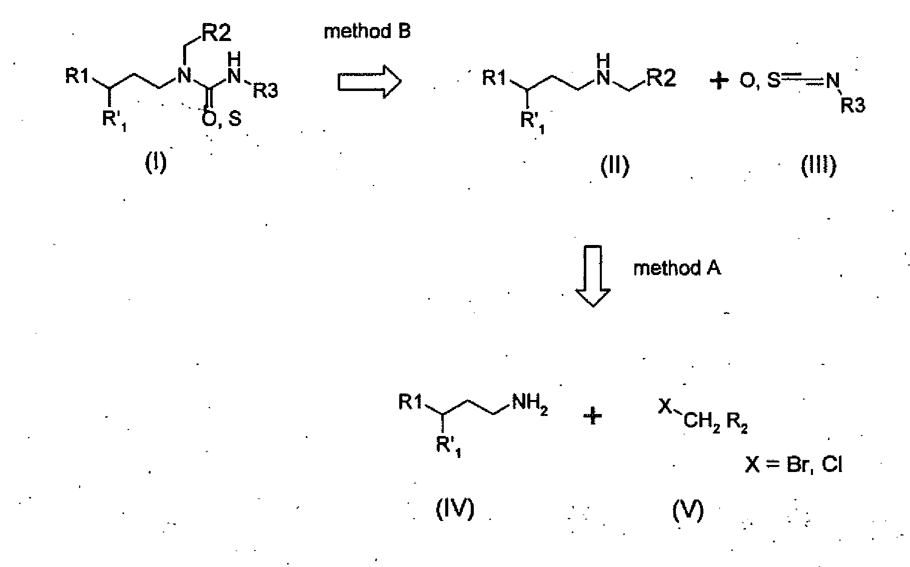

### Preparation of the secondary amines of formula (II):

It can be obtained by the method of synthesis described below:

### alkylatión of the primary amine of formula (IV)

### Method A: .

The alkyl halide (1 mmol, 1 eq) of formula. (V) was dissolved in 40 mL of acetonitrile in a 100 mL flask equipped with a straight condenser, then 1 eq of K₂C03 was added to the medium. The primary amine of formula (IV) in excess (5 mmol, 5 eq) was subsequently added and the medium was heated under reflux for 12 hours.

After evaporation of acetonitrile, the residue was taken up with ethyl acetate. The organic phase was washed with an ammonium chloride solution, then with brine, dried over MgSO₄ and concentrated.

The oil obtained was subjected to chromatography over silica gel (elution gradient: CH₂Cl₂ to CH₂Cl₂/MeOH: 9/1 then CH₂Cl₂/MeOH/NH₃: 9/1/0.1) and the amine of formula (II) was obtained in an average yield of 65 %.

### Amines of formula (II) obtained by method A:

| **Structure** | **MS** | **NMR** |
|---|---|---|
| **Preparatory EXAMPLE 61** | | |
| (3,3-diphenyl-propylamino)-acetic acid tert-butyl ester | | |
| 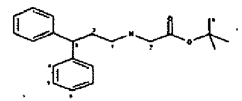 | b₂₆: [MH]⁺ 270: [MH]⁺ - tbu | 1.36 (s, 9H, H₈), 2.12 (q, 2H, H₂), 2.38 (t, 2H, H₁), 3.1.2 (s, 2H, H₇), 4.02 (t, 1H, H₃), 7.15 to 7.35 (m, 10H, Hₐᵣ). |
| **Preparatory EXAMPLE 62** | | |
| 4-(3,3-diphenyl-propylammo)-butync acid ethyl ester | | |
| 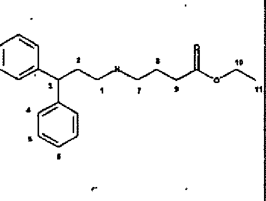 | 326: [MM]⁺ | 1.16 (t, 3H, H₁₁), 1.58 (m, 2H, H₈), 2.10 (q, 2H, H₂), 2.29 (t, 2H, H₁ or H₇ or H₉), 2.35 (t, 2H, H₁ or H₇ or H₉), 2.42 (t, 2H, H₁ or H₇ or H₉), 4.02 (q, 2H, H₁₂), 4.05 (1, 1H, H₃), 7.15 (m, 2H, Har), 7.24 to 7.35 (m, 8H, Hₐᵣ). |

### Preparation of the ureas of formula (I):

### Method B: synthesis of the ureas of formula (1) starting from isocyanates

The amine (0.5 mmol, 1 eq) of formula (II) in solution in 6 mL of dichloromethane, then the isocyanate (0.5 mmol, 1 eq) of formula (III) were introduced into a 100 mL flask under N₂.

After 2 hours of stirring, the reaction mixture was taken up with 60 mL of dichloromethane and washed with 20 mL of water then 20mL of brine. The organic phase was subsequently dried over MgSO₄ and concentrated in a rotary evaporator.

The product was recrystallised in diethyl ether or subjected to chromatography over silica gel with an eluant: CH₂Cl₂/AcOEt - 90/10. The expected ureas were obtained in an average yield of 79%.

### Ureas of formula (I) obtained by method B:

### EXAMPLE 63

Obtained from Preparatory Example 61.

### 3-[3-tert-butoxycarbonylmethyl-3-(3,3-diphenyl-propyl)-ureido]-benzoic acid methyl ester

| | | |
|---|---|---|
| 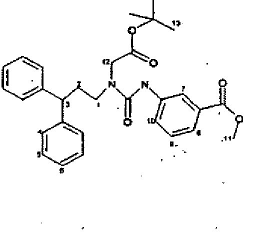 | 503: [MH]⁺ 525: [M + Na]⁺1005: [2M + H]⁺ 447: [MH]⁺ - (tBu) | 1.35 (s, 9H, H₁₃), 2.32 (m, 2H, H₂), 3.23 (m, 2H, H₁), 3.85 (s, 3H, H₁₁), 4.00 (m, 3H; H₃ and H₁₂), 7.16 (m, 2H, 116), 7.27 (m, 4H, H₅), 7.33 (m, 4H, H₄), 7.37 (m, 1H, H₁₀), 7.52 (m, 1H, H₈ or H₁₀), 7.73 (m, 1H, Hs or H₁₀), 8.05 (s, 1H, H₇). |

### EXAMPLE 64

Obtained from Preparatory Example 62.

### 3-[3-(3,3-diphenyl-propyl)-3-(3-ethoxycarbonyl-propyl)-ureido]-benzoic acid methyl ester,

| | | |
|---|---|---|
| 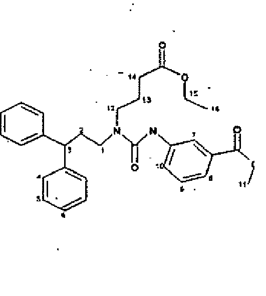 | 503: [MH]⁺ 525: [M + Na]⁺ 1027: [2M + Na]⁺ | 1.13 (t, 3H, H₁₆), 1.69 (m, 2H, H1₃), 2.25 (t, 2H, H₁₄), 2.28 (m, 2H, H₂), 3.20 (t, 2H, H₁ or R₁₂), 3.29 (t, ,2H, H₁ or H₁₂), 3.84 (s, 3H, H₁₁), 3.96 (t, 1H, H₃), 4.01 (q, 2H, H₁₅), 7.16 (m, 2H, H₆), 7.27 (m, 4H, H₅), 7.33 (m, 4H, H₄), 7.36 (m, 1H, H₉), 7.52 (m, 1H, H₈ or H₁₀), 7.76 (m,1H, H₈ or H₁₀), 8.08 (s, 1H, H₇). |

### Method D: Synthesis of carboxylic acid analogs, synthesis of ureas of formula (1) by saponification _{'}

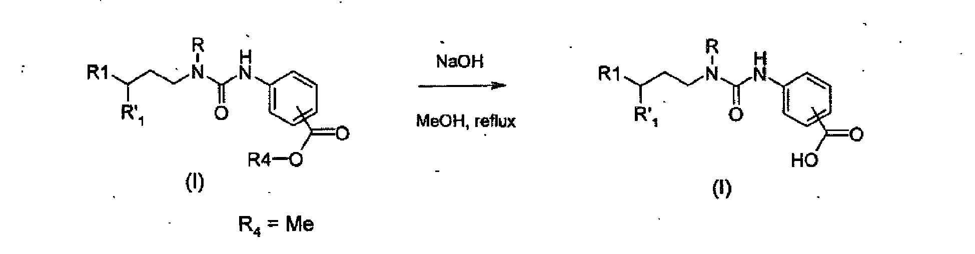

0.1 mmol of ester (1 eq) in solution in 2 mL of methanol then 1 mmol of 1 M sodium hydroxide solution (10 eq) were introduced into a 10 mL flask equipped with a straight condenser.

The reaction mixture was heated under reflux for 1 hour. It was subsequently concentrated to dryness and taken up with water then 1 N hydrochloric acid. The aqueous phase was extracted with ethyl acetate, then the organic phase was dried over MgSO₄ and concentrated. The expected acids were obtained in an average yield of 80 %.

### Ureas of formula tI1 obtained by method D:

### EXAMPLE 65

### Obtained from Example 64.

### 3-[3-(3-carboxy-propyl)-3-(3,3-dipbenyl-prop'yl)-ureido]-benzoic acid

| | | |
|---|---|---|
| 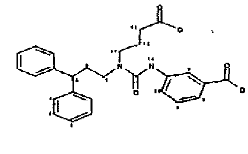 | 461: [MH]⁺ 483: [M + Na]⁺ 921: [2M + H]⁺943*:* [2M+Na]⁺ | 1.67 (m, 2H, H₁₂), 2.20 (t, 2H, H₁₃), 2.30 (m, 2H, H₂), 3.20 (m, 4H, H₁ and H₁₁), 3.97 (t, 1H, H₃), 7.16 (m, 2H, H₆), 7.28 (m, 4H, H₅), 7.34 (m, 4H, H₄), 7.51 (m, 2H, H₉ and H₁₀), 7.73 (m, 1H, H₈), 8.07 (s, 1H, H₇), 8.37 (s, 1H, H₁₄). |

### EXAMPLE 66

Obtained from Example 63.

### 3-[3-carboxymethyl-3-(3,3-diphenyl-propyl)-ureido]-benzoic acid

| | | |
|---|---|---|
| 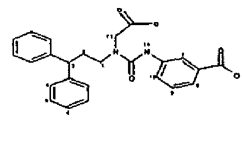 | 433: [MH]⁺ 455:[M + Na]⁺ 865: [2M + H]⁺ | 2.31 (m, 2H, H₂), 3.25 (m, 2H, H₁), 3.99 (s, 1H, H₃), 4.09 (s, 2H, H₁₁), 7.17 (m, 2H, H₆), 7.25 to 7.40 (m, 9H, Hₐᵣ), 7.51 (m, 1H, H₁₀), 7.70 (m, 1H, H₈), 8.03 (s, 1H, H₇). |

### Thiazole Preparation

Thiazoles not commercially available were prepared according to the procedures described in WO 07/1060026. In some cases the 5-chloro thiazole derivative was prepared. Chlorination was achieved by 1 of 2 procedures: either the 5-chloro group was introduced onto the thiazole before urea formation, or else following urea formation. Chlorination of urea products was described individually.

### Chlorination Pre-Urea:

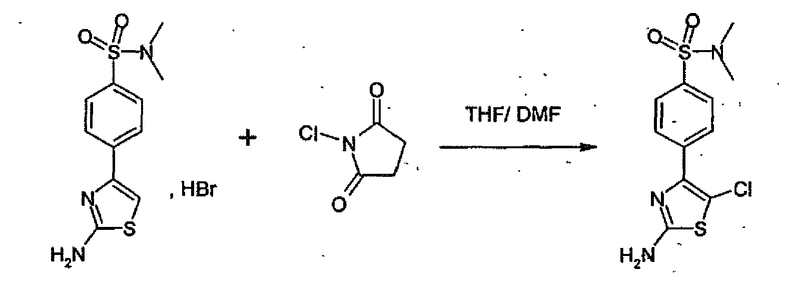

300 mg (1.06 mmol, 1 eq) of the 4-dimethylsulfonamide aminothiazole were dissolved into 4 mL of dry THF (low solubility in THF), and 3 mL of dry DMF then 170 mg (1.27 mmol, 1.2 eq) of NCS were added to the solution mixture. The mixture becomes red within 5 minutes, and was stirred overnight at RT. After evaporation of the solvents, the crude was purified by flash chromatography (DCM/ AcOEt , gradient from 10010 to 3/1) to' give 282 mg (91 % yield) of the desired chloride compound.

### EXAMPLE 67

### (R)-1-(3,3-diphenylpropyl)-3-(4-(4-(methylsulfonamido)phenyl)thiazol-2-yl)-1-(4-oxo-4-(piperidin-3-ylamino)butyl)urea

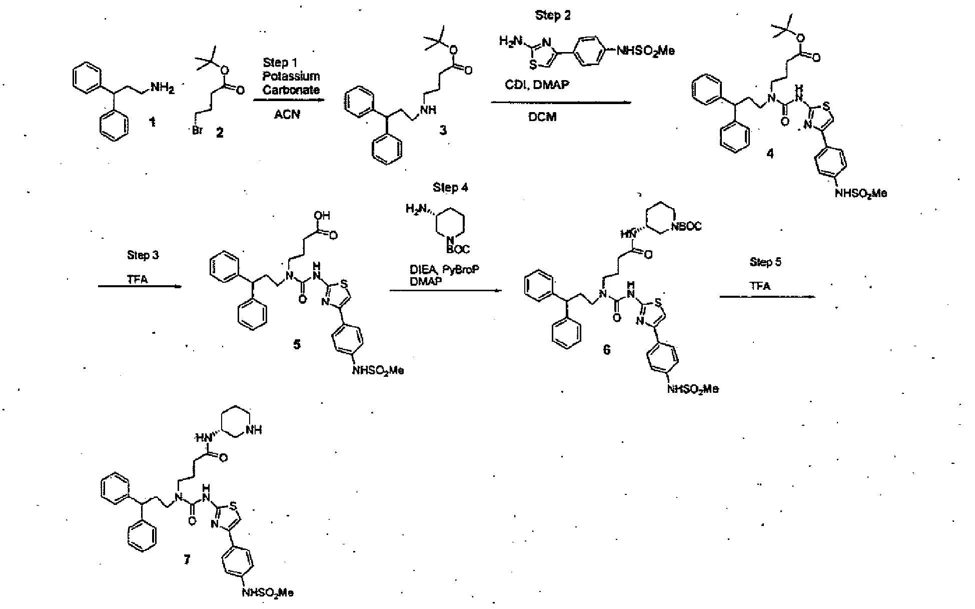

**Step 1:** Tert-butyl 3-bromopropionate 1 (0.5 mL, 3.0 mmol) was dissolved in 100 mL of dry acetonitrile. To this solution was added potassium carbonate (0.415g, 3.0 mmol) and 3,3-diphenylpropylamine **2**. The reaction mixture was refluxed overnight. The mixture was then allowed to cool to room temperature and was concentrated *in vacuo.* The crude residue obtained was dissolved in ethyl acetate (45 mL) and washed with saturated aqueous ammonium chloride solution and then with brine. The organics were dried over magnesium sulfate, filtered and concentrated to obtain the crude product as a clear oil. This product was purified by column chromatography on silica using 89:9:1/DCM:MeOH:ammonium hydroxide as eluent. Fractions containing product were combined and concentrated *in vacuo* to obtain **3** as a white solid (0.54g, 52.6% yield). **Step 2:** To a solution of N-(4-(2-aminothiazol-4-yl)phenyl)methanesulfonamide (0.158g, 0.59 mmol) in 1.5 mL of DCM was added 0.2 mL of DMF. Then added DMAP (0.1 lg, 0.88 mmol) and CDI (0:14g, 0.88 mmol). The reaction mixture was stirred at room temperature for 48h. Tert-butyl 4-(3,3-diphenylpropylamino)butanoate **3**, was then added to the reaction mixture and it was stirred at room temperature for 12h. The reaction mixture was partitioned between ethyl acetate and water. The organic layer was washed with brine, dried over sodium sulfate, filtered and concentrated. This crude product was purified by column chromatography on silica (gradient eluent from SO% ethyl acetate in hexane to 80% ethyl acetate in hexane). Fractions containing product were combined and concentrated to yield the product **4** (0.25g, 67% yield) as a tan solid. **Step 3:** Tert-butyl 4-(1-(3,3-diphenylpropyl)-3-(4-(4-(methylsulfbnamido)phenyl)-thiazol-2-yl)ureido)butanoate **4** (0.097g, 0.15 mmol) was dissolved in 2 mL of trifluoroacetic acid. The reaction mixture was stirred at room temperature for 1 h. The reaction mixture was then concentrated *in vacuo* and purified by RP-HPLC. Fractions containing product were combined and lyophilized to obtain the product **5** as a white solid (0.085g, 98% yield).

LC-MS ESI (neg.) m/e: 591.6 (M-H)

1H NMR (400 MHz, *DMSO-d₆*) δ ppm 10.76 (1 H, s), 9.81 (1 H, s), 7.78 - 7.92 (2 H, m), 7.32 - 7.38 (5 H, m), 7.26 - 7.32 (4 H, m), 7.20 - 7.26 (2 H, m), 7.13 - 7.20 (2 H, m), 4.48 (1 H, s), 4.01 (1 H, t, *J*=7.8 Hz), 3.27 - 3.37 (2 H, m), 3.19 - 3:28 (2 H, m), 3.01 (3 H, s), 2.26 - 2.37 (2 H, m), 2.20 (2 H, t, *J*=7.4 Hz), 1.58-1.73 (2 H, m)

**Step 4**: To a solution of 4-(1-(3,3-diphenylpropyl)-3-(4-(4-(methylsulfonamido)phenyl)thiazol-2-yl)ureido)butanoic acid 5 (0.29g, 0.493 mmol) in dray DMF (10 mL) was added R-3-amino-1-N-Boc-piperzdine (0.197g, 0.986 mmol), DIEA (0.34 mL, 1.97 mmol), DMAP (0.006g, 0.049 mmol) and PyBrOP (0.276g, 0.592 mmol). The reaction mixture was stirred at room temperature overnight, diluted with ethyl acetate (50 mL) and washed with brine. The organic layer was dried over magnesium sulfate, filtered and concentrated to give a yellow oil. This oil was further purfied by column chromatography on silica using ethyl acetate as eluent to give the pure product **6** (0.39g, 55% yield).

**Step 5:** (R)-tert-butyl 3-(4-(1-(3,3-diphenylpropyl)-3-(4-(4-(methylsulfonamido)phenyl)-thiazol-2-yl)ureido)butanamido)piperidine-1-carboxylate **6** (0.246g, 0.318 mmol) was dissolved in 10 mL of DCM. To this solution was added trifluoroacetic acid (2.0 mL) and the reaction was stirred at room temperature for 6h. The reaction mixture was made basic with saturated aqueous sodium bicarbonate solution and extracted with dichloromethane. The organic layer was dried over sodium sulfate, filtered and concentrated to yield a residue that was purified by column chromatography on silica using 89:9: 1 /DCM:MeOH:ammonium hydroxide as eluent to yield the product **7** as white solid (0.15g, 70.5% ly3ieid).

LC-MS ESI (pos.) m/e: 675 (M+H); 1H NMR (400 MHz, CHLOROFORM-*d*) δ ppm 1.40

- 1.50 (m, 1 H) 1.54 - 1.70 (m, 3 H) 1.70 - 1.80 (m, 2 H) 2.11 - 2.19 (m, 2 H) 2.22 - 2.33 (m, *J*=6.65 Hz, 2 H) 2.64 (dd, *J*=12.32, 5.67 Hz, 1 H) 2.69 - 2.77 (m, 2 H) 2.89 (s, 3 H) 2.94 (dd, *J*=11.93, 3.33 Hz, 1 H) 3.13 - 3.24 (m, 2 H) 3.26 - 3.34 (m, 2 H) 3.79 - 3.88 (m, 1 H) 4.00 (br. s., 1 H) 6.35 (br. s., 1 H) 6.90 (s, 1H) 7.07 -7.14 (m, 4 H) 7.15 - 7.24 (m, 12 H) 7.67 (d, *J*=8.21 Hz, 2 H)

### EXAMPLE 68

### 1-(4-amino-4-oxobutyl)-1-(3,3-diphenylpropyl)-3-(4-(4-(methylsulfonamido)-phenyl)thiazol-2-yl)urea

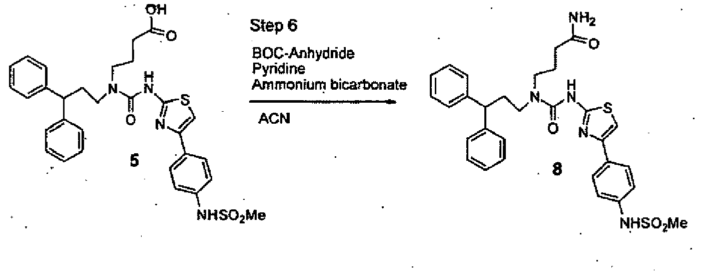

**Step 6**: To a solution of **5** (prepared as described in Example 67) (0.1 g, 0.168 mmol) in dry acetonitrzie.(1.5 mL) was added BOC-anhydride (0.048g, 0.22 mmol), pyridine (0.01 mL) and ammonium bicarbonate (0.017g, 0.22 mmol). The reaction mixture was stirred at room temperature overnight and was then purified by RP-HPLC. Fractions containing product were combined and lyophilized to obtain the product **8** as a white solid (0.070g, 75% yield).

LC-MS ESI (pos.) m/e: 592 (M+H) ¹H NMR (500 MHz, CHLOROFORM-*d*) δ ppm 1.86 - 1.91 (m, 2 H) 2.38 - 2.43 (m, 4 H) 3.07 (s, 3 H) 3.35-3.42 (m, 4 H) 4.0 - 4.1 (m, 1 H) 5.87 (br s, 1 H) 6.81 (br s, 1 H) 6.93 (s, 1 H) 7.16-7.23 (m, 2 H) 7.28-7.32 (m, 9 H) 7.69-7.72(m, 2 H)

### EXAMPLE 69

### 3-(1-(3,3-diphenylpropyl)-3-(4-phenylthiazol-2-yl)ureido)-2-methylpropanoic acid

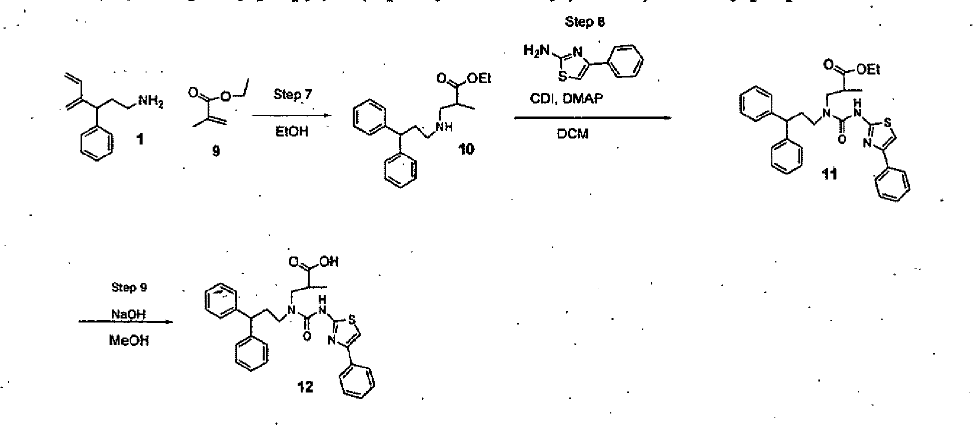

**Step 7:** To a solution of 3,3-diphenyl propylamine **1** (5.3 g, 25 mmol) in 25 mL of ethanol at room temperature was added ethyl methacrylate **9** (3.1 mL, 25 mmol) and the reaction was stirred at room temperature for 96 h. The reaction mixture was concentrated *in vacua* and purified by column chromatography on silica using 89:9:l/DCM:MeOH:ammonium hydroxide as an eluent to yield the product **10** as a colorless oil (5.85g, 72% yield). **Step 8:** The urea coupling between ethyl 3-(3,3-diphenylpropylamino)-2-methylpropanoate **10** and 2-aminophenylthiazole was carried out as described in Example 67, Step 2 and the product was obtained as a tan solid (50.0 mg, 0.095 mmol, 65% yield). **Step 9:** To a solution of ethyl 3-(1-(3,3-diphenylpropyl)-3-(4-phenylthiazol-2-yl)ureido)-2-methylpropanoate **11** (50.0 mg, 0.095 mmol) in 2.0 mL of methanol was added 1 mL of aqueous sodium hydroxide solution (IN). The reaction mixture was heated to reflux overnight, cooled to room temperature, concentrated *in vacuo* and purified by RP-HPLC. Fractions containing the product were combined and lyophilized to yield the product **12** as a white solid (29.5 mg, 62% yield):

LC-MS ESI (neg.) m/e: 498 (M-H)

1H NMR (400 MHz, *DMSO-d₆*) δ ppm 7.84 - 7.92 (2 H, m, *J*=8.4, 1.4 Hz), 7.46 (1 H, s), 7.38 - 7.44 (2 H, m, *J*=7.6, 7.6Hz), 7.31 - 7.37 (4 H, m, *J*=7.4, 7.4 Hz), 7.24 - 7.31 (5 H, m), 7.11 - 7.20 (2 H, run, *J*=7.2, 7.2 Hz), 4.01 (1 H, t, *J*=7.8 Hz), 3.44 (2 H, d, *J*=6.3 Hz), 3.15 - 3.28 (1 H, m), 2.55 - 2.72 (2 H, m), 2.22 - 2.38 (2 H, m), 1.00 (3 H, d, *J*=7.0 Hz)

### EXAMPLE 70

### 1-(3,3-diphenylpropyl)-3-(4-(4-(methylsulfonami4o)phenyl)thiazol-2-yl)-I-(4-oxo-4-(piperidin-4-ylamino)butyl)urea

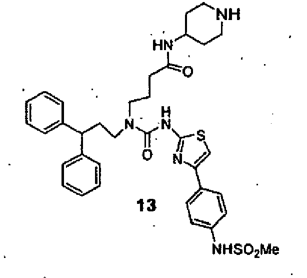

The procedure described in Example 67 was used with the exception of substituting tert-butyl 4-aminopiperidme-l-carboxylate for R-3-amino-1-N-Boc-piperidine in step 4 to prepare 1-(3,3-diphenylpropyl)-3-(4-(4-(methylsulfonamido)phenyl)thiazol-2-yl)-1-(4-oxo-4-(piperidin-4-ylamino)butyl)urea **13** as a white solid.

LC-MS ESI (pos.) m/e: 675 (M+H); 1H NMR (400 MHz, CHLOROFORM-*d*) δ ppm 1.30 -1.42 (m, 2 H) 1.78 - 1.88 (m, 2 H) 1.97 (d, *J*=12.13 Hz, 2 H) 2.15 - 2.25 (m, 2 H) 2.39 (q, *J*=7.69 Hz, 2 H) 2.64 - 2.76 (m, 2 H) 2.97 (s, 5 H) 3.04 3.12 (m, 2 H) 3.25 - 3.34 (m, *J*=5.48 Hz, 2 H) 3.39 (t, *J*=7.24 Hz, 2 H) 3.95 (t, *J*=7.43 Hz, 2 H) 6.98 (s, 2 H) 7.16 (d, *J*=8.61 Hz, 2 H) 7.18 - 7.24 (m, 2 H) 7.24 - 7.34 (m, 8 H) 7.74 (d, *J*=8.22 Hz, 2 H).

### EXAMPLE 71

### (R)-3-(benzo[d]thiazol-2-yl)-1-(3,3-diphenylpropyl)-1-(4-oxo-4-(piperidin-3-ylamino)butyl)urea

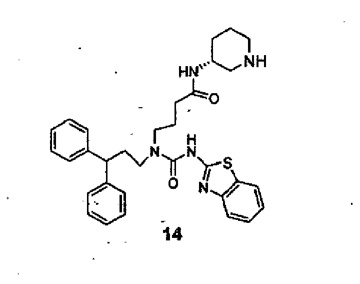

The procedure described in Example 67 was used with the exception of substituting 2-aminobenzothiazole for N-(4-(2-aminothiazol-4-yl) phenyl)methanesulfonamide in Step 2 to give **14** as a white solid.

LC-MS ESI (pos.) m/e: 556 (M+H); 1H NMR (400 MHz, CHLOROFORM-*d*) δ ppm 1.50 -1.61 (m, 1 H) 1.67 - 1.77 (m, 3 H) 1.77 - 1.86 (m, 2 H) 2.24 (q, *J*=5.48 Hz, 2 H) 2.43 (q, *J*=7.43 Hz, 2 H) 2.78 (none, 5 H) 2.88 - 2.97 (m, *J*=11.74 Hz, 2 H) 3.01 (did, *J*=11.93, 2.93 Hz, 1 H) 3.24 - 3.44 (m, 4 H) 3.45 - 3.55 (m, 1 H) 3.98 (t, *J*=7.83 Hz, 1 H) 4.22 (br. s., 1 H) 6.90 (br. s., 1 H) 7.15 - 7.24 (m, 3 H) 7.25 -7.38 (m, 9 H) 7.55 (d, *J*=7.82 Hz, 1 H) 7.70 (d, *J*=8.61 Hz, 1 H)

### EXAMPLE 72

### 4-(3-(5-chloro-4-(4-(methylsulfonyl)phenyl)thiazol-2-yl)-1-(3,3- diphenylpropyl)ureido)bntanoic acid

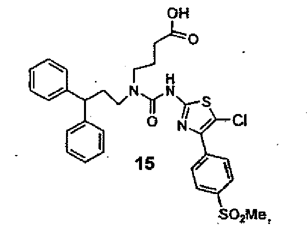

The procedure described-in Example 67 was used with the exception of substituting 5-chloro-4-(4-(methylsulfonyl) phenyl)thiazol-2-amine for N-(4-(2-aminothiazol-4-yl)phenyl)methanesulfonamide in step 2 to prepare **15** as a white solid. LC-MS ESI (neg.) m/e: 611.2 (M-H); 1H NMR (400 MHz, *DMSO-d₆*) δ ppm 11.18 (1 H, s), 8.11 - 8.18 (2 H, m), 7.99 - 8.06 (2 H, m), 7.32 - 7.38 (4 H, m), 7.28 (4 H, t, *J*=7.6 Hz), 7.13 - 7.21 (2 H, m), 4.00 (1 H, t, *J*=7.6 Hz), 3.42 - 3.76 (1 H, m), 3.28 - 3.38 (2 H, m), 3.26 (3 H, s), 3.23 (2 H, d, *J*=7.8 Hz), 2.23 - 2.38 (2 H, m), 2.20 (2 H, t, *J*=7*.*2 Hz), 1.58 - 1.74 (2 H, m)

### EXAMPLE 73

### 4-(3-(5-chloro4-(4-(methylsulfonamido)phenyl)thiazol-2-yl)-l,-(3,3-diphenylpropyl)ureido)butanoic acid

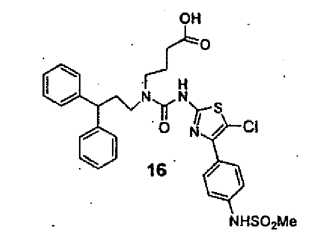

The procedure described in Example 67 was used with the exception of substituting N-(4-(2-amino-5-chierothiazol-4-yl)phenyl)methanesulfonamide for N-(4-(2-aminothiazol-4-yl)phenyl)methanesulfonamide in step 2 to prepare **15** as a white solid. LC-MS ESI (neg.) mule: 626 (M-H)
1H NMR (400 MHz, *DMSO-d₆*) δ ppm 11.05 (1H, s), 9.96 (1 H, s), 7.84 (2 H, dt, *J*=9.2, 2.3, 2.2 Hz), 7.32 - 7.37 (4 H, m), 7.25 - 7.31 (10 H, m), 7.16 (2 H, tt), 4.00 (1 H, t), 3.27 (4 H, dt, *J*=26.8, 7.4, 7.2 Hz), 3.04: (3 H, s), 2.30 (2 H, q), 2.19 (2 H, t, *J*=7.4 Hz), 1.56 - 1.71 (2 H, m)

### EXAMPLE 74

### (S)-3-(benzo[d]thiazol-2-yl)-1-(3,3-diphenylpropyl)-1-(4-oxo-4-(piperidin-3-ylamino)butyl)urea

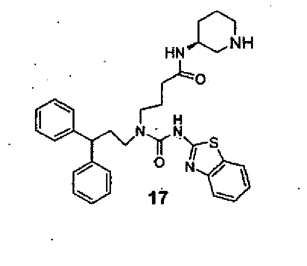

The procedure described in Example 67 was used with the exception of substituting 2-anfnobenzotbiazole for N-(4-(2-aminothiazol-4-yl)phenyl)methanesulfonamide in Step 2 and (S)-tert-butyl 3-aminopiperidine-1-carboxylate for (R)-tert-butyl 3-aminopiperidine-1-carboxylate in Step 4 to obtain **17** as a white solid.

LC-MS ESI (pos.) m/e: 556 (M+H); 1H.NMR (400 MHz, CHLOROFORM-*d*) δ ppm 1.45 - 1.54 (m, *J*=10.56 Hz, 1 H) 1.58 - 1.78 (m, 5 H) 2.12 - 2.21 (m, 2 H) 2.34 (q, .7=7.96 Hz, 2 H) 2.65 - 2.82 (m, 2 H) 2.90 - 2.99 (m, 2 H) 3.15 - 3.34 (m, 4 H) 3.34 - 3.45 (ni, 1 H) 3.89 (t, .7=7.43 Hz, 1 H) 4.14 (br. s., 1 H) 6.96 (br. s., 1 H) 7.07 - 7.16 (m, 3 H) 7.16 - 7.30 (m, 9 H) 7.48 (d, J=8.22 Hz, 1H) 7.62 (d, *J*=8.22 Hz, 1 H)

### EXAMPLE 75

### 3-(benzo[d]thiazol-2-yl)-1-(3,3-diphenylpropyl)-1-(4-oxo-4-(piperidin-4-ylamino)butyl)urea

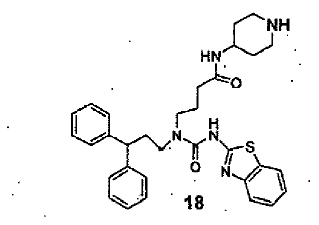

The procedure described in Example 67 was used with the exception of substituting 2-aminobenzothiazole for N-(4-(2-amiriothiazol-4-yl) phenyl)methanesulfonamide in Step 2 and tert-butyl 4-aminopiperidme-1-earboxylate for (R)-tert-butyl 3-aminopiperidine-1-carboxylate in Step 4 to obtain 18 as a white solid.

LC-MS ESI (pos.) m/e: 556 (M+H); 1H NMR (400 MHz, CHLOROFORM-*d*) δ ppm 1.74 - 1.92 (m, 5 H) 1.98 - 2.12 (m, *J*=17.61 Hz, 2 H) 2.18-2.28 (m, 2 H) 2.33 - 2.48 (m, 4 H) 2.92 - 3.06 (m, 2 H) 3.26 - 3.48 (m, 9 H) 3.86 - 4.01 (m, 2 H) 4.04 - 4.12 (m, 1 H) 7.14 - 7.36 (m, 13 H) 7.64 (br. s., 2 H) 7.74 (d, J 7.83 Hz, 1 H)

### EXAMPLE 76

### (R)-1-(3,3-diphenylpropyl)-1-(4-oxo-4-(piperidin-3-ylamino)butyl)-3-(4-phenylthiazal-2-yl)urea

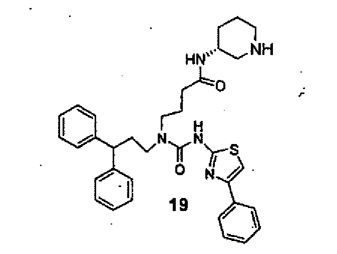

The procedure described in Example 67 was used with the exception of substituting 2-amino phenyl thiazole for N-(4-(2-aminothiazol-4-yl) phenyl)methanesulfonamide in Step 2 to obtain **19** as a whit solid.

LC-MS ESI (pos.) m/e: 582 (M+H); 1H NMR (500 MHz, CHLOROFORM-*d*) δ ppm 1:54 -1.61 (m, 1 H) 1.70 - 1.81 (m, 3 H) 1.82 - 1.90 (m, 2 H) 2.23 - 2.2-8 (m, 2 H) 2.39 - 2.46 (m, 2 H) 2.75 - 2.84 (m, 2 H) 2.88 - 2.95 (m, 1 H) 3.01 (dd, *J*=12.10, 2.81 Hz, 1 H) 3.27 - 3.34 (m, 2 H) 3.34 - 3.47 (m, 2 H) 3.96 (t, J 7.83 Hz, 1 H) 4.15 (br.s., 1H) 6.75 (br. s., 1 H) 7.05 (s, 2 H) 7.22 (t, *J*=6.36 Hz, 2 H) 7.26 - 7.35 (m, 9 H) 7.41 (t, *J*=7.70 Hz, 2 H) 7.86 (dd, *J*=8.31, 1.22 Hz, 2 H)

### EXAMPLE 77

### 1-(3-ammo-3-oxopropyl)-l-(3,3-dtphenylpropyt)-3-(4-(4-(methylsulfonamido)phenyl)thiazol-2-yl)urea

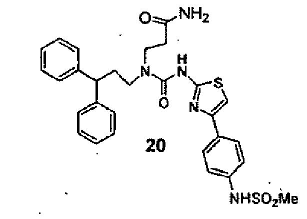

The procedure described in Example 68 was used to prepare **20** from 3-(1-(3,3-diphenylpropyl)-3-(4-(4-(methylsulfonamido)phenyl)thiazol-2-yl)ureido)propanoic acid. LC-MS ESI (pos.) m/e: 578 (M+H)
1H NMR (500 MHz, CHLOROFORM-*d*) δ ppm 2.21-2.25 (m, 2H), 2.42-2.44 (m, 2H), 3.32-3.35 (m, 4H), 4.12-4.25 (m, 1H), 6.65 (s, 1H), 7.2-7.24 (m, 2H), 7.3-7.33 (m, 9H), 7.71-7.73 (m, 2H)

### EXAMPLE 78 .

### (S)-1-(3,3-diphenylpropyl)-1-(4-oxo-4-(piperidn-3-ylamino)butyl)-3-(4-phenylthiazol-2-yl)urea

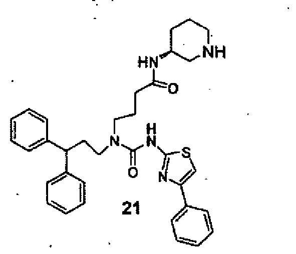

The procedure described in Example 67 was used with the exception of substituting 2-amino phenylthiazolee for N-(4-(2-aminothiazol-4-yl)phenyl)methanesulfonamide in Step 2 and (S)-tert-butyl 3-aminopiperidine-1-carboxylate for (R)-tert-butyl 3-aminopiperidine-1-carboxylate in Step 4 to obtain **21** as a white solid. LC-MS ESI (pos.) m/e: 582 (M+H); 1H NMR (400 MHz, CHLOROFORM-*d*) δ ppm 1.48 -1.59 (m, 1 H) 1.64 - 1.79 (m, 3 H) 1.79 - 1.88 (m, 2 H) 2.23 (dd, J=7.24, 5.28 Hz, 2 H) 2.34 - 2.45 (m, Z H) 2.70 - 2.81 (m, 2 H) 2.81 - 2.91 (P1, 1 H) 3.00 (dd, *J*=12.13, 3.13 Hz, 1 H) 3.23 - 3.32 (m, 2 H) 3.32 - 3.47 (m, 2 H) 3.93 (t, J 7.63 Hz, 1 H) 4.11 (br. s., 1 H) 6.61 (d, J 6.65 Hz, 1 H) 7.05 (s, 1 H) 7.20 (t, J 7.04 Hz, 2 H) 7.24 - 7.36 (m, 9 H) 7.40 (t, *J*= 7.63 Hz, 2 H) 7.86 (d, *J*= 7.04 Hz, 2 H)

### EXAMPLE 79

### 4-(1-(3,3-diphenylpropyl)-3-(4-(4-(methylsulfouyl)phenyl)thiazol-2-yl)ureido)butanoic acid

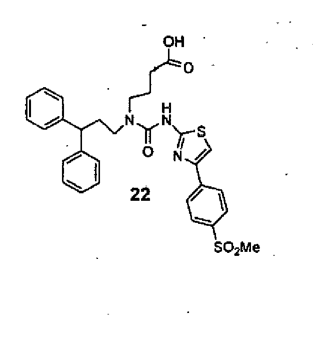

The procedure described in Example 67 was used with the exception of substituting 4-(4-(methylsulfonyl)phenyl)thiazol-2-amine for N-(4-(2-aminothiazol-4-yl)phenyl)methanesulfonamide in Step 2.

LC-MS ESI (neg.) m/e: 576 (M-H) .

1H NMR (400 MHz, *DMSO-d₆*) δ ppm 10.89 (1H, s), 8.10 - 8.19 (2 H, m), 7.90 - 8.00 (2 H, m), 7.75 (1 H, s), 7.32 - 7.38 (4 H, m), 7.26 - 7.32 (4.H, m), 7.17 (2 H, tt, *J*=7.2,1.4. Hz), 4.01 (1 H, t, *J*=7.6 Hz), 3.30 (2 H, t), 3.24 - 3.29 (2 H, m, *J*=7.0 Hz), 3.23 (3 H, s), 2.31 (2 H, q, *J*=7.4 Hz), 2.21 (2 H, t, *J*=7.2 Hz), 1.55 -1.72 (2 H, m)

### EXAMPLE 80

### 1-(4-amino-4-oxobutyl)-3-(benzo[d]thiazol-2-yl)-1-(3,3-diphenylpropyl)urea

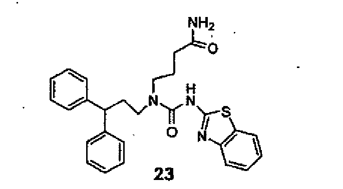

The procedure described in Example 68 was used to prepare **23** from 4-(3-(benzo[d]thiazol-2-yl)-1-(3,3-diphenylpropyl)ureido)butanoic acid. LC-MS ESI (pos.) m/e: 473.5 (M+H)

1H NMR (500 MHz, *MeOH*) δ ppm 7.72 (1 H, d, *J*=7.8 Hz), 7.49 (1 H, d, *J*= 8.1 Hz), 7.36 - 7.42 (1 H, m), 7.32 - 7.36 (4 H, m), 7.27 - 7.32 (4 H, m), 7.23 - 7.27 (2. H, m), 7.16 - 7:20 (2 H, m), 4.02'(1 H, t, *J*=7.8 Hz), 3.25 - 3.32 (2 H, m), 2.44 (2 H, q; *J*=7.8 Hz)3 2:38 (2 H, t, *J*=8. Hz), 1.96 - 2.10 (-2 H, m), 1.72 - 1.84 (2.H, m)

### EXAMPLE 81

### 1-(4-amino-4-oxobutyl)-3-(5-chloro4-phenylthiazol-2-yl)-1-(3,3-diphenylpropyl)urea

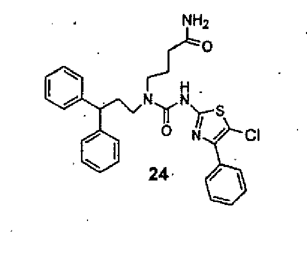

The procedure described in Example 68 was used to prepare **24** from 4-(3-(5-chloro-4-phenylthiazol-2-yl)-1 -(3,3 -diphenylpropyl)ureido)butanoic acid. LC-MS ESI (pos.) m/e: 534.0 (M+H)

1H NMR (500 MHz, *CHLOROFORM-d*) δ ppm 7.71 (2 H, d, J=-S.7 Hz), 7.45 - 7.55 (3 H, m), 7.28 - 7.34 (8 H, m), 7.16 - 7.23 (2 H, m, *J*=6.7, 6.7 Hz), 6.15 (1 H, s), 3.92 - 4.24 (1 H, m), 3.23 - 3.49 (4 H, m), 2.35 - 2.49 (2 H, m, *J*=7.9,7.9, 7.9 Hz), 2.19 - 2.34 (2 H, m), 1.80 -1.98 (2 H, m)

### EXAMPLE 82

### 3-(benzo[d]thiazol-2-yl)-1-(3,3-diphenylpropyl)-1-(4-oxo-4-(piperazin-1-yl)butyl)urea

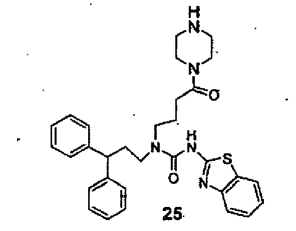

The procedure described in Example 67 was used to prepare **25** using 1-Boc-piperazine instead of (R)-tert-butyl 3-aminopiperidine-1-carboxylate in Step 4 and 2-amino benzothiazole instead of N-(4-(2-aminothiazol-4-yl) phenyl)methanesulfonamide in Step 2.

LC-MS ESI (pos.) m/e: 542 (M+H); 1H NMR (500 MHz, CHLOROFORM-*d*), δ ppm 1.78 - 1.88 (m, 2 H) 2.23 - 2.28 (m, 2 H) 2.30 - 2.38 (m, 2 H) 2.78 - 2.87 (m, 4 H) 3.15 - 3.39 (m, 6 H) 3.39 - 3.43 (m,2 H) 3:70 (s, 2 H) 3.87 - 3.93 (m, 1 H) 7.03 - 7.30 (m, -12 H) 7.66 (d, *J*= 7.58 Hz, 2 H)

### EXAMPLE 83

### 4-(3-(5-chloro-4-phenylthiazol-2-yl)-1-(3,3-diphenylpropyl)ureido)butanoic acid

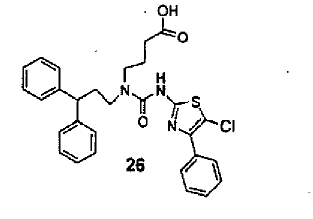

The procedure described in Examples 67 was used to prepare **26** using 5-chloro-4-phenylthiazol-2-amine instead of N-(4-(2-aminothiazol-4-yl)phenyl)methanesulfonamide in Step 2.

LC-MS ESI (neg.) m/e: 533.0 (M-H)

1H NMR (500 MHz, *DMSO-d₆*) δ ppm 7.87 (2 H, d, *J*=7.3 Hz), 7.4.7 (2 H, t, J=7.6 Hz), 7.36 - 7.42 (1 H, m, *J*=7.3, 7.3 Hz), 7.31 - 7.36 (4 H, m), 7.28 (4 H, t, *J*=7.9 Hz), 7.16 (2 H, t, J=7.0 Hz), 4.00 (1 H, t, *J*=7.6 Hz), 3.27 (4 H, dt, *J=*32.8*,* 7.5, 7.3 Hz), 2.30 (2 H, q, *J*=7.9 Hz), 2.19 (2 H, t, *J*=7.0 Hz), 1.58 -1.70 (2 H, m)

### EXAMPLE 84

### 1-(4-(4-acetylpiperazin-1-yl)-4-oxobutyl)-1-(3,3-diphenylpropyl)-3-(4-phenylthiazol-2-yl)urea

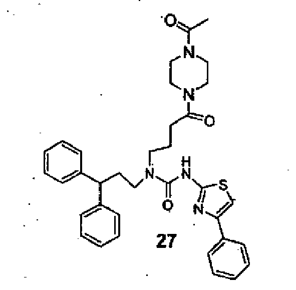

The procedure described in Example 67 was used to prepare **27** using 2-amino phenyl thiazole instead of N-(4-2-aminothiazol-4-yl)phenyl)methanesulfonamide in Step 2 and 1-(piperazin-1-yl)ethanone instead of (R)-tert-butyl 3-aminopiperidine-1-carboxylate in Step 4.

LC-MS ESI (pos.) m/e: 610 (M+H); Elemental analysis: Theoretical C 68.94, H 6.45, N 11.49; Found C 66.66, H 6.28, N 10.96; 1H NMR (500 MHz, CHLOROFORM-*d*) δ ppm 1.88 -1.97 (m, .7=5.14 Hz, 2 H) 2.08 - 2.15 (m, 3 H) 2.31 - 2.37 (m, 2 H) 2.41 (dd, 2 H) 3.28 - 3.39 (m, 4 H) 3.39 - 3.43 (m, 1 H) 3.46 (s, 3 H) 3.59 - 3.66 (m, 2 H) 3.71 - 3.82 (m, 2 H) 3.97 (t, J=7.70 Hz, 1 H) 7.06 (s, 1 H) 7.17 - 7.24 (m, 2 H) 7.25 - 7.35 (m, 8 H) 7.40 (t, *J=*7.58 Hz, 2 H) 7.88 (d, *J*=7.34 Hz, 2 H}

### EXAMPLE 85

### 3-(benzo[d]thiazol-2-yl)-1-(3,3-diphenylpropyl)-1-(4-(ethylamino)-4-oxobutyl)urea

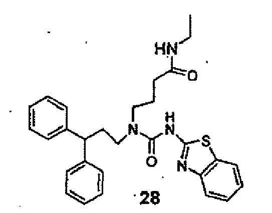

The procedure described in Example 67 was used to prepare **28** using 2-ammo benzothiazole instead of N-(4-(2-aminothiazol-4-yl) phenyl)methanesulknamide in Step 2 and ethanamine instead of (R)-tert-butyl 3-aminopiperidine-1-carboxylate in Step 4. LC-MS ESI (pos.) m/e: 501 (M+H); 1H NMR (500 MHz, CHLOROFORM-*d*) δ ppm 1.18 (t, *J*=7.34 Hz, 3 H) 1.83 1.91 (m, 2 H) 2.23 - 2.29 (m, 2 H) 2.40 - 2.47 (m, 2 H) 3.31 - 3.45 (m, 8 H) 3.99 (t, *J*=8.19 Hz, 1 H) 4.11 - 4.18 (m, 2 H) 7.21 (t, *J*=6.60 Hz, 4 H) 7.24 - 7.35 (m, 7 H) 7.39 - 7.45 (m, 1 H) 7.77 (d, *J*=8.07 Hz, 2 H)

### EXAMPLE 86

### 3-(benzo[d]thiazol-2-yl)-1-(4-(benzylamino)-4-oxobutyl)-1-(3,3-diphenylpropyl)urea

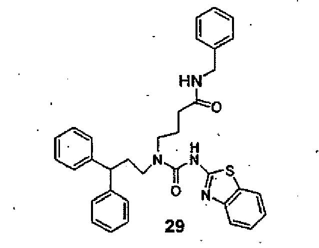

The procedure described in Example 67 was used to prepare **29** using 2-amino benzothiazole instead of N-(4,-(2-aminothiazol-4-yl) phenyl)methariesulfonamide in Step 2 and benzylamine instead of (R)-tert-butyl 3-aminopiperidine-1-carboxylate in Step 4. LC-MS ESI (pos.) m/e: 563 (M+H);

1H NMR (400 MHz, D*MSO-d₆*) δ ppm 10.94 - 11.19 (1 H, m), 8.33 (1H, s), 7. 86 (1H, s), 7.62 (1 H, s), 7.32 - 7.38 (5 H, m), 7.25 - 7.31 (5 H, m), 7.19 - 7.25 (4 H, m), 7.16 (2 H, t, *J*=7.2 Hz), 4.25 (2 H, d, *J*=5.9 Hz), 3.93 - 4.05 (1 H, m), 3.16 - 3.30 (3 H, m), 2.24 - 2.38 (2 H, m), 2.03 - 2.21 (2 H, m), 1.70 (2 H, s), 1.18 - 1.34 (1 H, m)

### EXAMPLE 87

### 3-(benzo[d]thiazol-2-yl)-1-(4-(4-(dimethylcarbamoyl)piperazin-1-yl)-4-oxobutyt)-1-(3,3-diphenylpropyl)nrea

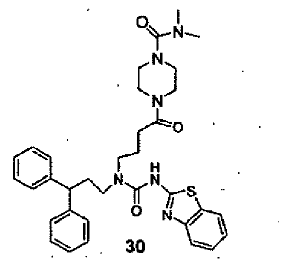

The procedure described in Example 67 was used to prepare **30** using 2-amino benzothiazole instead of N-(4-(2-aminothiazol-4-yl) phenyl)znethanesulfonamide in Step 2 and N,N-dimethylpiperazine-1-carboxarnide instead of (R)-tert-bntyl 3-aminopiperidme-1-carboxylate in Step 4.

LC-MS ESI (pos.) m/e: 613 (M+H); Elemental analysis: Theoretical C 66.64, H 6.58, N 13.71 Found C 65:16, H 6.63, N 13.33; 1H NMR (500 MHz, CHLOROFORM-*d*) δ ppm 1.90 2.00 (m, 3H) 2.412.50 (m, 4 H) 2.81 - 2.89 (m, 3 H) 2.88 (s, 6 H) 3.20 - 3.31 (m, 4H) 3.37 - 3.48 (m, 4H) 3.49 - 3.56 (m, 2H) 3.68 - 3.77 (m, 2H) 7.17 - 7.23 (in, 3H) 7.23 - 7.34 (m, 9 H) 7:76 (d, *J*=8.80 Hz, 1 H) 7.80 (d, *J*=7.58 Hz, 1 H)

### EXAMPLE 88

### 1-(3,3-diphenylpropyl)-1-(4-oxc-4-(piperaztn-l-yi)butyt)-3-(4-phenytthtazot-2-yi)urea

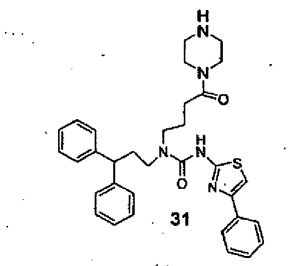

The procedure described in Example 67 was used to prepare **31** using 2-amino phenyl thiazole instead of N-(4-(2-anmhothiazol-4-yl) phenyl)methanesulfonamide in Step 2 and 1-Boc piperazine instead of (R)-tert-butyl 3-aminopiperidine-1-carboxylate in Step 4.

LC-MS ESI (pos.) m/e: 568 (M+H); 1H NMR (500 MHz, CHLOROFORM-*d*) δ ppm 1.78 -1.87 (m, 2 H) 2.19 - 2.26 (m, 2 H) 2.29 - 2.37 (m, 2 H) 2.74 - 2.85 (m, 4 H) 3.18 - 3.30 (m, 4 H) 3.33 - 3.39 (m, 2H) 3.64 - 3.72 (m, 2 H) 3.89 (t; *J*=7.58 Hz, 1 H) 6.96 (s, 1H) 7.11 (t, *J*=6.72 Hz, 2 H) 7.14 7.26 (m, 9 H) 7.29 (t, *J*=7_{:}83 Hz, 2 H) 7.80 (d, *J*=7.5g Hz, 2 H)

### EXAMPLE 89

### 3-(benzo[d]thiazol-2-yl)-1-(4-(dimethylamino)-4-oxobutyl)-1.-(3,3_

### diphenyIpropyt)urea

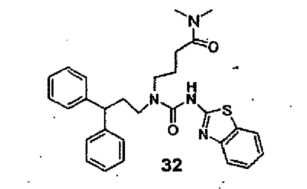

The procedure described in Example 67 was used to prepare 32 using 2-amino benzothiazole instead of N-(4-(2-aminothiazol-4-yl) phenyl)methanesulfonamide in Step 2 and dimethylamine instead of (R)-tert-butyl 3-aminopiperidine-1-carboxylate in Step 4. LC-MS ESI (pos.) m/e: 501 (M+H; 1H NMR (500 MHz, CHLOROFORM-*d*) δ ppm 1.90. -1.99 (m, 2 H) 2.37 - 2.50 (m, 4 H) 3.08 (s, 3 H) 3.33 - 3.47 (m, 4 H) 4.04 (br. s., 1) 7.16 - 7.23 (m, 4 H) 7.24 - 7.33 (m, 7 H) 7.43 (t, *J=*7.83 Hz, 1 H) 7.78 (dd, *J*=7.34, 2.20 Hz, 2 H)

### EXAMPLE 90

### (S)-1-(3,3-diphenylpropyl)-1-(4-(3-methylpiperazin-1-yl)-4-oxobutyl)-3-( 4-ptienylthiazol-2-yi)urea

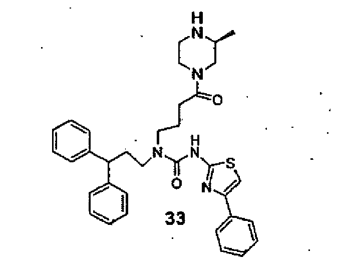

The procedure described in Example 67 was used to prepare 33 using 2-amino phenyl thiazole instead of N-(4-(2-aminothiazol-4-yl) phenyl)methanesulfonamide in Step 2 and (S)-1-Boc-2-methylpiperazineinstead of (R)-tert-hutyl 3-anfnopipefidine-I - carboxylate in Step 4.

LC-MS ESI (pos.) m/e: 582 (M+H); 1H NMR (500 MHz, CHLOROFORM-*d*)δppm 1.11 (dd, *J*=10.64, 5.50 Hz,3 H) 1.86- 1.95 (m, 2 H) 2.22 - 2.46 (m, 5 H) 2.71 - 2.84 (m, 3 H) 3.00 - 3.14 (m, 2 H) 3.25 - 3.42 (m, 4 H) 3.57 - 3.68 (m, 1 H) 3.97 (t,*J*=7.58 Hz, 1 H) 4.61 - 4.76 (m, 1 H) 7.06 (s, 1 H) 7.16 - 7.23 (m, 2 H) 7.23 - 7.34 (m, 9 H 7.35 - 7.42 (m, 2 H) 7.91 (t, *J*=7.70 Hz, 2 H)

### EXAMPLE 91

### (R)-1-(3,3-diphenylpropyl)-1-(4-(2-methylpiperazin-1-yl)-4-oxobutyl)-3-(4-phenylthiazol-2-yl)urea

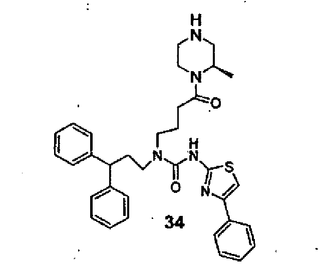

The procedure described in Example 67 was used to prepare 34 using 2-amino phenyl thiazole instead of N-(4-(2-aminothiazol-4-yl) phenyl)methanesulfonamide in Step 2 and (S)-1-Boc-3-methylpiperazineinstead of (R)-tert-butyl 3-aminopiperidine-1-carboxylate in Step 4.

LC-MS ESI (pos.) m/e: 582 (M+H); 1H NMR (500 MHz, CHLOROFORM-*d*) δ ppm 0.73 - 0.90 (m, 2 H) 1.13 - 1.42 (m, 4 H) 1.71 1.89 (m, 2 H) 2.13 - 2.48 (m, 4 H) 2.73 - 2.89 (m, 1H) 2.97 - 3.14 (m, 2 H) 3.20 - 3.44(m, 4 H) 3.83 - 3.99 (m, I H) 6.88 (s, I H) 7.06 - 7.34 (m, 11H) 7.35 - 7.50 (m, 2 H) 7.60 - 7.72 (m, 2 H) 7.88 (br. s., 1H)

### EXAMPLE 92

### 4-(3-(5-chloro4-(4-eyanophenyl)tbiazol-2-yl)-l-(3,3-diphenylpropyl)ureido)butanoic acid

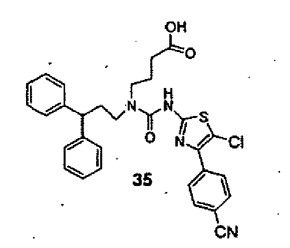

The procedure described in Example 67 was used to prepare 35 using 4-(2-amino,-5-chlolorothiazol-4-yl)benzonitrile instead of N-(4-(2-aminothiazol-4-yl)phenyl)methanesulfonamide in Step 2 LC-MS ESI (pos.) m/e: 559.2 (M)

1H NMR (400 MHz, *CHLOROFORM-d)* δ ppm 7.74 (4 H, q,*J*=8.3 Hz), 7.29 - 7.33 (3 H, m), 7.22 - 7.26 (3 H, m), 7.17 - 7.23 (2 H, m), 4.00 (1 H, s), 3.28 - 3.48 (4 H, m, *J*=15.6 Hz), 2.38 (4 H, t, *J*=6.7 Hz), 1.78 - 1.92 (2 H, m)

### EXAMPLE 93

### (S)-1-(3,3-diphenylpropyl)-1-(3-oxo-3-(piperidin-3-ylamino)propyl)-3-(4-phenylthiazol-2-yl)urea

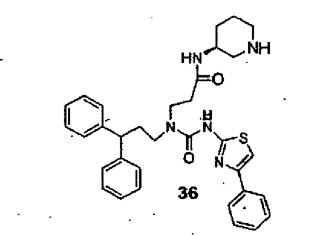

The procedure described in Example 67 was used to prepare 36 using 2-amino phenyl thiazole instead of N-(4-(2-aminotbiazol-4-yl) phenyl)methanesulfonamide in Step 2, (S)-tert-butyl 3-ammopiperidine-1-carboxylate instead of (R)-tert-butyl 3-aminopiperidine-1-carboxylate in Step 4 and tert-butyl 3-bromopropanoate instead of tertbutyl 4-bromobutanoate in Step 1 .

LC-MS ESI (pos.) m/e: 582 (M+H); Elemental analysis: Theoretical C 70:19, H 6.76, N 12.04; Found C 68.82, H 6.68, N11.60; 1H NMR (500 MHz,-CHLOROFORM-*d)* δ ppm 1.33 (d, *J=*6.36 Hz, 3 H) 1.86 - 1.98 (m, *J*=5.14 Hz, 2 H) 2.19 - 2.37 (m,2H) 2.42 (q, *J*=7.74 Hz, 2 H) 2.68 (td, *J*=12.29, 3.55 Hz, 1 H) 2.80 - 3.11 (m, 3 H) 3.24 - 3.42 (m, 4 H) 3.98 (t, *J*=7.58 Hz, 1 H) 7.06 (s, 1 H) 7.18 - 7.23 (m, 2 H) 7.25 - 7.35 (m, 9 H) 7-39 (t, *J=*7.70 Hz, 2 H) 7.91 (d, *J=7*.58 Hz, 2 H)

### Example 94

### 3-(benzo[d]thiazol-2-yl)-1-(3,3-diphenylpropyt)-1-(4-morpholio-4-oxobutyl)urea

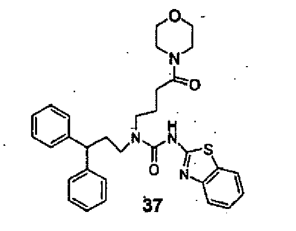

The procedure described in Example 67 was used to prepare 37 using 2-amino benzothiazole instead of N-(4-(2-aminathiazol-4-yl) phenyl)methanesulfomide in Step 2, and morpholine instead of (R)-tert-butyl 3-aminopiperidine-i-earboxylate in Step 4. LC-MS ESI (pos.) m/e: 543 (M+H); 1H NMR (500 MHz, CHLOROFORM-*d*) δ ppm 1.91 1.99 (m, 2 H) 2.32 - 2.39 (m,2 H) 2.40 - 2.48 (m, 2 H) 3.28 - 3.34 (m, 2 H) 3.36 - 3.43 (m,2 H) 3.43 - 3.49 (m, 2 H) 3.65 - 3.73 (m, 4 H) 3.73 - 3.80 (m, 2 H) 4.00 (t, *J*=7.70 Hz, 1 H) 7.17 - 7.34 (m, 11 H) 7.36 - 7.41 (m, 1 H) 7.77 (d, *J*=7.83 Hz, 2 H)

### EXAMPLE 95

### tert-butyl 4-(4-(3-(benzo[dlthiazol-2-yi)-I-(3,3-diphenylpropyl)ureido)b utanoyl)piperazine-l-carboxylate

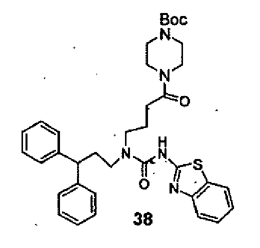

The procedure described in Example 67 was used to prepare 38 using 2-amino benzothiazole instead of N-(4-(2-aminothiazol-4-yl) phenyl)methanesulfonamide in Step 2, and 1-Boc piperazine instead of (R)-tert-butyl 3-aminopiperidine-1-carboxylate in Step 4

LC-MS ESI (pos.) m/e: 642 (M+H); 1H NMR (500 MHz, CHLOROFORM-*d*) δ ppm 1.50 (d, J=1.47 Hz, 9 H) 1.88 - 1.97 (m, 2 H) 2.32 - 2.39 (m, 2 H) 2.40 - 2.47 (m, 2 H) 3.25 - 3.34 (m, 2 H) 3.35 - 3.48 (m, 8 H) 3.69 (br. s., 2 H) 4.00 (t, *J*=8.19 Hz, 1 H) 7.15 - 7.39 (m, 12H) 7.76 (d, ./=7.58 Hz, 2 H)

### EXAMPLE 96

### tert-butyl 4-(4-(1-(3,3-diphenylpropyl)-3-(4-phenylthiazol-2-yl)ureido)butanoyl)piperazine-l-ca'rboxylate

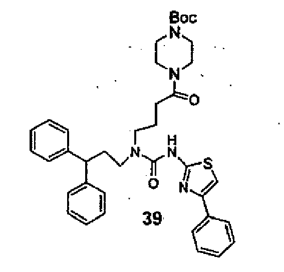

The procedure described in Example 67 was used to prepare 39 using 2-amino phenyl thiazole instead of N-(4-(2-aminothiazol-4-yl) phenyl)methanesulfonamide in Step 2, and 1-Boc piperazine instead of (R)-tert-butyl 3-aminopiperidine-1-carboxylate in Step 4.

LC-MS ESI (pos.) m/e: 668 (M+H); 1H NMR (500 MHz; PMSO-*d*₆) δ ppm 1.41 (s,9 H) 1.64- 1.73 (m, 2 H) 2.27 - 2.38 (m, 4 H) 2.53 - 2.67 (m, 2 H) 3.16 - 3.25 (m, 4 H) 3.31 (s, 2 H) 3.37 (s, 2 H) 3.41 - 3.46 (m, 2 H) 3.99 - 4.03 (m, 1 H) 7.18 (t, J=6.72 Hz, 2 H) 7.30 (t,*J*=7.70 Hz, 6 H) 7.34 - 7.38 (m, 4 H) 7.38 - 7.47 (m, 3 H) 7.90(d, *J*=8.07 Hz, 2 H)

### EXAMPLE 97

### 3-(benzo[d]thiazol-2-yl)-1-(4-(cyclohexylamino)-4-oxohutyl)-1-(3,3-diphenylpropyl)urea

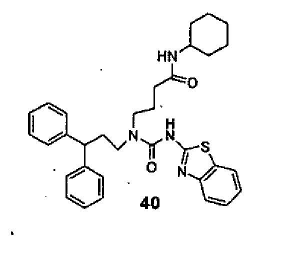

The procedure described in Example 67 was used to prepare 40 using 2-amino benzothiazole instead of N-(4-(2-aminothiazol-4-yl) phenyl)methanesulfonamide in Step 2, and cyclohexylamine instead of (R)-tert-butyl 3-aminopiperidine-1-carboxylate in Step 4.

LC-MS ESI (pos.) m/e: 555 (M+H) 1H NMR (500 MHz, CHLOROFORM-*d)* δ ppm 0.85 - 0.95 (m, 4 H) 1.25 - 1.3C (m, 4 H) 1.36 - 1.48 (m, 4 H) 1.61 - 1.69 (m, 1 H) 1.74 (d, *J=*14.92 Hz; 2 H) 1.82 - 1.90 (m,2 H) 1.93 - 2.00 (m, *J*=11.74 Hz, 2 H) 2.21 - 2.27 (m, 2 H) 2.39 - 2.49 (m, 2 H) 3.31 - 3.45 (m, 4 H) 3.87 - 4.03 (m, 1 H) 7.16 - 7.35 (m, 11 H) 7.39 - 7.45 (m, 1 H) 7.70 - 7.79 (m, 2 H)

### EXAMPLE 98

### 3-(3-(5-chloro-4-(4-(methylsulfonamido)phenyl)thiazol-2-yl)-l-(3-diphenylpropyl)ureido)propanoic acid

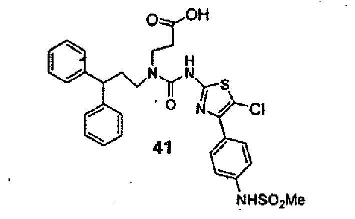

The procedure described in Example 67 was used to prepare 41 using N-(4-(2-amino-5-chlorothiazol-4-yl) phenyl)methanesulfonamide instead of N-(4-(2-aminothiazol-4-yl)phenyl)methanesulfonamide in Step 2, and tert-butyl 3-bromopropanoate instead of tert-butyl 4-bromobutanoate in Step 1.

LC-MS ESI (pos.) m/e: 614.0 (M+H)

1H NMR (400 MHz, *DMSO-d₆*) δ ppm 11.15 (1H, s), 9.96 (1 H, s), 7.84 (2 H, d, *J=*8.6 Hz), 7.31 - 7.38 (4 H, m), 7.25 - 7.31 (6 H, m), 7.13 - 7.20 (2 H, m, *J*=7.2, 7.2 Hz), 4.43 (1 H, s), 4.00 (1H, t, *J*=7.6 Hz), 3.42 - 3.58 (2 H, m), 3.20 3.33 (2 H, m), 2.96 - 3.10 (3 H, m), 2.44 (2 H, t, *J*=7.2 Hz), 2.23 - 2.36 (2H, m)

### EXAMPLE 99

### (R)-1-(3,3-dipbenylpropyl)-1-(4-(3-metbylpiperazin-l-yl)4-oxobutyl)-3-(4-phenylthiazol-2-yl)urea

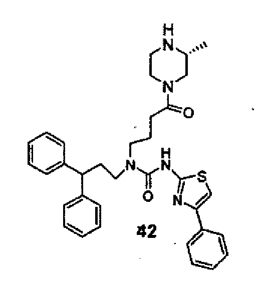

The procedure described in Example 67 was used to prepare 42 using 2-amino phenyl thiazole instead of N-(4-(2-aminothiazol-4-yl) phenyl)methanesulfonamide in Step 2, and (R)-tert-butyl 2-methylpiperazine-1-carboxylate instead of (R)-tert-butyl 3-aminopiperidine-1-carboxylate in Step 4.

LC-MS ESI (pos.) m/e: 582 (M+H); Elemental analysis: Theoretical C 70.19, H 6.76, N 12.04; Found C 68.05, H 6.72, N 11.46; 1H NMR (500 MHz, CHLOROFORM-*d*) δ ppm 0.94 - 1.05. (m, 3 H) 1.74 - 1.85 (m, 2H) 2.12 - 2.38 (m, 5 H) 2.62 - 2.77 (m, 3 H) 2.97 (d, J=11.74 Hz, 1 H) 3.02 - 3.10 (m, 1 H) 3.14 - 3.30 (m, 4 H) 3.48 - 3.61 (m, 1 H) 3.86 (t, *J*=7.83 Hz, 1 H) 4.52 - 4.64 (m, 1 H).6.94 (s, 1 H) 7.09 (t, J=6.36 Hz, 2 H) 7.13 - 7.23 (m, 9 H) 7.23 - 7.30 (m, 2 H) 7.78 (t, *J*=7.58 Hz, 2 H)

### EXAMPLE 100

### (S)-1-(3,3-diphenylpropyl)-1-(4-(2-methylpipernin-1-yl)-4-oxobutyl)-3-(4-pheayltlxiazol-2-yl)urea

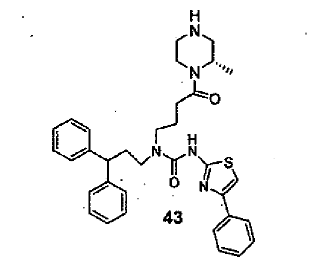

The procedure described in Example 67 was used to prepare **42** using 2-amino phenyl thiazole instead of N-(4-(2-aminothiazol-4-yl) phenyl)methanesulfonamide in Step 2, and (R)-tert-butyl 3-methylpiperazine-1-carboxylate instead of (R)-tert-butyl 3-aminopiperidine-1-carboxylate in Step 4.

LC-MS ESI (pos.) m/e: 582 (M+H); Elemental analysis: Theoretical C 70.19, H 6.76, N 12.04; Found C 68.82, H 6.68, N 11.60; 1H NMR (500 MHz, CHLOROFORM-*d*) δ ppm 1.33 (d, *J*=6.36 Hz, 3 H) 1.86 - 1.98 (m, *J*=5.14 Hz, 2 H) 2.19 - 2.37 (m, 2 H) 2.42 (q, *J*=7.74 Hz, 2 H) 2.68 (td, *J*=12.29, 3.55 Hz, 1 H) 2.80 - 3.11 (m, 3 H) 3.24 - 3.42 (m, 4 H) 3.98 (t, J=7.58 Hz, 1 H) 7.06 (s, 1 H) 7.18 - 7.23 (m, 2 H) 7.25 - 7.35 (m, 9 H) 7.39 (t, *J*=7.70 Hz, 2 H) 7.91 (d, *J*=7.58 Hz, 2 H)

### EXAMPLE 101

### tert-butyl 4-(3-(benzo [d]thiazol-2-yl)-1-(3,3-diphenylpropyl)ureido)butanoate

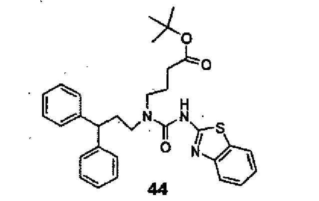

The procedure described in Example 67 was used to prepare 42 using 2-amino benzothiazole instead of N-(4-(2-aminothiazol-4-yl) phenyl)methanesulfonamide in Step 2.

LC-MS ESI (pos.) m/e: 530 (M+H); 1H NMR (500 MHz, CHLOROFORM-*d*) δ ppm 1.50 (s, 9 H) 1.81 - 1.90 (m, 2 H) 2.29 (t, *J=6.72* Hz, 2 H) 2.41 - 2.49 (m, 2 H) 3.31 - 3.42 (m, 4 H)4.00 (t, *J*=7.83 Hz, 1 H) 7.18 - 7.24 (m, 2 H) 7.26 - 7.35 (m, 9 H) 7.39 - 7.44 (m, 1 H) 7.70. (d, *J=*8.56 Hz, 1 H) 7.78 (d, *J*=8.80 Hz, 1 H)

### EXAMPLE 102

### (R)-1-(3,3-diphenylpeopyl)-1-(3-oxo-3-(piperidin-3-ylamino)gropyl)-3-(4-phenylthiazol-2-yl)urea

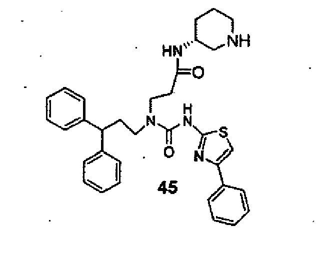

The procedure described in Example 67 was used to prepare 45 using 2-amino phenyl thiazole instead of N-(4-(2-aminothiazol-4-yl) phenyl)methanesulfonamide in Step 2, and tert-butyl 3-bromopropanoate instead of tert-butyl 4-bromobutanoate in Step 1. LC-MS ESI (pos.) m/e: 568 (M+H); 1H NMR (400 MHz, CHLOROFORM-*d*) δ ppm 1.34 -1.46 (m, 1 H) 1.47 - 1.65 (m, 3 H) 2.27 - 2.41 (m, 4 H) 2.56 - 2.67 (m, 2 H) 2.69 - 2.78 (m, 1 H) 2.82 (dd, *J*=11.93, 2.93 Hz, 1 H) 3.17 (dd, *J*=9.00, 5.48 Hz, 2 H) 3.52 (t, *J*=5.87 Hz, 2 H) 3.87 (t, *J=*7.83 Hz, 1 H) 3.92 - 4.00 (m, 1 H) 6.57(d, *J*=6.65 Hz, 1 H) 6.94 (s, 1 H) 7.08 - 7.16 (m, 2 H) 7.16 - 7.27 (m, 9 H) 7.31 (t, *J=*7.63 Hz, 2 H) 7.76 (d, *J=*7.43 Hz, 2 H)

### EXAMPLE 103

### 3-(benzo[d]thiazol-2-yl)-1-(3,3-diphenylpropyl)-1-(3-morpholino-3-oxopropyl)urea

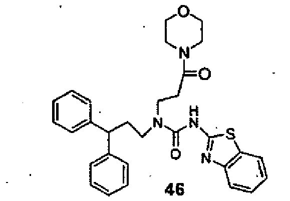

The procedure described in Example 67 was used to prepare 46 using 2-amino benzothiazole instead of N-(4-(2-aminothiazol-4-yl) phenyl)melhanesulflonamide in Step 2, morpholine instead of (R)-tert-butyl 3-aminopiperidine-1-carboxylate in Step 4, and tert-butyl 3-bromopropanoate instead of tert-butyl 4-bromobutanoate in Step 1. LC-MS ESI (pos.) m/e: 529 (M+H);

1H NMR (400 MHz, *DMSO-d*₆),δ ppm 11.35 (1 H, s), 7.86 (1 H, s), 7.63 (1 H, s), 7.31 - 7.41 (5 H, m), 7.28 (4 H, t, *J*=7.6 Hz), 7.17 (3 H, t, *J*=7.2 Hz), 3.88 - 4.07 (1 H, m), 3.47 - 3.72 (5 H, m), 3.35 - 3.45 (4 H, m), 3.15 - 3.28 (3 H, m), 2.54 - 2.66 (2 H, m), 2.25 - 2-43 (2H,m)

### EXAMPLE 104

### 4-(3-(benzo[d]thiazol-2-yl)-1-(3,3-diphenylpropyl)ureido)butanoic acid

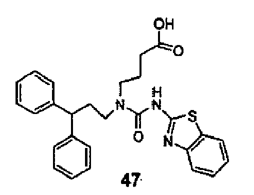

The procedure described in Example 67 was used to prepare 47 using 2-amino benzothiazole instead of N-(4-(2-aminathiazal-4-yl) phenyl)methanesulfonamide in Step 2.

LC-MS ESI (pos.) m/e: 474 (M+H); 1H NMR (400 MHz, DMSO-*d*₆) δ ppm 1.59 - 1.73 (m, 2 H) 2.19 (t, *J*=7.43 Hz, 2 H) 2.31 (q, *J*=7.56 Hz, 2 H) 3.20 - 3.30 (m, 2 H) 3.30 - 3.40 (m, 2 H) 3.99 (t, *J*=8.02 Hz, 1 H) 7.12 - 7.23 (m, 3 H) 7.28 (t, *J*=7.63 Hz, 4 H) 7.32 - 7.39 (m, 5 H) 7.51 (br. s., 1 H) 7.80 (d, *J*=9.78 Hz, 1 H)

### EXAMPLE 105

### 3-(benzo[d] thiazol-2-yl)-1-(3,3-diphenylpropyl)-1-(4-oxo-4-(piperidin-1-yl)butyl)urea

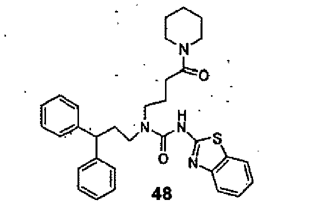

The procedure described in Example 67 was used to prepare 48 using 2-amino benzothiazole instead of N-(4-(2-aninothiazol-4-yl) phenyl)methanesulfonamide in Step 2, and piperidine instead of (R)-tert-butyl 3-aminopiperidine-1-carboxylate in Step 4. LC-MS ESI (pos.) m/e: 541 (M+H); 1H NMR (500 MHz, CHLOROFORM-*d*) δ ppm 1.43 - 1.56 (m, 2 H) 1.58 - 1.73 (m, 6 H) 1.90 - 2.00 (m, 2 H) 2.38 - 2.48 (m, 4 H) 3.29 - 3.37 (m, 2 H) 3.42 (dd, *J=*10.88, 5.99 Hz, 4 H) 3.72 - 3.78 (m, 2 H) 3.98 - 4.05 (m, 1 H) 7.18 - 7.24 (m, 2 H) 7.27 - 7.35 (m, 9 H) 7.42 - 7.48 (m, 1 H) 7.76 - 7.85 (m, 2 H)

### EXAMPLE 106

### 3-(benzo[d]thtazol-2-yl)-1-(3,3-diphenytpropyl)-1-(3-oxo-3-(piperazin-1-yl)propyl)urea

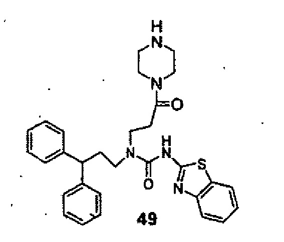

The procedure described in Example 67 was used to prepare 49 using 2-amino benzothiazole instead of N-(4-(2-aminothiazol-4-yl) phenyl)methanesulfonamide in Step 2,1-Boc piperazine instead of (R)-tert-butyl 3-aminopiperidine-1-carboxylate in Step 4, and tert-butyl 3-bromopropanoate instead of tert-butyl 4-bromobutanoate in Step 1. LC-MS ESI (pos.) m/e: 528 (M+H); 1H NMR (500 MHz, CHLOROFORM-*d*) δ ppm 2.30 - 2.47 (m, 4 H) 2.69 - 2.80 (m, 4 H) 3.15 - 3.22 (m, 2 H) 3.28 - 3.34 (m, 2 H) 3.52 - 3.61 (m, 4 H) 3.86 - 3.93 (m, 1 H) 7.08 - 7.14 (m, 3 H) 7.14 - 7.25 (m, 8 H) 7.2 7.(t, *J=*7.70 Hz, 1 H) 7.65 (d, *J*=7.34 Hz, 2 H)

### EXAMPLE 107

### 3-(benzo[d]thiazol-2-yl)-1-(3-(dimethylamino)-3-oxopropyl)-1-(3,3-diphenylpropyl)urea

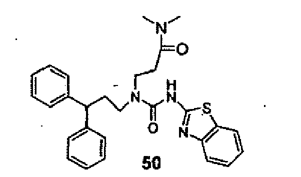

The procedure described in Example 67 was used to prepare 50 using 2-amino benzothiazole instead of N-(4-(2-aminothiazol-4-yl) phenyl)methanesulfonxnide in Step 2, dimethylamine instead of (R)-tert-butyl 3-aminopiperidine-1-carboxylate in Step 4, and tert-butyl 3-bromopropanoate instead of tert-butyl 4-bromobutanoate in Step 1. LC-MS ESI (pos.) m/e: 487 (M+H); 1H NMR (500 MHz, CHLOROFORM-*d* δ ppm 1.47 (d, *J=*7.34 Hz, 1 H) 1.49 - 1.56 (m, 1 H) 2.43 - 2.50 (m, 4 H) 2.54 - 2.59 (m, 2 H) 2.96 - 3.01 (m, 6 H) 3.25 - 3.37 (m, 2 H) 3.63 - 3.71 (m, 2 H) 7.19 - 7.23 (m, 2 H) 7.25 - 7.37 (m, 9 H) 7.48 (t, *J*=8.19 Hz, 1 H) 7.76 - 7.81 (m, 2 H)

### EXAMPLE 108

### 3-(beazo[d]thiazol-2-yl)-1-(3,3-dipbenylpropyl)-1-(4-(4-tuethylpiperazin-1-yl)-4-oxobutyl)urea

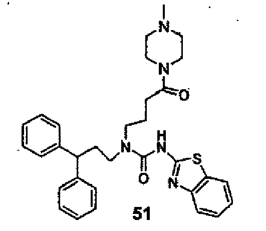

The procedure described in Example 67 was used to prepare 51 using 2-amino benzothiazole instead of N-(4-(2-aminothiazol-4-yl) phenyl)methanesulfonamide in Step 2, and 1-methyl piperazine instead of (R)-tert-butyl 3-aminopiperidine-1-carboxylate in Step 4.

LC-MS ESI (pos.) m/e: 556 (M+H); 1H NMR (500 MHz, CHLOROFORM-*d*) δ ppm 1.17 (t, *J*=6.97 Hz, 2 H) 1.36 (s, 1 H) 1.78- 1.87 (m, 2 H) 2.21 - 2.29 (m, 6 H) 2.30 - 2.37 (m, 4 H) 2.42 (dd, *J*=13.20, 5.38 Hz, 4 H) 3.16 - 3.25 (m, 2 H) 3.26 - 3.33 (m, 2 H) 3.37 - 3.49 (m, 3H) 3.60 - 3.68 (m, 1 H) 3.74 - 3.82 (m, 2 H) 3.90 (t, *J*=7.83 Hz, 1 H) 7.04 - 7.24 (m, 1 H) 7.27 (t, *J*=7.70 Hz, 1 H) 7.66 (d, *J*=7.83 Hz, 2 H)

### EXAMPLE 109

### 1-(3-amiao-3-oxopropyl)-3-(benzo[d]thiazol-2-yl)-1-(3,3-diphenylpropyl)urea

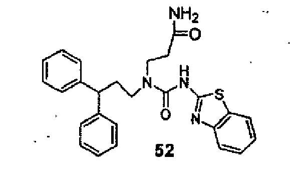

The procedure described in Example 68 was used to prepare **52** from 3-(3-(benzo[d]thiazol-2-yl)-1-(3,3-diphenylpropyl)ureido)propanoic acid. LC-MS ESI (pos.) m/e: 459 (M+H)

1H NMR (500 MHz, *MeOH)* δ ppm 7.74 (1 H, d, *J=*7.7 Hz), 7.51 (1 H, d, *J*=8.0 Hz), 7.35. - 7.40 (1 H, m), 7.30 - 7.34 (4 H, m), 7.25 - 7.32 (4 H, m), 7.21 - 7.26 (2 H, m), 7.1 7.20 (2 H, m), 4.02 (1 H, t, *J*=7.8 Hz), 3.25 - 3.32 (2 H, m), 2.38 (2 H,m), 1.96 - 2.10 (2 H, m), 1.72 - 1.84 (2 H, m)

### EXAMPLE 110

### 1-(3-amino-3-oxopropyl)-3-(5-chloro-4-phenylthiazol-2-yl)-1-(3,3-diphenylpropyl)urea

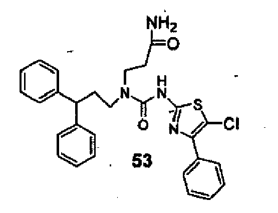

The procedure described in Example 68 was used to prepare **52** from 3-(3-(5-chloro-4-phsnylthiazol-2-yl)-1-(3,3-diphenylpropyl)ureido)propanoic acid. LC-MS ESI (pos.) m/e: 519 (M+H)

1H NMR (500 MHz, *CHLOROFORM-d) δ* ppm 7.89 (1 H, d, *J*=7.3 Hz), 7.67 - 7.73 (2 H, m), 7.47 - 7.57 (3 H, m), 7.28 - 7.34 (7 H, m), 7.17 - 7.23 (2 H, m), 3.99 (1 H, t, J=7.6 Hz), 3.55 - 3.69 (2 H, m), 3.46 - 3.54 (1 H, m), 3.38 - 3.46 (2 H, m), 2.71 (1 H, t, *J*=.7 Hz), 2.50-2.59 (2H, m), 2.35-2.48(2H,m)

### EXAMPLE 111

### 4-(1-(3,3-diphenylpropyl)-3-(4-phenylthiazol-2-yl)ureido)butanoic acid

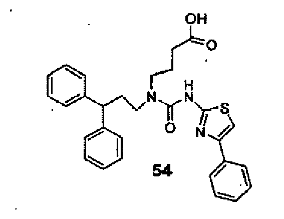

The procedure described in Example 67 was used to prepare 54 using 2-amino phenyl thiazole instead of N-(4-(2-aminothiazol-4-yl) phenyl)methanesulfonamide in Step 2.

LC-MS ESI (pos.) m/e: 500 (M+H); 1H NMR (500 MHz, DMSO-*d*₆) δ ppm 1.60 -1.73 (m, 2 H) 2.22 (t, *J*=7.2 Hz, 2 H) 2.28 - 2.36 (m, 2 H) 3.26 (dd, *J*=16.14,9.05 Hz, 4 H) 3.98 - 4.07 (m, 1 H) 7.18 (t, *J*=7.83 Hz, 2 H) 7.30 (t, *J*=7.58 Hz, 5 H) 7.33 - 7.38 (m, 4 H) 7.41 (t, *J*=7.58 Hz, 2 H) 7.45 (s, 1 H) 7.90 (d, *J=*8.31 Hz, 2H)

### EXAMPLE 112

### 3-(benzo[d]thiazol-2-yl)-1-(3-(cyclohezylamino)-3-oxop)opyl)-1-(3,3-diphenylpropyl)urea

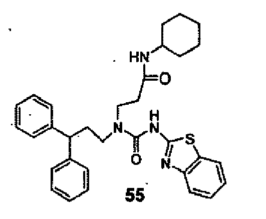

The procedure described in Example 67 was used to prepare 55 using 2-amino benzothiazole instead of N-(4-(2-aminothiazol-4-yl) phenyl)methanesulfonamide in Step 2, cyclohexylamine instead of (R)-tert-butyl 3-aninopiperidine-1-carboxylate in Step 4, and tert-butyl 3-bromopropanoate instead of tert-butyl 4-bromobutanoate in Step 1. LC-MS ESI (pos.) m/e: 541 (M+H); 1H NMR (500 MHz, CHLOROFORM-*d*) δ ppm 0.84 - 0.94 (m, 2 H) 1.02-1.19(m,4H) 1.56 1.72 (m, 4 H) 1.82 - 1.90 (m, 2 H) 2.38 - 2.48 (m, 4 H) 3.26 - 3.32 (m, 2 H) 3.59 - 3.65 (m, 2 H) 3.82 (br. s., 1 H) 3.98 (t, *J=7.70* Hz, 1 H) 5.57 (br, s., 1H) 7.17 - 7.35 (m, 11 H) 7.39 (t, *J*=7.34 Hz, 1 H) 7.76 (d,*J*=7.83 Hz, 2 H)

### EXAMPLE 113

### 3-(1-(3,3-diphenylpropyl)-3-(4-(4-(methylsulfonmnido)phenyl)thiazol-2-yl)ureido)propanoic acid

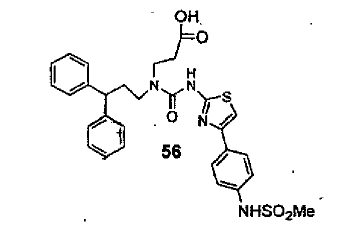

The procedure described in Example 67 was used to prepare **56** using tert-butyl 3-bromopropanoate instead of tert-butyl 4-bromobutanoate in Step 1. LC-MS ESI (neg.) m/e: 577.0 (M-H)

1H NMR (400 MHz, *CHLOROFORM-d*) δ ppm 8.02 (2 H, d, *J*=7.8 Hz), 7.97 (2 H, dd, *J*=7.8, 1.6 Hz), 7.72 - 7.81 (2H, m), 7.51 - 7.62 (2 H, m), 7.38 - 7.47 (3 H, m), 7.34 (2 H, t,*J=*7.4 Hz), 7.13 - 7.21 (2 H, m), 4.47 - 4.70 (2 H, m), 4.25 (1 H, t, *J=*5.5 Hz), 3.98 - 4.17 (2 H, m), 3.34 - 3.59 (4 H, m), 2.83 - 3.05 (2 H, m)

### EXAMPLE 114

### 3-(3-(benzo[d]thiazol-2-yl)1-(3,3-diphenylpropyl)ureido)propanoic acid

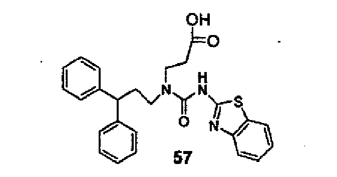

The procedure described in Example 67 was used to prepare **57** using 2-amino, benzothiazole instead of N-(4-(2-aminothiazol-4-yl) phenyl)methanesulfonamide in Step 2, and tert-butyl 3-bromopropanoate instead of tert-butyl 4-bromobutanoate in Step 1. LC-MS ESI (pos.) m/e: 460 (M+H); 1H NMR (500 MHz, DMSO-*d*₆) δ ppm 2.28 - 2.36 (m, 2H) 2.41 - 2.48 (m, 4H) 3.50 - 3.61 (m, 2 H) 4.01 (t, *J*=7.58 Hz, 1 H) 7.13 - 7.22 (m, 3 H) 7.29 (t, *J*=7.70 Hz, 4 H) 7.31 - 7.38 (m, 5 H) 7.50 (br. s., 1 H) 7.80 (d, *J=*7.58 Hz, 1 H)

### EXAMPLE 115

### (S)-3-(benzo[djthiazol-2-yl)-1-(3,3-diphenylpropyl)-1-(3-(3-methylpiperazin-1-yl)-3-oxopropyl)urea

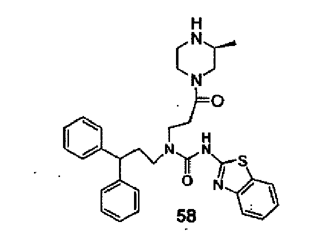

The procedure described in Example 67 was used to prepare 58 using 2-amino benzothiazole instead of N-(4-(2-aminothiazol-4-yl) phenyl)methanesulfonamide in Step 2, (S)-tert-butyl 2-methylpiperazine-1-carboxylate instead of (R)-tert-butyl 3-aminopiperidme-1-carboxylate in Step 4, and tert-butyl 3-bromopropanoate instead of tertbutyl 4-bromobutanoate in Step 1.

LC-MS ESI (pos.) m/e: 542 (M+H); 1H NMR (400 MHz, CHLOROFORM-*d)* δ ppm 0.94 - 1.02 (m, 3 H) 2.15 - 2.26 (m, 1 H) 2.30 - 2.47 (m, 4 H) 2.53 - 2.69 (m, 3 H) 2.85 - 3.04 (m, 2 H) 3.10 - 3.25 (m, 2 H) 3.40 - 3.64 (m, 3 H) 3.88 (t, *J*=7.63 Hz, 114) 4.41 - 4.55 (m, 1 H) 7.06 - 7.16 (m, 3 H) 7.16 - 7.23 (m, 8 H) 7.27 (t, *J*=7.63 Hz, 1 H) 7.65 (d, *J=*7.82 Hz, 2 H)

### EXAMPLE 116

### ethyl 4-(3-(benzo[dtthiazol-2-yl)-l-(3,3-diphenylpropyl)ureido)butanoate

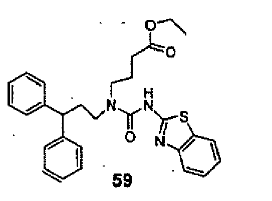

The procedure described in Example 67 was used to prepare 59 using 2-amino benzothiazole instead of N-(4-(2-ammothiazol-4-yl) phenyl)methanesulfonamide in Step 2, ethyl- 3-bromopropanoate instead of tert-butyl 4-bromobutanoate in Step 1 . LC-MS ESI (pos.) m/e: 502 (M+H); 1H NMR (500 MHz, CHLOROFORM-*d*) δ ppm 1.09 -1.16 (m, 4 H) 1.69 - 1.77 (m, 2 H) 2.21 (t, J=6.72 Hz, 2 H) 2.23 - 2.32 (m,2 H) 3.15 - 3.26 (m, 4 H) 3.83 (t, *J*=7.83 Hz, 1 H) 4.05 - 4.12 (m, 2 H) 7.05 (t, *J*=6.97 Hz, 2. H) 7.08 - 7.19 (m, 9 H) 7.25 (t, *J*=7.58 Hz, 1 H) 7.58 (dd, *J*=32.28, 7.83 Hz, 2 H)

### EXAMPLE 117

### 3-(benzo[d]thiazol-2-yl)-l-(3-(benzylamino)-3-oxopropyl)-1-(3,3-diphenylpropyl)urea

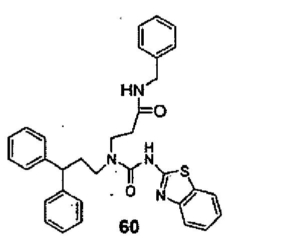

The procedure described in Example 67 was used to prepare 60 using 2-amino benzothiazole instead of N-(4-(2-aminothiazol-4-yl) phenyl)methanesulfonamide in Step 2, benzylamine instead of (R)-tert-butyl 3-aminapiperidine-1-carboxylate in Step 4, and tert-butyl 3-bromopropanoate instead of tert-butyl 4-bramobutarzoate in Step 1. LC-MS ESI (pos.) m/e: 549 (M+H); 1H NMR (500 MHz, CHLOROFORM-*d*) δ ppm 2.38 - 2.46 (m, 2 H) 2.46 - 2.51 (m, 2 H) 3.23 - 3.32 (m, 2 H) 3.61 - 3.68 (m, 2 H) 3.97 (t, *J=*7.58 Hz, 1 H) 4.44 (d, *J*=5.62 Hz, 2 H) 6.12 (br. s., 1 H) 7.15 - 7.34 (m, 11 H) 7.39 (t, *J*=7.70 Hz, 1 H) 7.66 - 7.78 (m, 2 H)

### EXAMPLE 118

### 3-(benzo[d]thiazol-2-yl)-1-(3,3-diphenylpropyl)-1-(4-(4-isopropylpiperazin-l-yl)-4-oxobutyl)urea

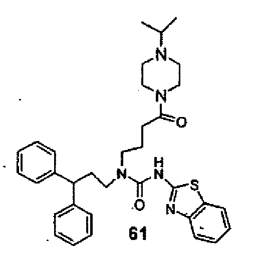

The procedure described in Example 57 was used to prepare **61** using 2-amino benzothiazole instead of N-(4-(2-aminothiazol-4-yl) phenyl)methanesulfonamide in Step 2, and 1-isopropylpiperazineinstead of (R)-tert-butyl 3-aminopiperidine-1-carboxylate in Step 4.

LC-MS ESI (pos.) m/e: 584 (M+H); 1H NMR (500 MHz, CHLOROFORM-*d*) δ ppm 1.16 - 1 .30 (m, 4 H) 1.44 (d, *J*=6.36 Hz, 3 H) 1.49 (d, *J*=5.38 Hz, 3 H) 1.51 - 1.55 (m, 2 H) 1.81 - 1.88 (m, 2 H) 2.36 - 2.47 (m, 3 H) 3.11 - 3.19 (m, 4 H) 3.27 - 3.41 (m, 2 H) 3.66 - 3.74 (m, 1 H) 3.96 - 4.02 (m, 1 H) 7.14 - 7.40 (m, 12 H) 7.75 (d, *J*=7.82 Hz, 2 H)

### EXAMPLE 119

### 3-(1-(3,3-diphenylpropyl)-3-(4-(4-(methylsulfonyl)phenyl)thiazol-2-yl)ureido)propanoic acid

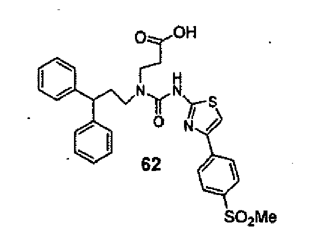

The procedure described in Example 67 was used to prepare **62** using 4-(4-(methylsulfonyl) phenyl)thiazol-2-amine instead of N-(4-(2-aminothiazoi-4-yl)phenyl)methanesulfonamide in Step 2, and tert-butyl 3-bromopropanoate instead of tert-butyl 4-bromobutanoate in Step 1 .

LC-MS ESI (pos.) m/e: 554.1 (M+H)

1H NMR (400 MHz, *DMSO-d₆*) δ ppm 8.12 - 8.18 (2 H, m), 7.91 - 8.00 (2 H, m), 7.76 (1 H, s), 7.32 - 7.40 (4 H, m), 7.28 (4 H, t, *J*=7.8 Hz), 7.11 - 7.20 (2 H, m), 4.01 (1 H, t, *J*=7.6 Hz), 3.47 - 3.60 (2 H, m), 3.27 (3 H, t), 3.23 (3 H, s), 2.46 (2 H, t, *J*=7.0 Hz), 2.31 (2 H, q)

### EXAMPLE 120

### (R)-3-(benzo[d]thiazol-2-yl)-1-(3,3-diphenylpropyl)-1-(3-(2-methylpiperazin-1-yl)-3-oxopropyl)urea

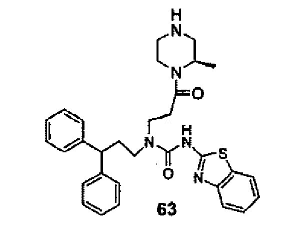

The procedure described in Example 67 was used to prepare **63** using 2-amino benzothiazole instead of N-(4-(2-aminothiazol-4-yl) phenyl)methanesulfonamide in Step 2, (R)-tert-butyl 3-methylpiperazine-1-carboxylateinstead of (R)-tert-butyl 3-aminopiperidine-1-carboxylate in Step 4, and tert-butyl 3-bromopropanoate instead of tert-butyl 4-bromobutanoate in Step 1.

LC-MS ESI (pos.) m/e: 542 (M+H); 1H NMR (400 MHz, CHLOROFORM-*d*) δ ppm 1.19 (dd, 4 H) 2.24 - 2.43 (m, 4 H) 2.72 2.83 (m, 5 H) 2.86 (s, 3 H) 2.91 - 3.01 (m, 1H) 3.05 - 3.42 (m, 4 H) 3.44 - 3.66 (m, 2 H) 3.88 (t, *J*=7.63 Hz, 1 H) 7.04 - 7.14 (m, 3 H) 7.14 - 7.23 (m, 7 H) 7.25 (t, *J*=7.63 Hz, 1 H) 7.63 (d, *J*=7.43 Hz, 2 H) 7.92 (s, 1 H)

### EXAMPLE 121

### 3-(3-(5-chloro-4-phenylthiazol-2-yl)-1-(3,3-diphenylpropyl)ureido)propanoic acid

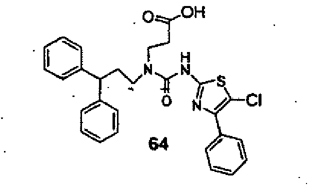

The procedure described in Example 67 was used to prepare **64** using 2-amino, 5-chloro phenyl thiazole instead of N-(4-(2-aminothiazol-4-yl) phenyl)methanesulfonamide in Step 2, and tert-butyl 3-bromopropanoate instead of tert-butyl 4-bromobutanoate in Step 1.

LC-MS ESI (neg.) mule: 518 (M-H)

1H NMR (500 MHz, *DMSO-d₆*) δ ppm 11.13 (1 H, s), 7.87 (2 H, d, *J*=6.7 Hz), 7.47 (2 H, t, *J*=7.6 Hz), 7.36 - 7.41 (1 H, m, *J*=7.3, 7.3 Hz), 7.32 - 7.36 (4 H, m), 7.28 (4 H, t, *J*=7.6 Hz),-7.10 - 7.20 (2 H, m), 4.33 - 5.90 (2 H, m), 4.00 (1 H, t, *J*=7.6 Hz), 3.41 --3.60 (2 H, m), 3.18 - 3.35 (2 H, m), 2.45 (2 H, t, *J*=7.0 Hz), 2.23 - 2.37 (2 H, m)

### EXAMPLE 122

### 3-(3-(benzofdjthiazol-2-yl)-I-(3,3-diphenylpropyl)ureido)-2-methylpropanoic acid

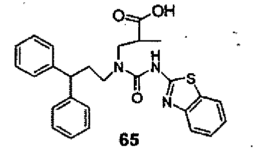

The procedure described in Example 69 was used to prepare **65** using 2-amino benzothiazole instead of 2-amino phenyl thiazole in Step 8.

LC-MS ESI (neg.) m/e: 472.1(M-H)

1H NMR (400 MHz, *DMSO-d₆*) δ ppm 7.84 - 7.93 (2 H, m), 7.44 - 7.48 (1 H, m), 7.38 - 7.44 (2 H, m, *J*=7.6, 7.6 Hz), 7.31 - 7.37 (4 H, m, *J*=7.4, 7.4 Hz), 7.24 - 7.31 (4 H, m), 7.11 1 - 7.20 (2 H, m, *J*=6.8, 6.8 Hz), 4.01 (1 H, t, *J*=7.8 Hz), 3.38 - 3.51 (2 H, m, *J*=6.3 Hz), 3.16 - 3.28 (1 H, m), 2.56 - 2.69 (2 H, m), 2.16 - 2.38 (2 H, m, *J*=13.5, 13.5 Hz), 1.00 (3 H, d, *J*=7.0 Hz)

### EXAMPLE 123

### (S)-3-(benzo[d]thiazol-2-yl)-1-(3,3-diphenylpropyl)-1-(3-(2-methylpiperazin-1-yl)-3-oxopropyl)urea

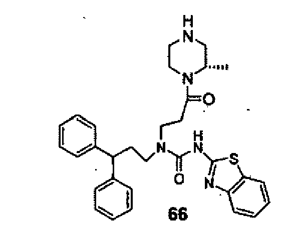

The procedure described in Example 67 was used to prepare **66** using 2-amino benzothiazole instead of N-(4-(2-aminothiazol-4-yl) phenyl)methanesulfonamide in Step 2, (S)-tert-butyl 3-methylpiperazine-1-carboxylate instead of (R)-tert-butyl 3-aminopiperidine-1-carboxylate in Step 4, and tert-butyl 3-bromopropanoate instead of tert-butyl 4-bromobutanoate in Step 1.

LC-MS ESI (pos.) m/e: 542 (M+H); 1H NMR (400 MHz, CHLOROFORM-*d*) δ ppm 1.13 3 - 1.30 (m, 4 H) 2.22 - 2.47 (m, 4 H) 2.75 - 2.84 (m, 5 H) 2.88 (s, 3 H) 2.92 - 3.03 (m, *J*=11.74 Hz, 1 H) 3.09 - 3.44 (m, 4 H) 3.47 - 3.69 (m, 2 H) 3.90 (t, *J*=7.83 Hz, 1 H) 7.07 - 7,17. (m, 3 H) 7.17 - 7.25 (m, 7 H) 7.28 (t, *J*=7.63 Hz, 1 H) 7.66 (d, *J*=7.82 Hz, 2 H) 7.94 (s, 1 H)

### EXAMPLE 124

### 4-(1-(3J-diphenylpropyl)-3-(4-(4-sulfonamido)phenyl)thiazol-2-yl)ureido)butanoic acid

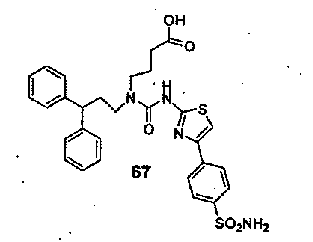

The procedure described in Example 67 was used to prepare **67** using 4-(2-aminothiazol-4-yl)benzenesulfonamide instead of N-(4-(2-aminothiazol-4-yl)phenyl)methanesulfonamide in Step 2, only one equivalent of CDI and no DMAP in Step 2.

LC-MS ESI (pos.) m/e: 579 (M+H)

1H NMR (400 MHz, *DMSO-*d₆) δ ppm 10.59 - 11.00 (1 H, m), 8.07 (2 H, dt, *J*=8.6, 2.0 Hz), 7.86 (2 H, dt, *J*=8.7, 1.9 Hz), 7.65 (1 H, s), 7.32 - 7.40 (6 H, m), 7.25 - 7.32 (4 H, m), 7.17 (2 H, tt, *J*=7.2, 1.4 Hz), 4.01 (1 H, t, *J*=7.6 Hz), 3.67 - 3.95 (2 H, m), 3.28 (4 H, dt, *J*=30.0, 7.5 Hz), 2.31 (2 H, q), 2.21 (2 H, t, *J*=7.4 Hz), 1.59 - 1.75 (2 H, m,

### EXAMPLE 125

### (R)-3-(benzoldlthiazol-2-yl)-1-(3,3-diphenylpropyl)-1-(3-(3-methylpiperazin-1-yl)-3-oxopropyl)urea

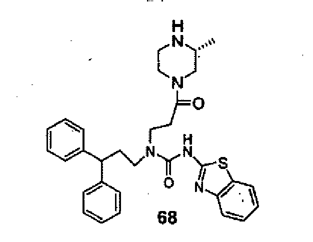

The procedure described in Example 67 was used to prepare **68** using 2-amino benzothiazole instead of N-(4-(2-amhxotlliazol-4-yl)phenyl)metllanesulfonanfde in Step 2, (R)-tert-butyl 2-methylpiperazine-1-carbaxylate instead of (R)-tert-butyl3-aminopiperidine-1-carboxylate in Step 4, and tert-butyl 3-bromopropanoate instead of tert-butyl 4-bromobutanoate in Step 1 .

LC-MS ESI (pos.) m/e: 542 (M+H); 1H NMR (400 MHz, CHLOROFORM-*d*) δ ppm 0.77 -0.90 (m,4H) 0.97 (d, *J*=6.26 Hz, 3H) 1.15-1.41 (m, 6H) 1.55-1.68 (m, 1H) 2.14-2.27 (m, 1 H) 2.29 - 2.49 (m, 4 H) 2.53 - 2.72 (m, 3 H) 2.84 - 3.07 (m, 1 H) 3.10 - 3.27 (m, 2 H) 3.40 - 3.65 (m, 3 H) 3.88 (t, *J*=7.83 Hz, 1 H) 4.07 - 4.22 (m, 1 H) 4.40 - 4.55 (m, *J*=12.13 Hz, 1 H) 7.16 - 7.23 (m, 7 H) 7.26 (t, *J*=7.63 Hz, 1 H) 7.44 (dd, *J*=5.48, 3.52 Hz, 1 H) 7.55 - 7.76 (m, 2 H)

### EXAMPLE 126

### 3-(1-(3,3-dipbenylpropyl)-3-(4-phenylthiazol-2-yl)ureido)propanoic acid

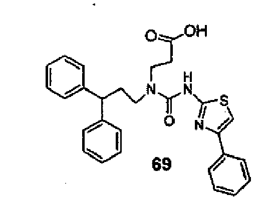

The procedure described in Example 67 was used to prepare **69** using 2-amino phenyl, thiazole instead of N-(4-(2-aminothiazol-4-yl) phenyl)methanesulfonamide in Step 2, and tert-butyl 3-bromopropanoate instead of tert-butyl 4-bromobutanoate in Step 1. LC-MS ESI (pos.) m/e: 486 (M+H);

1H NMR (400 MHz, *DMSO-d₆*) δ ppm 12.29 (1 H, s), 10.86 (1 H, s), 7.88 (2 H, t, *J*=7.0 Hz), 7.45 (1 H, s), 7.40 (2 H, t, *J*=7.6 Hz), 7.33 - 7.37 (4 H, m), 7.25 - 7.32 (5 H, m), 7.17 (2 H, t, *J*=7.2 Hz), 4.01 (1 H, t, *J*=7.6 Hz), 3.53 (2 H, t, *J*=6.8 Hz), 3.20 - 3.29 (2 H, m), 2.46 (2 H, t, *J*=7.0 Hz), 2.31 (2 H, q, *J*=7.7 Hz)

### EXAMPLE 127

### 3-(benzo[d]thiazol-2-yl)-1-(3,3-diphenylpropyl)-1₋(4-(piperidine-4-carboxamido)butyl)urea (9)

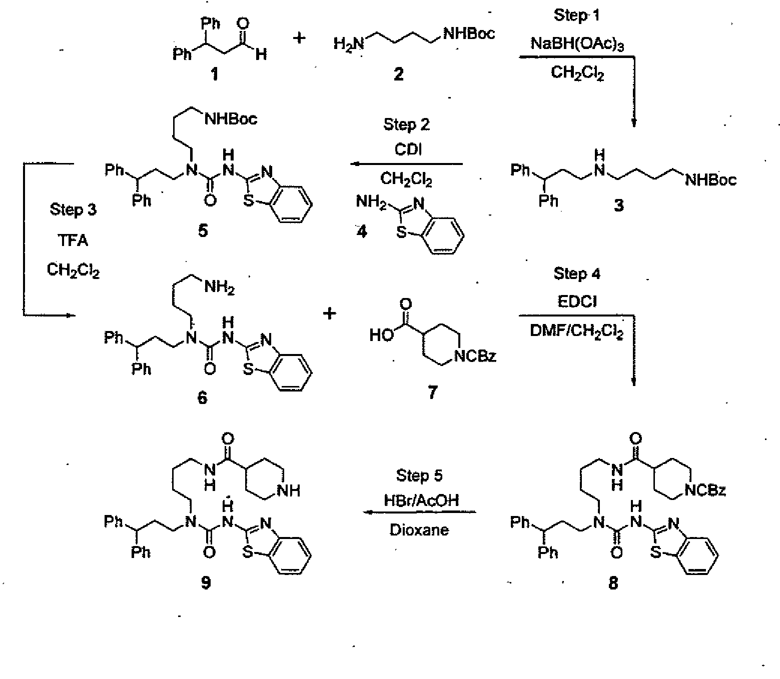

**Step 1:** To a solution of 3,3-diphenylpropanal **1** (1.05 g, 5 mmol) and tert-butyl 4-aminobutylcarbamate **2** (1.18 g, 6.25 mmol) in CH₂Cl₂ (32 ml) was added sodium triacetoxyborohydride (2.65 g, 12.5 mmol). The reaction mixture was stirred for 4 h at 25 °C. The reaction mixture was poured into 30 mL of aqueous saturated NaHCO₃, allowed to stir for 1 h, and extracted with dichloromethane (3 x 20 mL). The combined organic extracts were dried with magnesium sulfate, filtered, and concentrated. The residue was purified by chromatography on silica using 0 -> 10% MeOH/CH₂Cl₂ + 0 -> 1% NH₄OH as eluent.) to yield **3** as a faint yellow oil (1.12 g, 58% yield).

LCMS (ES) calcd for C₂₄H₃₄N₂O₂ 382.2 found 383.2 (MH⁺).

**Step 2:** To a solution of benzo [d]thiazol-2-amine **4** (378 mg, 2.52 mmol) in CH₂Cl₂ (10 ml) was added di(1H-imidazol-1-yl)methanone (162 mg, 1 mmol). The reaction mixture was stirred overnight at 35 °C. Tert-butyl 4-(3,3-diphenylpropylamino)butylcarbamate **3** (1.12 g, 2.94 mmol) was added and the reaction mixture was heated to 35 °C overnight. The reaction mixture was poured into 20 mL of aqueous saturated NaHCO₃ and extracted with dichloromethane (3 x 20 mL). The combined organic extracts were dried with magnesium sulfate, filtered, and concentrated. The residue was purified by chromatography on silica using EtOAc/Hexanes as eluent to give **5** as a white solid (500 mg, 35.5% yield).

LCMS (ES) calcd for C₃₂H₃₈N₄O₃S 558.3 found 559.3 (MH⁺).

**Step 3:** To a solution of tert-butyl 4-(3-(benzo[d]thiazol-2-yl)-1-(3,3-diphenylpropyl)ureido)butylcarbamate (500 mg, 895 µmol) in CH₂Cl₂ (5 ml) was added an equivalent volume of TFA (5 mL). The reaction mixture was stirred for 4 h at of 25 °C. Volatiles were removed by concentration under reduced pressure. The leftover residue was solubilized with 60 mL of a 1:1 mixture of aqueous saturated NaHCO₃ and Dichloromethane. The organic phase was separated and the aqueous phase was extracted further with Dichloromethane (2 x 20 mL). The combined organic extracts were dried with magnesium sulfate, filtered, and concentrated. The amine **6** was concentrated to an off white foam that was used without further purification.

**Step 4:** To a solution of 1-(benzylaxycarbonyl)piperidine-4-carboxylic acid **7** (115 5 mg, 0.435 mmol) in 9:1 CH₂Cl₂/DMF (10 mL) was added *N*-(3-dimethylaminopropyl)-*N*'-ethylcarbodiimide hydrochloride (94.5 mg, 0.493 mmol). The reaction mixture was stirred for 30 min at room temperature. Then 1-(4-aminobutyl)-3-(benzo[d]thiazol-2-yl)-1-(3,')-diphenylpropyl)urea (133 mg, 0.290 mmol) 6 was added and the reaction mixture was heated to 39 °C overnight. The reaction mixture was poured into 50 mL of water and extracted with ethyl acetate (3 x 20 mL). The combined organic extracts were dried with magnesium sulfate, filtered, and concentrated. The residue was purified by chromatography on silica using EtOAc/Hexanes as eluent to give **8** as a clear oil (119 mg, 58% yield).

LCMS (ES) calcd for C₄₁H₄₃NₛO₄S 703.3 found 704.3 (MH⁺).

**Step 5:** To a solution of benzyl 4-((4-(3-(benzo[d]thiazol-2-yl)-1-(3,3-diphenylpropyl)ureido)butyl)carbamoyl)piperidine-1-carboxylate **8** (119 mag, 169 µmol) in dioxane (1.5 ml) was added 0.5 mL of HBr 33% by wt. in AcOH. The reaction mixture was stirred for 40 min at 25 °C. Volatiles were removed by concentration under reduced pressure. The residue was re-concentrated twice from heptane (2 x 10 mL). Finally, the leftover residue was purified by injection as a solution in DMF on a reverse phase HPLC column using 5 -> 95% CH₃CN/0.1% aqueous TFA as eluent to give **9** as a white solid (69.7 mg, 60% yield). Significant broadening due to rotamers was observed in NMR spectra.

¹H NMR (400 MHz, CHLOROFORM-*d*) δ ppm 1.41 - 1.7 (m. 4 H) 1.91 - 2.15 (m, 4 H) 2.30 - 2.60 (m, 3 H) 2.88 - 3.13 (m, 2 H) 3.17 - 3.57 (m, 8 H) 7.15 - 7.23 (m, 2 H) 7.26-7.34 (m, 8 H) 7.45 (t, *J*=7.63 Hz, 1 H) 7.56 (t, *J*=7.63 Hz, 1 H) 7.79 (dd, *J*=7.83, 4.30 Hz, 2 H) 8.88 (br. s., 1 H) 9.53 (br. s., 1 H);

LCMS (ES) calcd for C₃₃H₃₉N₅O₂S 569.3 found 570.3 (MH⁺).

### EXAMPLE 128

### 3-(benzo[d]thiazol-2-yl)-1-(3,3-diphenylpropyl)-1-(4-(piperidine-2-carboxamido)butyl)urea (10)

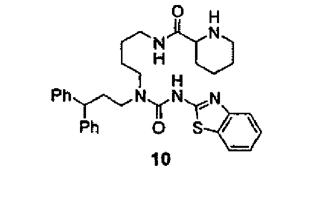

The procedure described in Example 127 with the exception of substituting 1-(benzyloxycarbonyl)piperidine-2-carboxylic acid for 1-(benzyloxycarbonyl)piperidine-4-carboxylic acid 7 in Step 4,was used to prepare 3-(benzo[d]thiazol-2-yl)-1-(3,3-diphenylpropyl)-1-(4-(piperidine-2-carboxamido)butyl)urea **10** as a white solid. Significant broadening due to rotamers was observed in NMR spectra.

¹H NMR (400 MHz, CHLOROFORM-*d*) δ ppm 1.42 - 1.66 (m, 5 H) 1.68 - 1.97 (m, 4 H) 2.03-2.15 (m, 1 H) 2.32 - 2.48 (m, 2 H) 3.02 - 3.16 (m, 1 H) 3.20 - 3.57 (m, 7 H) 3.93 - 4.08 (m, 2 H) 7.13 - 7.23 (m, 2 H) 7.25 - 7.35 (m, 7 H) 7.42 (t, *J*=7.63 Hz, 1 H) 7.49 - 7.58 (m, 1 H) 7.60 - 7.69 (m, 1 H) 7.69 - 7.76 (m, 1 H) 7.79 (d, *J*=7.82 Hz, 1 H); LCMS (ES) calcd for C₃₃H₃₉N₅O₂S 569.3 found 570.3 (MH⁺).

### EXAMPLE 129

### 3-(benzo[d]thiazol-2-yl)-1-(3,3-diphenylpropyl)-1-(2-(piperidine-3-carboxamido)ethyl)urea (11)

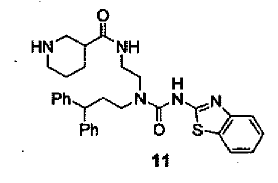

The procedure described in Example 127 with the exceptions of substituting tert-butyl 2-aminoethylcarbamate for tert-butyl 4-aminobutylcarbamate **2** in Step 1 and of substituting 1-(benzyloxycarbonyl)pipendine-3-carboxylic acid for 1-(benzyloxycarbonyl)piperidine-4-carboxylic acid 7 in Step 4 was used to prepare 3-(benzo[d]thiazol-2-yl)-1-(3,3-diphenylpropyl)-1-(2-(piperidine-3-carboxamido)ethyl)urea **11** as a white solid. Significant broadening due to rotamers was observed in NMR spectra. ¹H NMR (400 MHz, CHLOROFORM-*d*) δ ppm 1.41 - 1.53 (m, 1 H) 1.53 - 1.77 (m, 2 H) 1.86 - 2.00 (m, 1 H) 2.40 (q, *J*=7.82 Hz, 2 H) 2.46 2.57 (m, 1 H) 2.65 - 2.75 (m, 1 H) 2.87 (dd, *J*=12.13, 3.13 3 Hz, 2 H) 3.02 (dd, *J*=11.35, 3.52 Hz, 1 H) 3.33 - 3.52 (m, 6 H) 3.95 (t, *J*=7.83 Hz, 1 H) 4.97 (br. s., 2 H) 7.16 - 7.39 (m, 10 H) 7.69 (d, *J*=8.22 Hz, 2 H) 7.72 (d, *J*=7.82 Hz, 2 H) 8.55 (br. s., 1 H);

LCMS (ES) calcd for C₃₁H₃₅N₅O₂S 541.3 found 542.3 (MH⁺).

### EXAMPLE 130

### 1-(3,3-diphenylpropyl)-3-(4-(4-(methylsulfonamido)phenyl)thiazol-2-yl)-1-(2-piperidine-3-carboxamido)ethyl)urea (12)

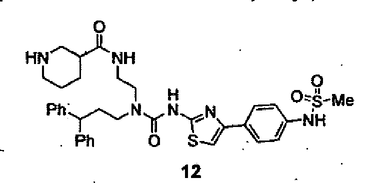

The procedure described in Example 127 with the exceptions of substituting tert-butyl 2-aminoethylcarbamate for tert-butyl 4-aminobutylcarbamate **2** in Step 1, of substituting N-(4-(2-aminothiazol4-yl)phenyl)methanesulfonamide for benzo[d]thiazol-2-amine **4** in Step 2, and of substituting 1-(benzyloxycarbonyl)piperidine-3-carboxylic acid for 1-{benzyloxycarbonyl}piperidine-4-carboxylic acid **7** in Step 4 was used to prepare 1-(3,3-diphenylpropyl)-3-(4-(4-(methylsulfonamido)phenyl)thiazol-2-yl)1-(2-(piperidine-3-carboxamido)ethyl)urea **12** as a white solid.

¹H NMR (500 MHz, MeOH) δ ppm 1.75 -1.85 (m, 2 H) 1.85 - 1.93 (m, 2 H) 2.38 - 2.49 (m,3 H) 2.93 (td, *J*=12.36, 3.36 Hz, 2 H) 2.99 - 3.03 (m, 3 H) 3.34 - 3.43 (m, 6 H) 3.50 (t, *J*=5.80 Hz, 2 H) 4.05 (t, *J*=7.63 Hz, 1 H) 7.19 (t, *J*=7.32 Hz, 2 H) 7.23 (s, 1 H) 7.28 - 7.33 (m, 6 H) 7.33 - 7.38 (m, 4 H) 7.87 (d, *J*=8.54 Hz, 2 H);

LCMS (ES) calcd for C₃₄H₄₀N₆O₄S₂ 660.3 found 661.2 (MH⁺).

### EXAMPLE 131

### 3-(5-chloro-4-(4-(methylsulfonamido)phenyl)thiazol-2-yl)-1-(3,3-diphenylpropyl)-1-(2-(piperidine-3-carboxamido)ethyl)urea (13)

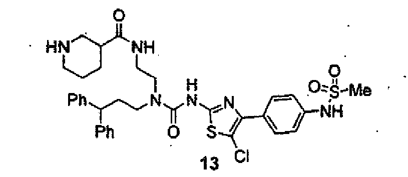

The procedure described in Example 127 with the exceptions of substituting tert-butyl 2-aminoethylcarbamate for tert-butyl 4-aminobutylcarbamate 2 in Step 1, of substituting N-(4-(2-amino-5-chlorothiazol-4-yl)phenyl)methanesulfonamide for benzo[d]ttuazol-2-amine **4** in Step 2, and of substituting 1-(benzyloxycarbonyl)piperidine-3-carboxylic acid for 1-(benzyloxycarbonyl)piperidine-4-carboxylic acid 7 in Step 4 was used to prepare 3-(5-chloro-4-(4-(methylsulfonamido)phenyl)thiazol-2-yl)-1-(3,3-diphenylpropyl)-1-(2-(piperidine-3-carboxamido)ethyl)urea **13** as a white solid. ¹H NMR (400 MHz, MeOH) δ ppm 1.73 - 1.93 (m, 4 H) 2.35 - 2.48 (m, 3 H) 2:95 (td, *J*=12.13, 3.52 Hz, 2 H) 3.02 (s, 3 H) 3.34 - 3.41 (m, 6 H) 3.47 (t, *J*=5.87 Hz, 2 H) 4.03 (t, *J*=7.83 Hz, 1 H) 7.14 - 7.21 (m, 2 H) 7.26 - 7.37 (m, 10 H) 7.91 - 7.97 (m, 2 H); LCMS (ES) calcd for C34H39C1N604S2 694.2 found 695.2 (MH⁺).

### EXAMPLE 132

### 2-(3-(5-chloro-4-(4-(methylsuvonamido)phenyl)thiazol-2-yl)-1-(3,3-diphenylpropyl)ureido)ethyl carbamate (1)

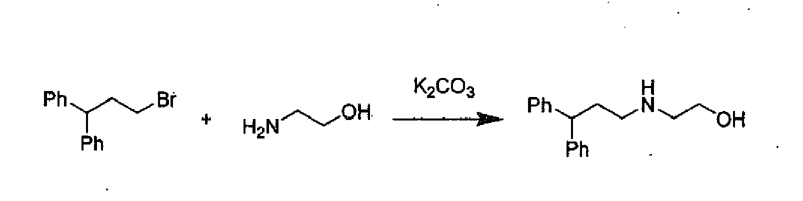

Step 1. **Procedure A**. A microwave compatible vial was charged with 3-bromo-1,1-diphenylpropane (5.02 g, 18.2 mmol), 2-aminoethanol (2.19 ml, 36.5 mmol), potassium carbonate (3.78 g, 27.4 mmol), and 8 mL af AcCN. The vial was sealed and purged with N₂ for 5 min. The reaction was subjected to microwave irradiation for 30 ruin at 130 °C. The solution was diluted with EtOAc and was washed with water and brine. The organic phase was dried over MgSO₄, filtered, and concentrated. The crude material was purified by ISCO column chromatography using a 10% to 90% gradient of 10% MeOH- CH₂Cl₂/ CH₂Cl₂ eluent. The desired fractions were combined and concentrated to give 2-(3,3-diphenylpropylamino)ethanol (3.20 g, 68.7% yield) as a colorless oil. Mass spectrum: calculated for C₁₇H₂₁NO 255.4; found 256.3 (M⁺ + 1).

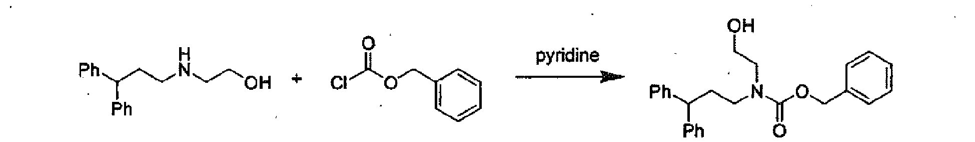

Step 2. A solution of 2-(3,3-diphenylpropylamino)ethanol (3.20 g, 13 mmol) and pyridine (1.2 ml, 15 mmol) in CH₂Cl₂ was chilled to 0 °C in an ice bath. To this solution was added Cbz-Cl (2.0 ml, 14 mmol) slowly *via* syringe. The reaction mixture was allowed to warm to room temperature and was stirred under N₂ for 4 h. The solution was diluted with CH₂Cl₂ and then washed with water and brine. The organic layer was dried over MgS0₄, filtered, and concentrated. The crude material was purified by ISCO column chromatography using a 5% to 70% gradient of EtOAc/hexane as eluent. The desired fractions were combined and concentrated to give benzyl 3,3-diphenylpropyl(2-hydroxyethyl)carbamate (3.41 g, 70% yield) as a colorless, viscous oil. Mass spectrum: calculated for C₂₅H₂₇NO₃ 389.5; found 390.4 (M⁺ + 1).

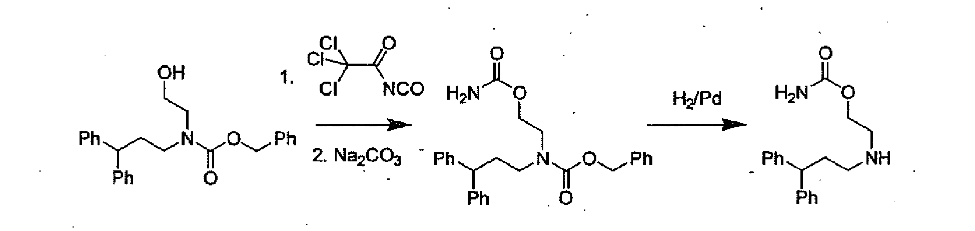

Step 3. A solution of benzyl 3,3-diphenylpropyl(2-hydroxyethyl)carbarnMe (150 mg, 385 µmol) in THF was chilled to 0 °C in an ice bath. To this solution was added 2,2,2-trichloroacetyl isocyanate (73 mg, 385 µµol) and the mixture was stirred at 0 °C for 3 h. The reaction was warmed to room temperature and stirred overnight under a N₂ atmosphere. The solvent was removed *in vacuo* and the crude material was dissolved with MeOH. Pd/C (41 mg, 385 gaol) was added to solution and the reaction flask was evacuated and purged with H₂ (0.7 mg, 385 µmol) three times. The reaction was stirred for 4 h under H₂ atmosphere using a balloon. The balloon was removed and the solution was purged with N₂ for 10 min. A solution of 1 U% aqueous Na₂CO₃ was added to the mixture and the reaction was stirred overnight. The mixture was filtered to remove the solid material and the filtrate was concentrated. The crude material was diluted with EtOAc and was extracted with saturated aqueous NaHC0₃ and brine. The organic layer was dried over MgSO₄, filtered, and concentrated. The crude material was purified by ISCO column chromatography using a 5% to 90% gradient of EtOAc/hexane as eluent. The desired fractions were combined and concentrated to give 2-(3,3-diphenylpropylamino)ethyl carbamate (71 mg, 62% yield) as a colorless oil.

Mass spectrum: calculated for C₁₈H₂₂N₂0₂ 298.4; found 299.3 (1via + 1).

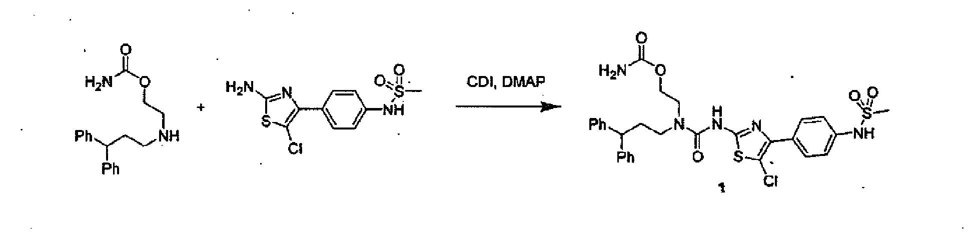

Step 4. **Procedure B**. A solution ofN-(4-(2-amino-5-chlorothiazol-4-yl)phenyl)-.. methanesulfonamide (134 mg, 442 µmol), DMAP (63 mg, 516 µmol), and CDI (84 mg, 516 µmol) in 3 mL of dry DMF was heated to 55°C under a N₂ atmosphere. After 18 h, a solution of 2-(3,3-diphenylpropylamino)ethyl carbamate (110 mg, 369 µmol) in 2 mL of DMF was added to the reaction mixture. The reaction temperature was increased to 85°C and the mixture was stirred for 8 h. The mixture was cooled to room temperature and then water was added to the reaction to precipitate the product. The precipitate was filtered and washed with water. The crude solid was dissolved in MeOH/ CH₂Cl₂, and was absorbed onto silica gel. The material was purified by ISCO column chromatography using a 10% to 90% gradient of 10%MeOH- CH₂Cl₂/ CH₂Cl₂ eluent. The desired fractions were combined and concentrated to give the product as a colorless oil. The product was converted to the HCI salt by adding 1 N HCl in ether to give 2-(3-(5-chloro-4-(4-(methylsulfonamido)phenyl)thiazol-2-yl)-1-(3,3-diphenylpropyl)ureido)ethyl carbamate hydrochloride (55 mg, 24% yield) as a white solid

Mass spectrum: calculated for C₂₉H₃₀CIN₅O₅S₂ 628.2; found z629.2 (M⁺ + 1) 1H NMR (400 MHz, CDCl₃) δ ppm 2.37-2.43 (q, 2 H) 2.91 (s, 3 H) 3.34-3.38 (m, 2H) 3.52-3.56 (m, 2 H) 3.98-4.02 (t, 1H) 4.15-4.20 (m, 2H) 6.98-7.01 (m, 2H) 7.17- 7.20 (m, 2H) 7.2b-7.31 (m, 8 H) 7.57-7.59 (d, 2 H).

### EXAMPLE 133

### 3-(5-chloro4-(4-(methylsulfonamido)phenyl)thiazol-2-yl)-1-(3,3-diphenylpropyl)-1-(2-hydroxyethyl)urea (2)

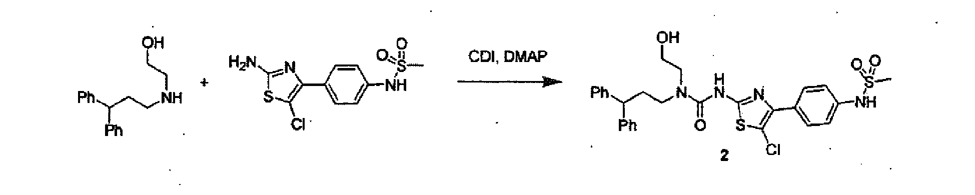

Step 1. The compound 2 shown above was prepared using Procedure B of Example 132. The reaction conditions yielded 3-(S-chloro-4-(4-(methylsulfonamido)phenyl)thiazol-2-yl)-1-(3 ,3-diphenylpropyl)-1-(2-hydroxyethyl)urea hydrochloride (144 mg, 74.8% yield) as a white solid. Mass spectrum: calculated for C₂₈H₂₉CIN₄O₄S₂ 585.1; found 589.2 (M⁺ + 1). 1H NMR (400 MHz, MeOH) δ ppm 2.40-2.46 (q, 2 H) 3.02 (s, 3 H) 3.37-3.48 (m, 4H) 3.69-3.73 (m, 2 H) 4.01-4-04 (t, 1H) 7.15-7.19 (m, 2H) 7.26-7.36 (m, 10 H) 7.87-7.89 (d, 2 H).

### EXAMPLE 134

### 3-(5-chloro4-(4-(methylsulfonamido)phenyl)thiazol-2-yl)-1-(3,3-diphenylpropyl)-1-(3-hydroxypropyl)urea (3)

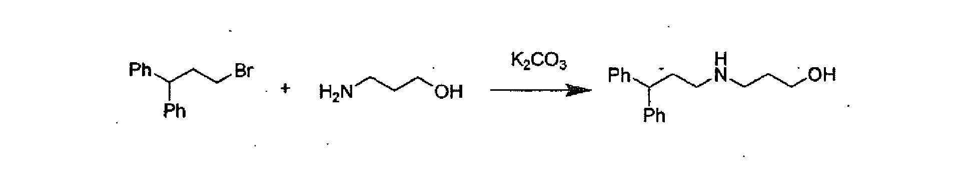

Step 1. The alcohol shown above was prepared using Procedure A of Example 132. The reaction conditions yielded 3-(3,3-diphenylpropylarnino)propan-1-ol (1.13 g, 58.4% yield) as a colorless oil.

Mass spectrum: calculated for C₁₈H₂₃NO 269.4; found 270.2 (M⁺ + 1).

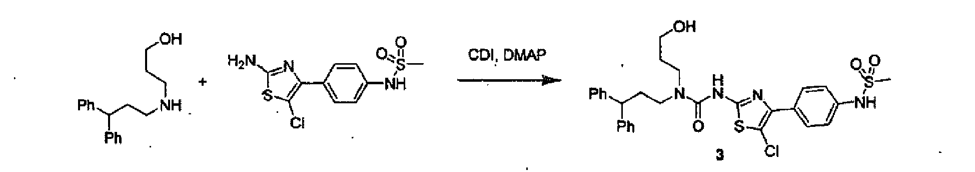

Step 2. The compound **3** shown above was prepared using Procedure B of Example 132. The reaction conditions yielded 3-(5-chloro-4-(4-(methylsulfonamido)phenyl)thiazol-2-yl)-1-(3,3-diphenylpropyl)-1-(3 -hydroxypropyl)urea hydrochloride (120 mg, 73.0% yield) as a white solid.

Mass spectrum: calculated for C₂₉H₃₁CIN₄0₄S₂ 599.2; found 600.3 (M⁺ + 1). 1H NMR (400 MHz, MeOH) δ ppm 1.72-1.75 (m, 2H) 2.39-2.44 (q, 2 H) 3.01 (s, 3 H) 3.32-3.36 (m, 2H) 3.40-3.44 (m, 2H) 3.57-3.59 (m, 2 H) 3.99-4.03 (t, 1H) 7.15-7.19 (m, 2H) 7.26-7.36 (m, 10 H) 7.86-7.90 (d, 2 H).

### EXAMPLE 1.35

### 1-(2-aminoethyl)-3-(5-chloro-4-(4-(methylsulfonamido)phenyl)thiazol-2-yl)-1-(3,3-diphenylpropyl)urea (4)

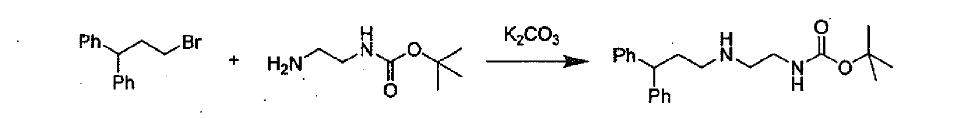

Step 1. The alcohol shown above was prepared using Procedure A of Example 132. The reaction conditions yielded tert-butyl 2-(3,3-diphenylpropylamino)ethylcarbamate (820 mg, 42.4% yield) as a colorless oil. Mass spectrum: calculated for C₂₂H₃₀N₂O₂ 354.5; found 355.4 (M⁺ + 1).

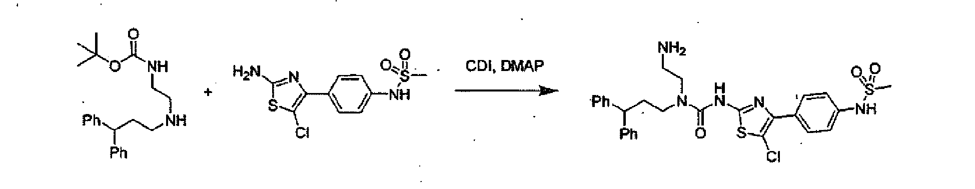

Step 2. The compound **4** shown above was prepared using Procedure B of Example 132 followed by stirring of the Boc protected intermediate with 2 mL of 4 N HC1 in dioxane and 10 mL of dichloromethane for 8 h at room temperature. Concentration of the reaction mixture yielded tert-butyl 2-(3-(5-chloro-4-(4-(methylsulfbnamido)phenyl)thiazol-2-yl)-1-(3,3-diphenylpropyl)ureido)ethylcarbamate hydrochloride (120 mg, 73.0% yield) as a white solid.

Mass spectrum: calculated for, C₂₈H₃₀CIN₅O₃S₂ 584.2; found 585.2 (M⁺ + 1). 1H NMR (400 MHz, MeOH) δ ppm 2.43-2.48 (m, 2 H) 3.02 (s, 3 H) 3.06-3.08 (m, 2H) 3.43-3.45 (m, 2 H) 3.60-3.62 (m, 2 H) 4:06-4.10 (t, 1H) 7.17-7.20 (m, 2H) 7.26-7.37 (m, 10 H) 7.91-7.93 (d, 2 H).

### EXAMPLE 136

### 2-[{[(5-chloro-4-{4-[(methylsulfionyl)amino]phenyl}-1,3-thiazol-2-yl)amino]carbonyl}(3,3-diphenylpropyl)amino]ethanesulfonamide (5)

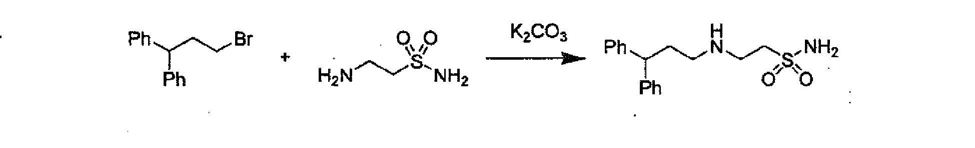

Step 1. The sulfonamide shown above was prepared using Procedure A of Example 132. The reaction conditions yielded 2-(3,3-diphenylpropylamino)ethanesulfonamide (101 mg, 43.6% yield) as a light yellow oil. Mass spectrum: calculated for C₁₇H₂₂N₂O₂S 318.4; found-319.3 (M⁺ + 1).

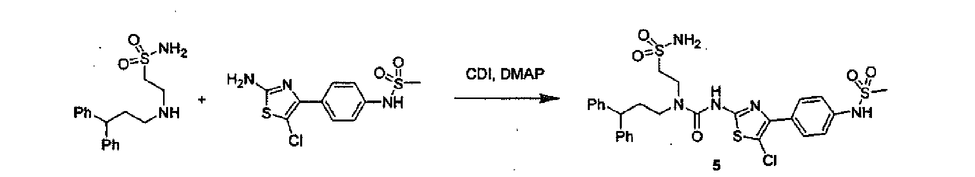

Step 2. The compound 5 shown above was prepared using Procedure B of Example 132. The reaction conditions yielded 2-[{[(5-chloro-4-{4-[(methylsulfonyl)amino]phenyl}-1,3-thiazol-2-yl)amino]carbonyl}(3,3-diphenylpropyl)amino]ethanesulfonamide hydrochloride (72 mg, 34% yield) as a yellow solid.

Mass spectrum: calculated for C₂₈H₃₀ClN₅O₅S₃ 648.2; found 649.2 (M⁺ + 1). 1H NMR (400 MHz, MeOH) δ ppm 2.42-2.47 (m, 2 H) 3.02 (s, 3 H) 3.30-3.35 (m, 2H) 3.41-3.45 (m, 2 H) 3.76-3.80 (m, 2 H) 4.04-4.06 (t, 1H) 7.15-7.19 (m, 2 H) 7.27-7.35 (m, 10 H) 7.89 - 7.92 (m, 2 H).

### EXAMPLE 137

### 3-(5-chloro-4-(4-(methylsulfonamido)phenyl)thiazol-2-yl)-1-(3,3-diphenylpropyl)-1-(2-(methythio)ethyl)urea (6)

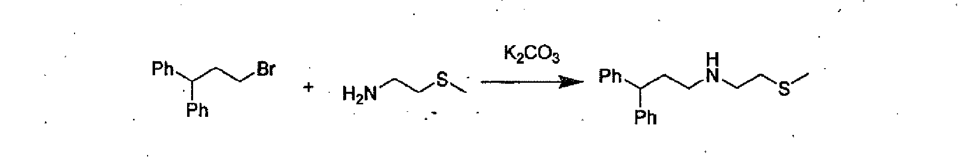

Step 1. The thioether shown above was prepared using Procedure A of Example 132. The reaction conditions yielded N-(2-(methylthio)ethyl)-3,3-diphenylpropan-1-amîne (180 mg, 86.8% yield) as a colorless oil.

Mass spectrum: calculated for C₁₈H₂₃NS 285.4; found 286.3 (M⁺ + 1).

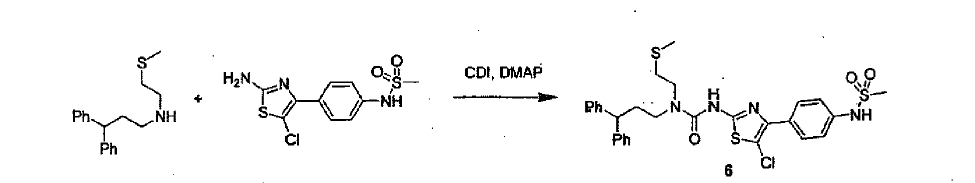

Step 2. The compound shown above was prepared using Procedure B of Example 132. The reaction conditions yielded 3-(5-chloro-4-(4-(methylsulfonamido)phenyl)thiazol-2-yl)-1-(3,3-diphenylpropyl)-1-(2-(methylthio)ethyl)urea hydrochloride (85 mg, 42% yield) as a white solid. Mass spectrum: calculated for C₂₉H₃₁CIN₄₀O₃S₃ G15.2; found 616.2 (M⁺ + 1).

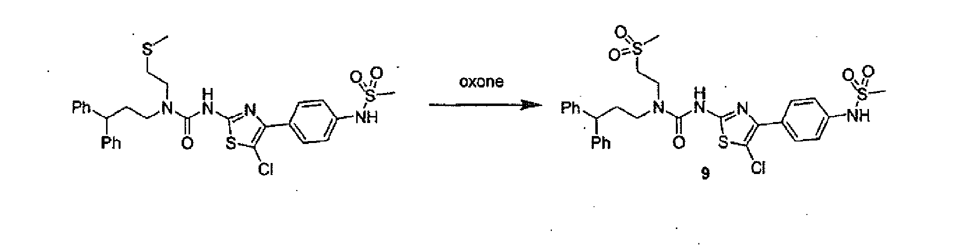

Step 3. Procedure C. A solution of 3-(S-chloro-4-(4-(methylsulfonamido)phenyl)-thiazol-2-yl)- 1-(3,3 -diphenylpropyl)-1-(2-(methylthio)ethyl)urea (60 mg, 98 µmol) in MeOH was chilled to 0 °C in an ice bath. To this solution was added a solution of oxone (90 mg, 146 µmol) in water. The mixture was slowly warmed to room temperature and stirred for 18 h. The solution was diluted with water and CH₂Cl₂. The aqueous layer was extracted with 3XCH₂Cl₂. The combined organic solution was washed with brine. The organic layer was dried over MgSO₄, filtered, and concentrated. The crude material was purified by ISCO column chromatography using 5% to 80% of 10%MeOH-CH₂Cl₂/CH₂Cl₂ eluent. The desired fractions were combined, concentrated, and converted to the HCl salt to give 3-(5-chloro-4-(4-(methylsulfonamido)phenyl)thiazol-2-yl)-1-(3,3-diphenylpropyl)-1-(2-(methylsulfonyl)ethyl)urea hydrochloride (55 mg, 87% yield) as a white solid. Mass spectrum: calculated for C₂₉H₃₁ClN₄O₅S₃ 647.2; found 648.3 (M⁺ + 1). 1H NMR (400 MHz, CDCl₃) δ ppm 2.33-2.37 (m, 2 H) 2.97 (s, 3 H) 3.05 (s, 3H) 3.30-3.36 (m, 4H) 3.6&.3.71 (m, 2 H) 4.00-4.04 (t, 1 H) 7.1 S-7.17 (m, 2H) 7.26-7.30 (m, 6 H) 7.34-7.38 (m, 4 H) z6 (m, 2 H) 9.95 (s, 1H) 11.25 (s, 1H).

### EXAMPLE 138

### 3-(5-chloro-4-(4-(methylsulfonamido)phenyl)thiazol-2-yl)-1-(3,3-dipbenylpropyl)-1-(3-(methylthio)propyl)urea (7)

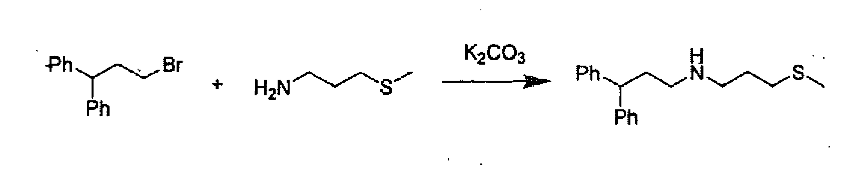

Step 1. The thioether shown above was prepared using Procedure A of Example 132. The reaction conditions yielded N-(3-(methylthio)propyl)-3,3-diphenylpropan-1-amine (210 mg, 77.2% yield) as a colorless oil.

Mass spectrum: calculated for C₁₉H₂₅NS 299.5; found 300.3 (M⁺ + 1).

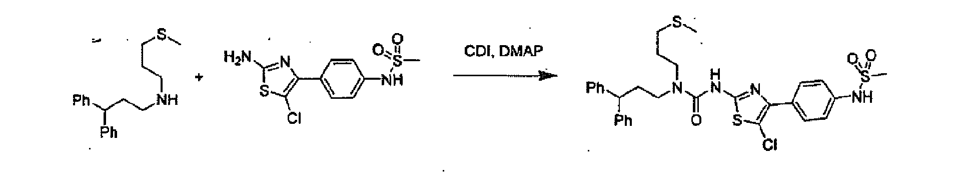

Step 2. The compound shown above was prepared using Procedure B of Example 132. The reaction conditions yielded 3-(5-chloro-4-(4-(methylsulfonamido)phenyl)thiazol-2-yl)-1-(3,3-4iphenylpropyl)-I-(3-(methylthio)propyl)urea hydrochloride (97 mg, 47% yield) as a white solid. Mass spectrum: calculated for C₃₀H₃₃CIN₄O₃S₃ 629.3; found 630.2 (M⁺ + 1).

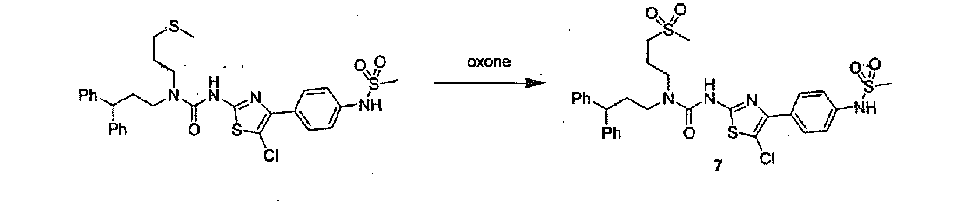

Step 3. The compound **7** shown above was prepared using Procedure C. The reaction conditions yielded 3-(5-chloro-4-(4-(metliylsulfonamido)phenyl)thiazol-2-yl)-1-(3,3-diphenylpropyl)-1-(3-(methylsulfonyl)propyl)urea hydrochloride (35 mg, 36% yield) as a white solid.

Mass spectrum: calculated for C₃₀H₃₃CIN₄O₅S₃ 661.3; found 662.3 (M⁺ + 1). 1H NMR (400 MHz, CDCl₃) δ ppm 1.97-2.00 (m, 2 H) 2.38-2.43 (m, 2H) 2.94 (s, 3 H) 3.00 (s, 3H) 3.08-3.12 (m, 2H) 3.33-3.37 (m, 2 H) 3.41-3.50 (m, 2H) 3.99-4.03 (t, 1 H) 7.14-7.17 (m, 2H) 7.26-7.33 (m, 10 H) 7.89-7.92 (m, 2 H).

### EXAMPLE 139

### 3-(5-chloro-4-(4-(methylsulfionamido)phenyl)thiazol-2-yl)-1-(3,3-diphenylpropyl)-1-(2-(methylsulfnnamido)ethyl)urea (8)

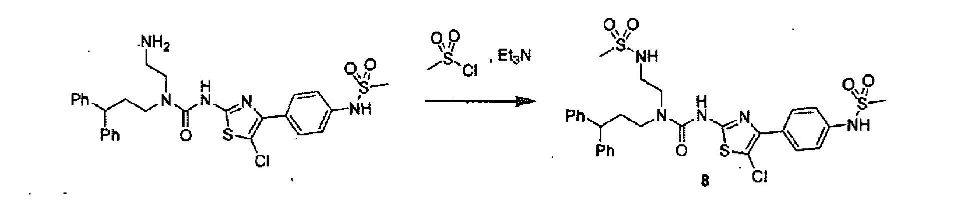

Step 1. A solution of 1-(2-aminoethyl)-3-(5-chloro-4-(4-(methylsulfonamido)-phenyl)thiazol-2-yl)-1-(3,3-diphenylpropyl)urea hydrochloride (35 mg, 56 µmol) and pyridine (11 µl, 141 µmol) in dry CH₂Cl₂ was chilled to 0 °C in an ice bath. Methanesulfonyl chloride (4.6 pl, 59 µmol) was then added to the mixture via syringe. The reaction mixture was warmed slowly to room temperature and stirred for 3 h. The reaction was quenched with 1 N NaOH, and then the solution was diluted with CH₂Cl₂ and water. The water layer was extracted with CH₂Cl₂X2, and the combined organic extracts were washed with brine. The organic phase was dried over MgSO₄, filtered, and concentrated. The crude material was purified by ISCO column chromatography using a 5% to 90% gradient of 10%MeOH-CH₂Cl₂/ CH₂Cl₂ eluent. The desired fractions were combined, concentrated, and converted to the HCl salt to give 3-(5-chloro-4-(4-(methylsulfonamido)phenyl)thiazol-2-yl)-1-(3,3-diphenylpropyl)-1-(2-(methylsulfonamido)-ethyl)urea hydrochloride (15 mg, 40% yield) as a colorless oil. Mass spectrum: calculated for C₂₉H₃₂CIN₅O₅S₃ 662.4; found 663.3 (M⁺ + 1). 1H NMR (400 MHz, MeOH) δ ppm 2.41-2.46 (m, 2 H) 2.90 (s, 3H) 3.02 (s, 3 H) 3.19-3.22 (m, 2H). 3.40-3.47 (m, 4 H) 4.00-4.04 (t, 1H) 7.14-7.18 (m, 2H) 7.26-7.35 (m, 10 H) 7.90-7.92 (d, 2 H).

### EXAMPLE 140

### (R)-3-(5-chloro-4-(4-(methylsulfonamido)phenyl)thiazol-2-yl)-1-(3,3-diphenylpropyl)-1-(2-(piperidin-3-ylamino)ethyl)urea (8)

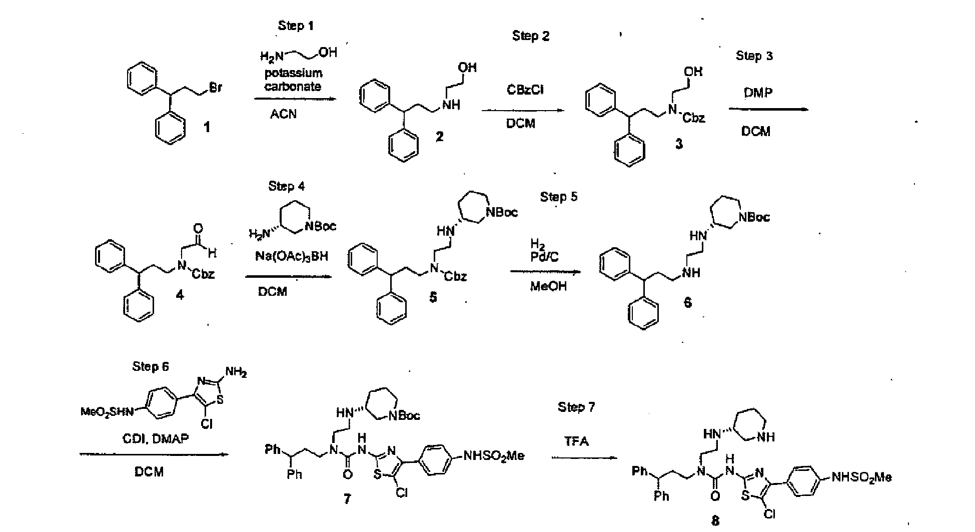

**Step 1:** To a solution of 3-bromo-1,1-diphenylpropane 1 (4.50 g, 16.4 mmol) in 100mL of acetonitrile was added potassium carbonate (0.987 ml, 16.4 mmol), followed by 2-ethanolamine (9.81 ml, 164 mnaol). The reaction mixture was heated to 80 °C while stirring under nitrogen for 18 hours. The reaction was then removed from heat, allowed to cool to room temperature and concentrated *in vacuo.* The residue thus obtained was partitioned between water and ethyl acetate. The organic layer was washed with water and brine, dried over sodium sulfate, filtered and concentrated *in vacuo.* The crude residue was further purified by column chromatography on silica using a gradient eluent: 0% to 15% of methanol in dichloromethane (with 0.1% ammonia) to give **2** as a white amorphous solid (3.4g, 81.4% yield).

**Step 2:** 2-(3,3-diphenylpropylamino)ethanol **2** (3.4 g, 13 mmol) was dissolved in 100 mL of dichloromethane, stirring at 0 °C under an atmosphere of N₂. Benzyl chloroformate (2.3 mL, 16 mmol) was slowly injected into the round bottom flask using a 5 cc syringe. The reaction was allowed to warm up to room temperature and was stirred at room temperature for 40 h. The reaction mixture was partitioned between water and dichloromethane. The organic layer was washed with 1M HCl, dried over sodium sulfate, filtered and concentrated *in vacuo* to yield 3 as a colorless oil (2.16g, 42% yield).

**Step 3:** Benzyl 3,3-diphenylpropyl(2-hydroxyethyl)carbamate **3** (2.157 g, 5.54 mmol) was dissolved in 50 mL of dichloromethane. Dess-MartinPeriodinane (17.3 ml, 8.31 mmol) was added to the reaction in one portion. The reaction was stirred under N₂ at room temperature for 18 h. The reaction was diluted with aqueous sodium thiosulfate solution and extracted. The organic phase was washed with brine, dried over sodium sulfate, filtered and concentrated *in vacuo* to yield a crude product which was further purified by column chromatography on silica using a gradient eluent : 0% to 100% of ethyl acetate in hexane. Fractions containing product were combined and concentrated *in vacuo* to give **4** as a clear oil (1.75g, 82% yield).

**Step 4:** To a solution of benzyl 3,3-diphenylpropyl(2-oxoethyl)carbamate **4** (0.300 g, 0.774 mmol) in 20 ml of dichloromethane was added (R)-tert-butyl 3-aminopiperidine-1-carboxylate (0.140 g, 0.929 mmol) followed by sodium triacetoxyborohydride (0.246 g, 1.16 mmol). The reaction was stirred for 20 h and then diluted with dichloromethane (25 mL), washed with water and brine. The organic phase was dried over sodium sulfate, filtered and concentrated *in vacuo* to yield **5** as a colorless oil (0.34g, 90%). **Step 5:** (R)-tert-butyl 3-(2-(benzyloxycarbonyl)ethylamino)piperidine-1-carboxylate **5** (0.340 g, 0.700 mmol) was dissolved in 20 ml of Methanol and flushed with N₂. Then added palladium, 10wt. % (dry basis) on activated carbon (0.0745 ml, 0.700 mmol). The flask was evacuated and flushed with H₂ 3x and then left under a H₂ balloon and was allowed to stir for 1 hour. The reaction mixture was then filtered through celite and the filtrate was concentrated *in vacuo* to yield 6 as an off-white film (0. 1 94g, 79% yield). **Step 6:** To a solution of l.l'-carbonyldiimidazole (0.141 g, 0.868 mmol) in 10 ml of DCM, was added N-(4-(2-amina-5-chlorothiazol-4-yl)phenyl)methaziesulfanamide (0.176 g, 0:579 mmol) in 5 ml DCM and 2 ml of DMF. The reaction was stirred under N₂ at room temperature for 20 h. (R)-tert-butyl 3-(2-(3,3-diphenylpropylarnirro)ethylamino)piperidine-1-carboxylate 6 (0.304 g, 0.695 mmol) was dissolved in 5 ml DCM and this solution was added to the reaction mixture and the reaction was stirred for 6 hrs. The reaction mixture was diluted with DCM (25 mL) and washed with water 2x followed by brine. The organic phase was dried over magnesium sulfate, filtered and concentrated *in vacuo* to yield 7 as a white solid (0.34g, 77% yield). **Step 7:** To a solution of (R)-tert-butyl 3-(2-(3-(5-chloro-4-(4-(methylsulfonamido)phenyl)-thiazol-2-yl)-1-(3,3-diphenylpropyl)ureido)ethylamino)piperidine-1-carboxylate **7** (0.444 g, 0.579 mmol) in 5 ml of dichloromethane, was added trifluoroacetic acid (0.0430 ml, 0.579 mmol) and the reaction mixture was stirred under N₂ at room temperature for 16 hrs. The reaction mixture was then purified by RP-HPLC to yield the product **8** as a white solid (0.36g, 95% yield).

LC-MS ESI (pos.) m/e: 667.2 (M+H)

¹H MR (400 MHz, *DMSO-d₆*) δ ppm 11.26 (1 H, s), 9.99 (1 H,-s), 9.21 (1 H, s), 8.95 (2 H, s), 7.84 (2 H, d, *J*=8.6 Hz), 7.32 - 7.38 (4 H, m), 7.25 - 7.32 (6 H, m), 7.13 - 7.2 (2 H, m), 4.06 (1 H, t), 3.46 - 3.60 (3 H, m), 3.36 - 3.44 (1 H, m), 3.23 - 3.36 (3 H, m), 3.08 - 3.18 (2 H, m), 3.05 (3 H, s), 2.74 - 2.94 (2 H, m), 2.33 (2 H, q), 2.11 (1 H, d, *J*=10.2 Hz), 1.91 (1 H, d, *J*=14.1 Hz), 1.43 - 1.68 (2 H, m).

### EXAMPLE 141

### (S)-3-(5-chloro-4-(4-(methylsulfonamido)phenyl)thiazol-2-yl)-1-(3,3-diphenylpropyl)-1-(2-(piperidin-3-ylamino)ethyl)urea (9)

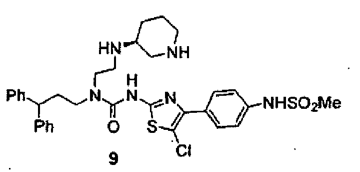

The procedure described in Example 140 was used to prepare **9**, using (S)-tert-butyl 3-aminopiperidine-1-carboxylate instead of (R)-tert-butyl 3-(2-(3,3-diphenylpropylamio)ethylammo)piperidine-1-carboxylate in Step 4. LC-MS ESI (pos.) m/e: 667.2 (M+H)

¹H NMR (400 MHz, *DMSO-d*₆) δ ppm 9.98 (1 H, s), 9.02 (1 H, s), 8.65 - 8.88 (2 H, m), 7.84 (2 H, d, *J*=8.6 Hz), 7.33 - 7.38 (4 H, m), 7.26 - 7.32 (6 H, m), 7.i8 (2 H, t, *J*=7.0 Hz), 4.01 - 4.12 (1 H, m), 3.27 - 3.39 (4 H, m), 3.20 - 3.27 (2 H, m), 3.08 - 3.18 (2 H, m), 3.05 (3 H, s), 2.72 - 2.92 (3 H, m), 2.27 - 2.41 (3 H, m), 2.04 - 2.16 (1 H, m), 1.83 -1.99 (1 H, m), 1.42 - 1.66 (2 H, rxi).

### EXAMPLE 142

### (S)-3-(5-chloro-4-phenylthiazol-2-yl)-1-(3,3-diphenylpropyl)-1-(2-(6-oxopiperidin-3-ylamino)ethyl)urea (10)

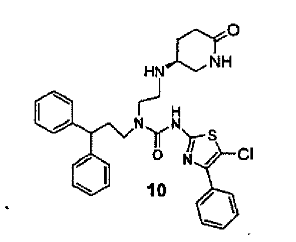

The procedure described in Example 140 was used to prepare **10**, using (S)-5-aminopiperidin-2-ane instead of (R)-tert-butyl 3-(2-(3,3-diphenylpropylami.no)ethylamino)piperidine-1-carboxylate in Step 4 and 5-chloro-4-phenylthiazol-2-amine instead of N-(4-(2-amino-5-chlorothiazol-4-yl)phenyl)methanesulfonamide in Step 6.

LC-MS ESI (pos.) m/e: 589 (M+H);

¹H NMR (400 MHz, MeOH) δ ppm 2.23 - 2.52 (m, 6 H) 2.79 - 2.88 (m, 2 H) 2.89 - 3.00 (m, 1 H) 3.11 - 3.21 (m, 1 H) 3.29 - 3.39 (m, 8 H) 3.45 (dd, *J*=12.13, 3.52 Hz, 1 H) 3.99 (t,*J*=7.83 Hz, 1H) 7.13 - 7.19 (m, 2 H) 7.24 - 7.37 (m, 9 H) 7.39 - 7.47 (m, 2 H) 7.85 - 7.94 (m, 2 H).

### EXAMPLE 143

### 3-(benzo[d]thiazol-2-yl)-1-(3,3-diphenylpropyl)-1-(2-(piperidin-4-ylamino)ethyl)urea (11)

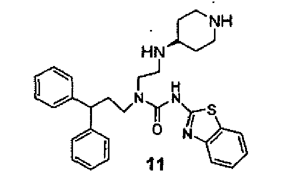

The procedure described in Example 140 was used to prepare **11**, using tert-butyl 4-aminopiperidine-1-carboxylate instead of (R)-tert-butyl 3-(2-(3,3-diphenylpropylamino-)ethylamino)piperidine-1-carboxylate in Step 4 and 2-amino benzothiazole instead ofN-(4-(2-amino-5-chlorothiazol-4-yl)phenyl)methanesulfonamide in Step 6. LC-MS ESI (pos.) m/e: 514 (M+H);

¹H NMR (400 MHz, CHLOROFORM-*d*) δ ppm 1.39 (dd, *J*=9.59, 4.89 Hz, 1 H) I.52 - 1. 73 (m, 3 H) 2.19 - 2.36 (m, 2 H) 2.59 (dd, *J*= 12.91, 7.04 Hz, 1 H) 2.73 - 2.96 (m, 8 H) 3.06 (dd, J=12.91, 2.74 Hz, 1 H) 3.11 - 3.34 (m, 5 H) 3.88 (t, *J=*7.63 Hz, 1 H) 7.00-7.07 (m, 1 H) 7.07 -7.14 (m, 2 H) 7.15 -7.25 (m, 8 H) 7.51 -7.60 (m, 2 H) 7.92 (s, 1 H).

### EXAMPLE 144

### (R)-3-(benzo [d]thiazol- 2-yl)-1-(3,3-diphenylpropyl)-1-(2-(6-oxopiperidin-3-ylamino)ethyl)urea(12)

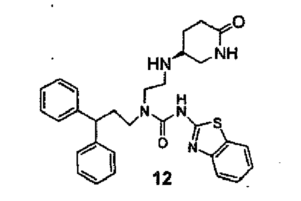

The procedure described in Example 140 was used to prepare **12**, using (R)-5-aminopiperidin-2-one instead of (R)-tert-butyl 3-(2-(3,3-diphenylpropylamino)ethylamino)-piperidine-1-carboxylate in Step 4 and 2-amino benzothiazole instead of N-(4-(2-amino-5-chlorothiazol-4-yl)phenyl)methanesulfonamide in Step 6.

LC-MS ESI (pos.) m/e: 528 (M+H);

¹H NMR (400 MHz, CHLOROFORM-*d*) δ ppm 1.77 - 1.89 (m, 2 H) 2.21 - 2.48 (m, 5 H) 2.60 (dd, *J*=12.13, 7.83 Hz, 1 H) 2.90 - 2.97 (m, 1 H) 2.97 - 3.06 (m, 1 H) 3.15 - 3.39 (m, 4 H) 3.48 - 3.60 (m, 2 H) 3.91 (t, *J*=7.83 Hz, 1H) 7.02 - 7.08 (m, 2 H) 7.08 - 7.15 (m, 2 H) 7.17 - 7.26 (m, 8 H) 7.39 - 7.47 (m, 1 H) 7.60 - 7.68 (m, 1 H).

### EXAMPLE 145

### (R)-3-(benzofdlthiazol-2-yl)-1-(3,3-diphenylpropyl)-1-(2-(piperidin-3-ylamino)ethyl)urea (13)

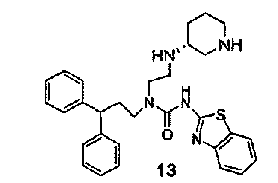

The procedure described in Example 140 was used to prepare **13**, using 2-amino benzothiazole instead of N-(4-(2-amino-5-chlorothiazol-4-yl)phenyl)methanesulfonamide in Step 6.

LC-MS ESI (pos.) m/e: 514 (M+H);

¹H NMR (400 MHz, METHANOL-*d*₄) δ ppm 1.61-1.74 (m, 1 H) 1.74 - 1.90 (m, 1 H) 2.03 - 2.14 (m, 1 H) 2.28 (d, *J=14.09* Hz, 1 H) 2.44 (q, *J=*7.69 Hz, 2 H) 2.96 (td, *J*=12.72, 3.13 Hz, 1 M 3.08 (t, *J=*11.74 Hz, 1 H) 3.28 (t, *J*=5.67 Hz, 2 H) 3.40 (d, *J*=12.52-Hz, 1 H) 3.46 - 3.80 (m, 6 H) 4.01 (t, *J*=7.63 Hz, 1H) 7.11 - 7.19 (m, 2 H) 7.19 - 7.34 (m, 9H) 7.34 - 7.41 (m, 2 H) 7.64 (d, *J=*7.82 Hz, 1H).

### EXAMPLE 146

### (R)-3-(benzo[d]thiazol-2-yl)-1-(3,3-diphenylpropyl)-1-(2-(6-oxopiperidin-3-ylamino)ethyl)urea (14)

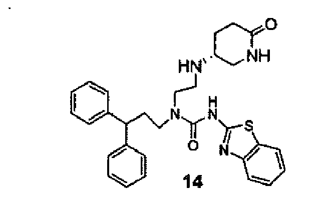

The procedure described in Example 140 was used to prepare **14**, using (R)-5-aminopiperidin-2-one instead of (R)-tert-butyl 3-(2-(3,3-diphenylpropylamino)ethylamino)-piperidine-1-carboxylate in Step 4 and 2-amino benzothiazole instead of N-(4-(2-amino-5-chlorothiazol-4-yl)phenyl)methanesulfonamide in Step 6.

LC-MS ESI (pos.) m/e: 528 (M+H);

¹H NMR (500 MHz, MeOH) δ ppm 1.96 - 2.08 (m, 1 H) 2.22 - 2.32 (m, 1 H) 2.39 - 2.54 (m, 4 H) 3.28 (q, *J* 5.90 Hz, 2 H) 3.36 - 3.43 (m, 1 H) 3.54 (s, 2 H) 3.61 - 3.77 (m, 4 H) 4.03 (t, *J*=7.63 Hz, 1 H) 7.18 (t, *J=*7.02 Hz, 2 H) 7.21 - 7.26 (m, 1 H) 7.27 - 7.36 (m, 8 H) 7.36 - 7.43 (m, 2 H) 7.66 (d, *J*= 7.93 Hz, 1 H).

### EXAMPLE 147

### (R)-3-(5-chloro-4-phenylthiazol-2-yl)-1-(3,3-diphenylpropyl)-1-(2-(6-oxopiperidin-3-ylamino)ethyl)urea (15)

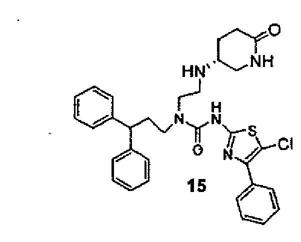

The procedure described in Example 140 was used to prepare **15**, using (R)-5-aminopiperidin-2-one instead of (R)-tert-butyl 3-(2-(3,3-diphenylpropylamino)ethylamino)-piperidine-1-carboxylate in Step 4 and 5-chloro-4-phenylthiazol-2-amine instead of N-(4-(2-amino-5-chlorothiazol-4-yl)phenyl)methanesulfonamide in Step 6.

LC-MS ESI (pos.) m/e: 589 (M+H);

¹H NMR (400 MHz, MeOH) δ ppm 2.24 - 2.50 (m, 4 H) 2.82 - 2.89 (m, 2 H) 2.92 - 3.00 (m, 1 H) 3.18 (dd, *J*=11.35, 9.00 Hz, 1 H) 3.32 - 3.41 (m, 4 H) 3.46 (dd, *J*=11.74, 3.91 Hz, 1 H) 4.00 (t, *J*=7.63 Hz, 1 H) 7.12 - 7.20 (m, 2 H) 7.23 - 7.38 (m, 9 H) 7.38 - 7.46 (m, 2 H) 7.85 - 7.91 (m, 2 H).

### Methods used in Example 148 et seq.

### Method A:

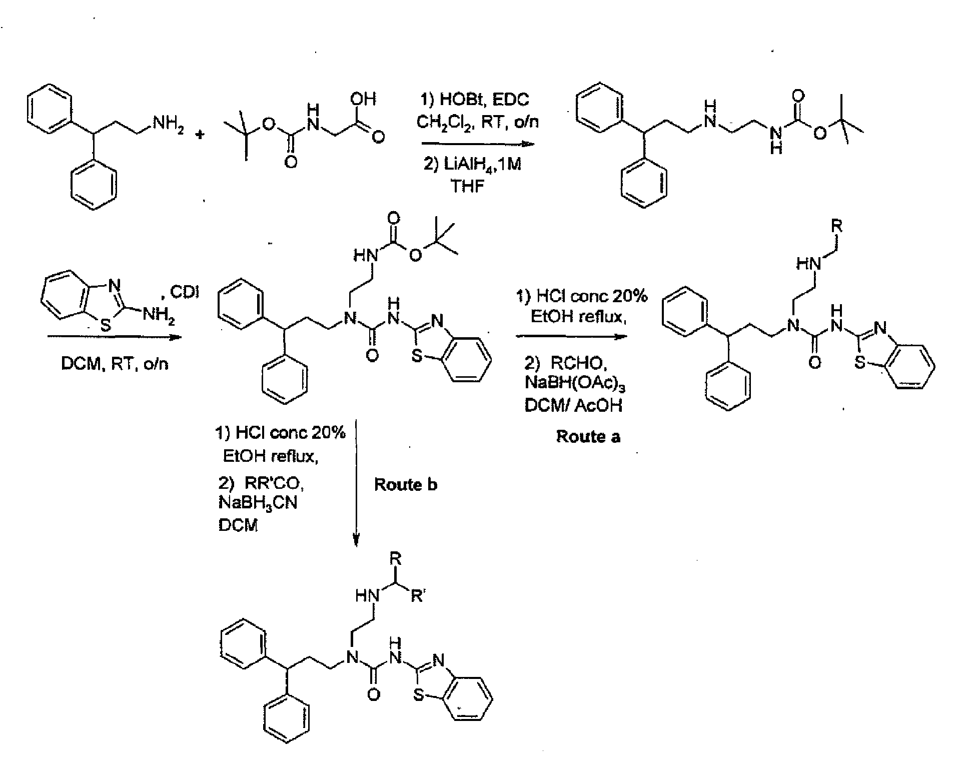

### Synthesis of [2-(3,3-Diphenyl-propylamino)-ethyl]-carbamic acid tert-butyl ester:

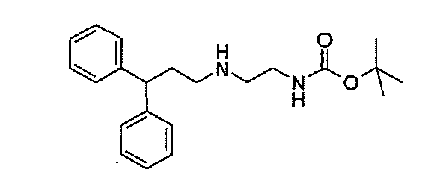

15 g of 3,3-diphenyl-propylamine (71.1 mmol, 1 eq) and 13.7 g of *N*-Boc-Glycine (78.2 mmol, 1.1 eq) were diluted in 100 mL of dry DCM. 10.b g of HOBt (78.2 mmol, 1.1 eq) and 15 g of EDC.HCI (78.2 mmol, 1.1 eq) were added to the solution. The reaction mixture was stirred for 16 h at room temperature, under argon, before addition of water. The aqueous phase was extracted several times with DCM, the organics were washed with brine, dried over MgS0₄, filtered then concentrated. The crude product was purified by flash chromatography over silica gel (DCM/ MeOH: 98/ 2 to 96/ 4) to give 23.6 g of the desired amide intermediate [(3,3-diphenyl-propylearbamoyl)-methyl]-carbamic acid tert-butyl ester (90% yield).

12 g of this amide intermediate (32.5 mmol, 1 eq) were solubilized in 100 mL of dry THF, under argon. After cooling the solution to 0°C, 71.6 mL of LiAlH₄ (1 M in THF, 71.6 mmol, 2.2 e^{q}) were added dropwise to the solution. The reaction mixture was stirred for 16 h at room temperature, then cooled to O'C before slow addition of water. After stirring for 1 h at room temperature, the mixture was filtered to get rid of aluminium salts and the solid was washed several times with EtOAc. The aqueous phase was extracted several times with EtOAc, then the organic phase was washed with brine, dried over MgS0₄, filtered and concentrated. The crude product was purified with flash chromatography over silica gel (DCM/ MeOH: 98/2 to 90/ 10) to give 8.6 g of the desired secondary amine [2-(3,3-diphenyl-propylamino)-ethyl]-carbamic acid tert-butyl ester (70% yield).

**¹H NMR** (400 MHz, DMSO): δ 7.28 (m, 8H, aromatic H), 7.15 (m, 2H, aromatic H), 6.68 (t, 1H, NH), 4.02 (t, 1H, CH), 2.93 (q, 2H, CH₂), 2.45 (t, 2H, CH₂), 2.38 (t, 2H, CH₂), 2.11 (q, 2H, CH₂), 1.36 (s, 9H, 3xCH₃).

**MS (ES⁺)**: 355.2⁺ (M+H)⁺

### EXAMPLE 1.48

### {2-[3-Benzothiazol-2-yl-1-(3,3-diphenyl-propyl)-ureido]-ethyl}-carbamic acid tert-butyl ester

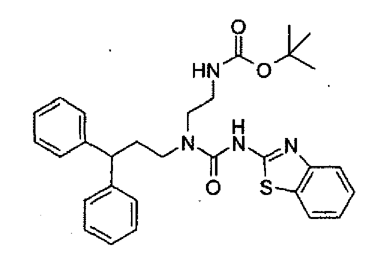

648.6 mg (4 mmol, 1.5 eq) of carbonyl diimidazole were dissolved in 6 mL of CH₂Cl₂ then 2.66 g of2-amino-benzothiazole (2.66 mmol, 1eq) were added to the solution. A white precipitate appeared. The suspension was stirred for 15 hours at room temperature. 1.13 g of [2-(3,3-diphenyl-propylamino)-ethyl]-carbamic acid tert-butyl ester (3.19 mmol, 1.2 eq) in CH₂Cl₂ were then added. The solution, which has become clear again, was stirred for 5 hours at room temperature. A sodium bicarbonate solution was added and the aqueous phase was extracted with dichloromethane. The organic phase was washed with brine, dried over MgSO₄, filtered and concentrated. The crude product was subjected to chromatography over silica gel (Hept/ EtOAc: 80! 20 to 70/ 30) to obtain 929 mg of the desired urea (65% yield).

**¹H NMR** (400 MHz, DMSO): δ 7.88 (m, 1H, aromatic H), 7.62 (m, 1H, aromatic H), 7.35 5 (m, 4H, aromatic H), 7.28 (m, 4H, aromatic H), 7.22 (m, 1H, aromatic H), 7.18 (m, 3H, aromatic H), 6.81 (t, 1H, NH), 4.01 (m, 1H, CH), 3.35 (m, 2H, CH₂), 3.28 (m, 2H, CH₂), 3.02 (m, 2H, CH₂), 2.32 (m, 2H, CH₂), 1.30 (s, 9H, 3xCH₃). **MS (ES⁺)**: 355.2⁺, 531.3⁺ (M+H)⁺

### EXAMPLE 1.49

### 4-({2-[3-Benzothiazol-2-yl-1-(3,3-diphenyl-propyl)-ureido]-ethylamino}-methyl)-piperidine-1-carboxylic acid benzyl ester

Method A, route a, was used to prepare the above product ol' formula:

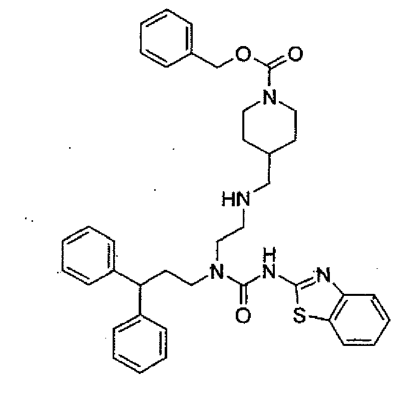

59S mg of the product of example 148 (1.12 mmol, 1eq) were diluted in 12 mL of EtOH, then 3 mL of HCl conc. (20% vol) were added to the solution. The reaction mixture was heated to reflux and stirred for 1 h 30. The mixture was evaporated to dryness then the insoluble matter was triturated with diethyl ether, filtered and washed again several times with Et₂O. A 1 N sodium hydroxide solution was added to the suspension in solution in DCM. The aqueous phase was extracted 3 times with DCM then the organic phases were washed with IN NaOH solution, water, brine, then dried over MgSO₄, filtered and concentrated to give 375 mg of the desired intermediate 1-(2-aminoethyl)-3-benzathia'zol-2-yl-1-(3,3-diphenyl-prapyl)-urea (78% yield for 2 steps). There was no need to purify this primary amine intermediate for the next steps.

140 mg of 1-(2-amino-ethyl)-3-benzothiazol-2-yl-1-(3,3-diphenyl-propyl)-urea (U.232 mmol, 1 eq) and 63 mg of 4-formyl-piperidine-1-carboxylic acid benzyl ester (0.255 mmol, 1.1 eq) were diluted in 3 mL of dry DCM and a catalytic amount of AcOH, under argon. 73.4 mg of NaBH(OAc)₃ (0.348 mmol, 1.5 eq) were then added to the solution. The reaction mixture was stirred for 16 h at RT before addition of a 1 N NaOH solution. The aqueous phase was extracted with EtOAc, and then the organic phases were washed with water, brine, dried over MgSO₄, filtered and concentrated. The crude product was purified by flash chromatography over Si0₂ (DCM/ MeOH: 99/1 to 98/2). 84 mg of the desired compound were obtained (64% yield).

**¹H NMR** (400 MHz, CD₃COCD₃): δ 7.82 (m, 1H, aromatic H), 7.60 (m, 1H, aromatic H), 7.38 (m, 8H, aromatic H), 7.30 (m, 6H, aromatic H), 7.18 (m, 3H, aromatic H), 5.10 (s, 2H, CH₂), 4.18 (m, 2H, CH₂), 4.07 (t, 1H, CH), 3.50 (m, 2H, CH₂), 3.38 (m, 2H, CH₂), 2.96 (m, 2H, CH₂), 2.80 (m, 2H, CH₂), 2.61 (m, 2H, CH₂), 2.43 (m, 2H, CH₂), 2.05 (m, 1H, CH), 1.92 (m, 2H, CH₂), 1.15 (m, 2H, CH₂).

**MS (ES⁺):** 486.2⁺, 662.2⁺ (M+H)⁺

### EXAMPLE 150

### 3-Denzothiazol-2-yl-1-12-(3-dimethylamino-2,2-dimethyl-propylamino)-ethyll-l-(3,3-diphenyl-propyl)-urea trihydrochloride

Method A, route a, followed by HCl salification was used to prepare the above product of formula:

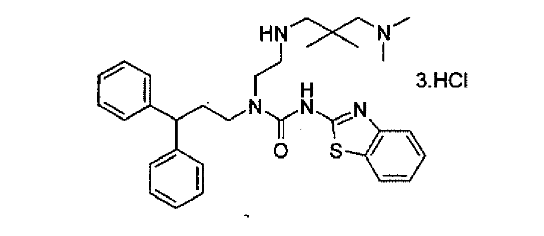

87.7 mg (0.161 mmol, 1 eq) of urea, 3-benzothiazol-2-yl-1-[2-(3-dimethylamino-2,2-dimethyl-propylamino)-ethyl]-1-(3,3-diphenyl-propyl), in a basic state were dissolved in a minimum amount of DCM (until complete dissolution). 322 µL (0.645 mmol, 4eq)of2N HCI in diethyl ether were added. The mixture was stirred for 20 sec then concentrated to dryness. The residue was taken up in the minimum of dichloromethane, and then diethyl ether was added to precipitate the product. The insoluble matter was filtered then washed with diethyl ether (m = 100 mg, 95% yield).

**¹H NMR** (400 MHz, DMSO): δ 8.90 (brs, 1H, NH), 7.80 (d, 1H, aromatic H), 7.49 (m, 1 H, aromatic H), 7.3 8 (zn, 5H, aromatic H), 7.30 (m, 4H, aromatic H), 7.18 (m, 3H, aromatic H), 4.06 (t, 1H, CH), 3.72 (m, 2H, CH₂), 3.38 (m, 2H, CH₂), 3.30 (m, 2H, CH₂), 3.12 (m, 4H, 2xCH₂), 2.82 (s, 6H, N(CH₃)₂), 2.38 (m, 2H, CH₂), 1.20 (s, 6H, 2xCH₃). **MS (ES)**: 368.2⁺, 544.2⁺ (M+H-3HCl)⁺

### EXAMPLE 151

### {2-[3-Benzothiazol-2-yl-1-(3,3-diphenyl-propyl)-ureido]-ethylamino}-acetic acid ethyl ester

Method A, route a, was used to prepare the above product of formula:

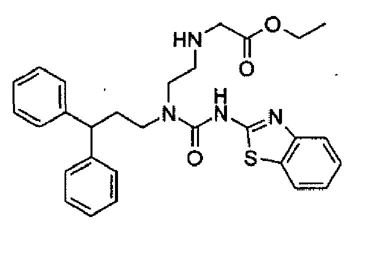

**¹H NMR** (400 MHz, DMSO): δ 7.82 (d, 1H, aromatic H), 7.51 (m, 1H, aromatic H), 7.35 (m, 4H, aromatic H), 7.30 (m, 5H, aromatic H), 7.18 (m, 3H, aromatic H), 4.09 (q, 2H, CH₂), 4.00 (t, 1H, CH), 3.30 (m, 6H, 3xCH₂), 2.70 (m, 2H, CH₂), 2.32 (m, 2H, CH₂), 1.18 (t, 3H, CH₃).

**MS (ES⁺)**: 341.1⁺, 517.0⁺ (M+H)⁺

### EXAMPLE 152

### 2-((2-[3-Benzothiazol-2-yl-1-(3,3-diphenyl-propyl)-ureido]-ethylamino)-methyl)-cyclopropanecarboxylic acid ethyl ester

Method A, route a, was used to prepare the above product of formula:

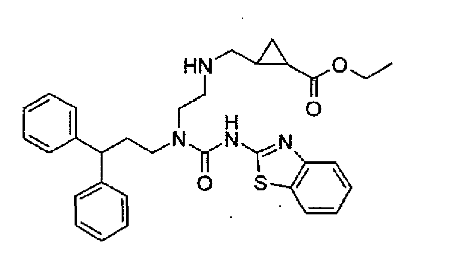

**MS (ES⁺):** 381.1⁺, 547.0⁺(M+H)⁺

### EXAMPLE 153

### 3-Benzothiazol-2-yl-1-12-(2,2-diniethoxy-ethylamino)-ethyll-l-(3,3-diphenyl-propyl)-urea

Method A, route a, was used to prepare the above product of formula:

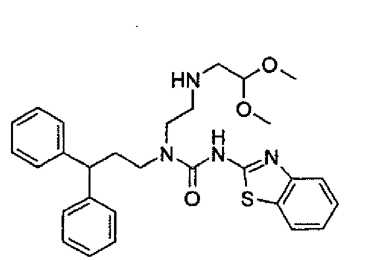

**¹H NMR** (400 MHz, DMSO): δ 7.82 (d, 1H, aromatic H), 7.52 (m, 1H, aromatic H), 7.35 (m, 4H, aromatic H), 7.30 (m, 5H, aromatic H), 7.18 (m, 3H, aromatic H), 4.70 (m, 1H, CH), 3.99 (t, 1H, CH), 3.38 (m, 2H, CH₂), 3.30 (s, 6H, 2xOCH₃), 3.25 (m, 2H, CH₂), 2.72 (m, 2H, CH₂), 2.65 (m, 2H, CH₂), 2.30 (m, 2H, CH₂). **MS (ES⁺)**: 519.0⁺ (M+H)⁺

### EXAMPLE 154

### 3-Benzothiazol-2-yl-1-(3,3-diphenyl-propyl)-1-{2-[(6-methoxy-pyridin-3-ylmethyl)-amino]-ethyl}-urea

Method A, route a, was used to prepare the above product of formula:

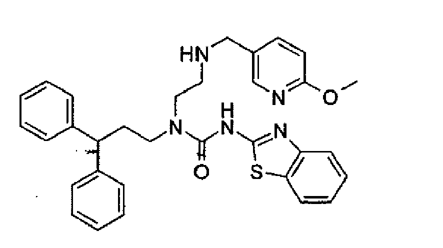

**¹H NMR** (400 MHz, DMSO): δ 8.10 (s, 1 H, aromatic HY, 82 (d, 1H, aromatic H), 7.78 (m, 1H, aromatic H), 7.51 (d, 1H, aromatic H), 7.35 (m, 5H, aromatic H), 7.30 (m, 4H, aromatic H), 7.18 (m, 3H, aromatic H), 6.72 (d, 1 H, aromatic H), 3.99 (t, 1 H, CH), 3.80 (s, 3H, OCH₃), 3.75 (m, 2H, CH₂), 3.39 (m, 2H, CH₂), 3.25 (m, 2H, CH₂), 2.68 (m, 2H, CH₂), 2.30 (m, 2H, CH₂).

**MS (ES⁺)**: 376.1⁺, 552.06⁺ (M+H)⁺

### EXAMPLE 155

### 3-Benzothiazol-2-yl-1-(3,3-diphenyl-propyl)-1-[2-(tetrahydro-thiopyran-4-ylamino)-ethyl]-urea

Method A, route b, was used to prepare the above product of formula:

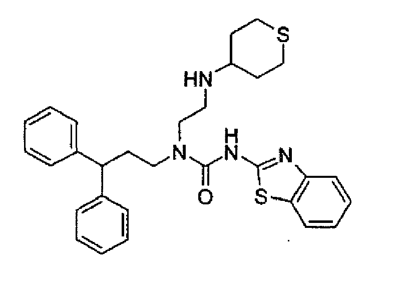

595 mg of the product of Example 148 (1.12 mmol, 1eg) were diluted in 12 mL of EtOH, then 3, mL of HCl conc. (20% vol) were added to the solution. The reaction mixture was heated to reflux and stirred for 1 h 30. The mixture was evaporated to dryness then the insoluble matter was triturated with diethyl ether, filtered and washed again several times with Et₂O. A 1 N sodium hydroxide solution was added to the suspension in solution in DCM. The aqueous phase was extracted 3 times with DCM then the organic phases were washed with 1 N NaOH solution, with water, brine, then dried over MgSO₄, filtered and concentrated to give 375 mg of the desired intermediate 1-(2-amino-ethyl)-3-benzotlfazol-2-yl- 1 -(3 ,3-diphenylpropyl)-urea (78% yield for 2 steps). There was no need to purify this primary amine intermediate for the next steps.

100 mg of 1-(2-amino-ethyl)-3-benzothiazol-2-yl-1-(3,3-diphenyl-propyl)-urea (0.232 mmol, 1.5 e^{q}) and 18 mg of tetrahydro-thiopyran-4-one (0.155 mmol, 1 eq) were diluted in 2 mL of dry DCM, under argon. 19.5 mg of NaBH₃CN (0.310 mmol, 2 eq) were then added to the solution. The reaction mixture was stirred for i 6 h at room temperature before addition of a 1 N NaOH solution. The aqueous phase was extracted with EtOAc, then the organic phases were washed with water, brine, dried over MgS0₄, filtered and concentrated. The crude product was subjected to flash chromatography over Si0₂ (DCM/ MeOH: 99/ 1 to 95/ 5). 40 mg of the desired urea were obtained (49% yield).

**¹H NMR** (400 MHz, DMSO): δ7.82 (d, 1H, aromatic H), 7.52 (d, 1H, aromatic H), 7.35 (m, 5H, aromatic H), 7.30 (m, 4H, aromatic H), 7.18 (m, 3H, aromatic H), 5.98 (m, 1H, NH), 3.99 (t, 1H, CH), 3.48 (m, 1H, CH), 3.30 (m, 5H, CH+2xCH₂), 2.75 (m, 1H, CH), 2.65 (m, 2H, CH₂), 2.52 (m, 2H, CH₂), 2.31 (m, 2H, CH₂), 2.18 (m, 2H, CH₂), 1.50 (m, 2H, CH₂).

**MS (ES⁺):** 355.1⁺, 531.1⁺ (M+H)⁺

### EXAMPLE 156

### 3-Benzothiazol-2-yl-1-(3,3-diphenyl-propyl)-1-[2-(tetrahydro-pyran-4-ylamino)-ethyl]-urea

Method A, route b, was used to prepare the above product of formula:

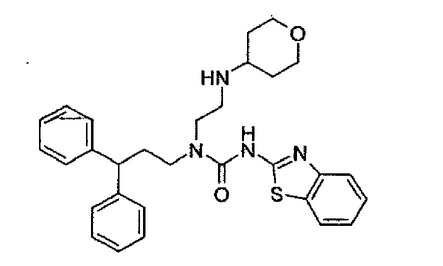

**¹H NMR** (400 MHz, DMSO): δ 7.82 (d, 1H, aromatic H), 7.52 (d, 1H, aromatic H), 7.35 (m, 5H, aromatic H), 7.30 (m, 4H, aromatic H), 7.18 (m, 3H, aromatic H), 3.99 (t, 1H, CH), 3.82 (m, 2H, CH₂),3.38 (m, 3H, CH+CH₂),3.28 (m, 3H, CH+CH₂), 2.80 (m, 2H, CH₂), 2.70 (m, 1H, CH), 2.31 (m, 2H, CH₂),1.82 (m, 2H, CH₂),1.38 (m, 2H, CH₂). **MS (ES⁺):** 339.1⁺, 515.1⁺ (M+H)⁺

### EXAMPLE 157

### 3- Benzothiazol-2-yl-1-(3,3-diphenyl-propyl)-1-[2-(2-methyl-tetrahydro-furan-3-ylamino)-ethyl]-urea

Method A, route b, was used to prepare the above product of formula:

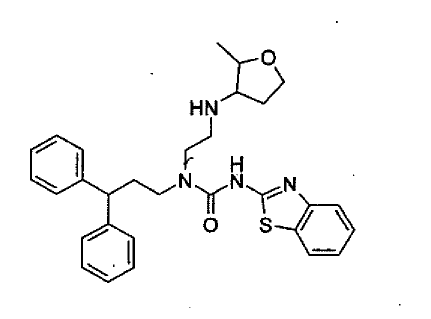

**¹H NMR** (400 MHz, DMSO): δ 7.82 (d, 1H, aromatic H), 7.52 (d, 1H, aromatic H), 7.35 (m, 5H, aromatic H), 7.30 (m, 4H, aromatic H), 7.18 (m> 3H, aromatic H), 5.98 (m, 1H, NH), 3.99 (t, 1H, CH), 3.72 (m, 2H, CH₂), 3.32 (m, 5H, CH+2xCH₂), 2.80-2.65 (m, 3H, CHC-CH₂), 2.32 (m, 2H, CH₂), 2.02 (m, 1H, CH), 1.71 (m, 1H, CH), 1.10 (m, 3H, CH₃). **MS (ES⁺):** 339.1⁺, 515.1⁺ (M+H)⁺

### EXAMPLE 158

### (4-{2-[3-Benzothiazol-2-yl-1-(3,3-diphenyl-propyl)-ureido]-ethylamino)-piperidin-1-yl)-acetic acid tert-butyl ester

Method A, route b, was used to prepare the above product of formula:

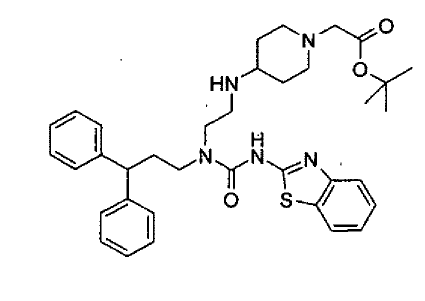

**¹H NMR** (400 MHz, DMSO): δ 7.82 (d, 1H, aromatic H), 7.52 (d, 1H, aromatic H), 7.35 (m, 4H, aromatic H), 7.30 (m, 5H, aromatic H), 7.18 (m, 3H, aromatic H), 3.99 (t, 1H, CH), 3.35 (m, 2H, CH₂), 3.25 (m, 2H, CH₂), 3.02 (s, 2H, CH₂), 2.77.(m, 4H, 2xCH₂), 2.70 (m, 1H, CH), 2.40 (m, 1H, CH), 2.32 (m, 2H, CH₂), 2.15 (m, 2H, CH₂), 1.85 (m, 2H, CH₂), 1.68 (m, 1H, C14),1.38 (s, 9H, 3xCH₃).

**MS (ES⁺):** 452.1⁺, 628.1⁺ (M+H)⁺

### EXAMPLE 159

### 4-{2-[3-Benzothiazol-2-yl-1-(3y3-diphenyl-propyl)-ureido]-ethylamino}-piperidine-1-carboxylic acid tert-butyl ester

Method A, route b, was used to prepare the above product of formula:

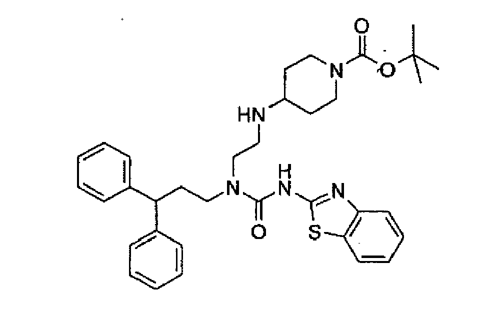

**¹H NMR** (400 MHz, DMSO): δ 7.82 (d, 1H, aromatic H), 7.52 (d, 1H, aromatic H), 7.35 (m, 4H, aromatic H), 7.30 (m, 5H, aromatic H), 7.18 (m, 3H, aromatic H), 3.99 (t, 1H, CH), 3.84 (m, 2H, CH₂), 3.36 (m, 4H, 2xCH₂), 3.25 (m, 2H, CH₂), 2.77 (m, 2H, CH₂), 2.60 (m, 1H, CH), 2.32 (m, 2H, CH₂), 2.15 (m, 2H, CH₂), 1.83 (m, 2H, CH₂), 1.35 (s, 9H, kCH₃).

**MS (ES⁺):** 614.2⁺ (M+H)⁺

### EXAMPLE 160

### 3-Benzothiazol-2-yl-1-(3,3-diphenyl-propyl)-1-[2-(piperidin-4-ylamino)-ethyl]-urea

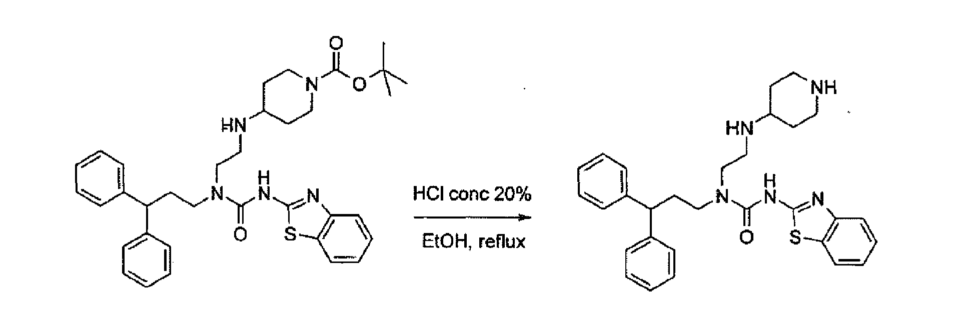

### Method B:

100 mg of the product of Example 159 (0.163 mmol, 1eq) were diluted in 4 mL of EtOH, then 1 mL of HCl conc. (20% vol) was added to the solution. The reaction mixture was heated to reflux and stirred for 1 h. The mixture was evaporated to dryness then the insoluble matter was diluted in a 1 N sodium hydroxide solution and extracted with EtOAc. The organic phases were washed with 1 N NaOH solution, with water, brine, dried over MS04, filtered and concentrated. Purification by flash chromatography over silica gel (DCM/ MeOH/ NH₄0H: 90/10/ 0.1 to 90/10/0.4) allowed to give 54 mg of the desired free piperidine (65% yield).

**¹H NMR** (400 MHz, DMSO): δ 7.80 (d, 1H, aromatic H), 7.50 (d, 1H, aromatic H), 7.35 (m, 4H, aromatic H), 7.30 (m, 5H, aromatic H), 7.18 (m, 3H, aromatic H), 3.99 (t, 1H, CH), 3.35 (m, 2H, CH₂), 3.25 (m, 2H, CH₂), 2.93 (m, 2H, CH₂), 2.78 (m, 2H, CH₂), 2.43 (m, 3H, CH+CH₂), 2.30 (m, 2H, CH₂), 1.85 (m, 2H, CH₂), 1.22 (m, 2H, CH₂). **MS (ES⁺):** 33 8.1⁺, 514.1⁺ (M+H)⁺

### EXAMPLE 161

### (2-[3-Benzothiazol-2-yl-1-(33-diphenyl-propyl)-ureido]-ethylamino)-acetic acid

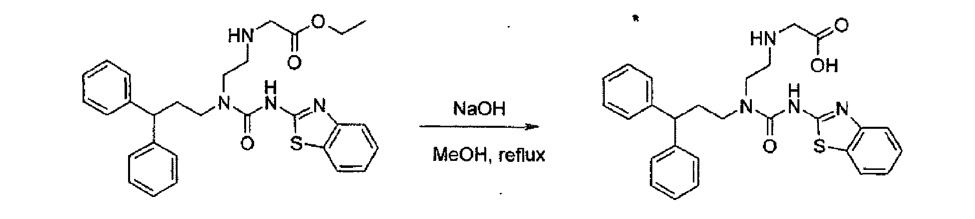

### Method C: Saponification

54 mg of the product of Example 151 1 (0.104 mmol, 1 eq) were diluted in 3 mL of MeOH, then 209 µL of 1 N NaOH solution (0.209 mmol, 2 eq) were added to the solution. The reaction mixture was heated to reflux and stirred for 16 h. The mixture was evaporated then 2 N HCl solution was added until the pH was 5-6, then extracted with EtOAc and with DCM. The organic phases were washed with with water, brine, dried over MgSO₄, filtered and concentrated. Purification by flash chromatography over silica gel (DCMI MeOH) NH₄OH: 90/10/ 0.2 to.90/ 10/ 0.4) followed by trituration with diethyl ether allowed to obtain 17 mg of the desired amino-acid (51 % yield). **¹H NMR** (400 MHz, DMSO): δ 7.80 (d, 1H, aromatic H), 7.49 (d, 1H, aromatic H), 7.35 (m, 4H, aromatic H), 7.30 (m, 5H, aromatic H), 7.1.8 (m, 3H, aromatic H), 3.99 (t, 1H, CH), 3.52 (m, 2H, CH₂), 3.25 (m, 4H, 2xCH₂), 2.95 (m, 2H, CH₂), 2.32 (m, 2H, CH₂). **MS (ES⁺):** 313.0⁺, 489.0⁺ (M+H)⁺

### EXAMPLE 162

### (4-(2-[3-Benzothiazol-2-yl-1-(3,3-diphenyl-propyl)-ureido]-ethylamino)-piperidin-1-yl)- acetic acid

Method C of Example 161 was used to prepare the above product of formula:

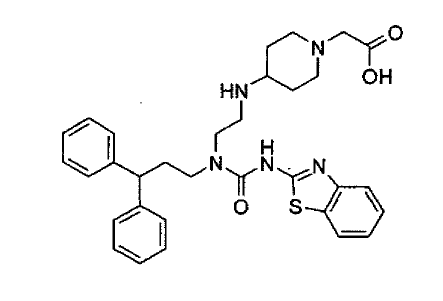

**¹H NMR** (400 MHz, DMSO): δ 7.82 (d, 1H, aromatic H), 7.49 (d, 1H, aromatic H), 7.35 (m, 5H, aromatic H), 7.30 (m, 4H, aromatic H), 7.18 (m, 3H, aromatic H), 3.99 (t, 1H, CH), 3.39 (m, 2H, CH₂),3.26 (m, 2H, CH₂ 3.20 (s, 2H, CH₂), 3.12 (m, 2H, CH₂), 2.78 (m, 2H, CH₂), 2.62 (m, 3H, CH+CH₂), 2.32 (m, 2H, CH₂), 1.93 (m, 2H, CH₂), 1.55 (m, 2H, CH₂).

**MS (ES⁺):** 396.2⁺, 5 72. 1 + (M+H)"

### Method D:

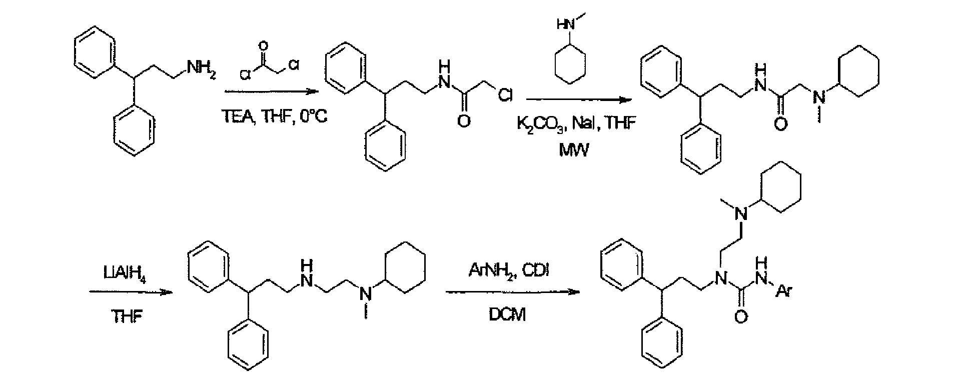

### Synthesis of 2-Chloro-N-(3,3-digheayl--propyl)-acetamide:

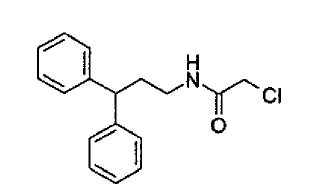

25 g (0.118 mol, 1 eq) of 3,3-diphenyl-propylamine and 20.7 mL (0.147 mol, 1.25 e^{q}) of triethylamine were diluted in 250 mL of THF at 0 °C, under argon. 9.85 mL (0.124 mol, 1.05 eq) of chloroacetyl chloride were added dropwise. White fumes formed then gradually dissipated. The mixture was stirred for 2 h at 0 °C, the solution became red and a white precipitate formed. The precipitate was filtered then washed several times with EtOAc. The organic phase was extracted with dilute HCl solution, with water then with brine, dried over MgS0₄, filtered and concentrated. The product was obtained as a yellow solid (m = 32 g, yield = 91 %).

**¹H NMR** (400 MHz, CDCl₃): δ 2.24 (q, 2H, CH₂), 3.21 (q, 2H, CH₂), 3.82-3.92 (m, 3H, CH+CH₂), 6.37-6.50 (m, 1H, NH), 7.06-7.25 (m, 10H, aromatic H).

**MS (ES⁺)**: 288.09⁺ (M+H)⁺, 329.11⁺ (M+H+CH₃CN)⁺

### Synthesis of N-cyclohexyl-N'-(3,3-diphenyl-propyl)-N-methyl-ethane-1,2-diamine:

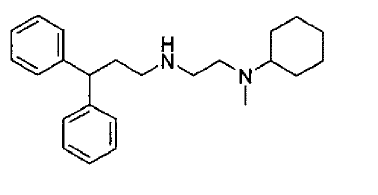

To a solution of 2-chloro-*N*-(3,3-diphenyl-propyl)-acetamide (1 eq) in THF in a microwave sealed tube was added 2 eq of cyclohexyl-methyl-amine, 1 eq of Nal and 2 eq of K₂CO₃. The mixture was irradiated with microwaves for 20 minutes at 110°C. The mixture was then filtered, washed with EtOAc, concentrated and purified by flash chromatography over silica gel to obtain the corresponding 2-(cyclohexyl-methyl-amino)-N-(3,3-diphenyl-propyl)-aceLunide.

The crude product was dissolved in 10 mL of THF then at 0°C, 2.5 eq of LiAlH₄ 1M in THF were added dropwise. The reaction mixture was stirred at room temperature for 20 h under argon. The mixture was then hydrolysed slowly at 0°C with a small amount of water, then Na₂SO₄ was added to dry the solution. After filtration, washing with EtOAc, then evaporation of the organic phase, the obtained crude product was used directly for the next step without any purification.

### EXAMPLE 163

### 3-Benzothiazol-2-yl-1-[2-(cyclohexyl-methyl-amino)-ethyl]-1-(3,3-diphenyl-propyl)-urea

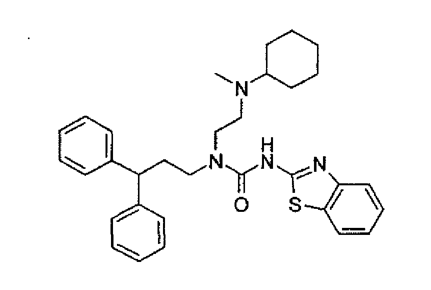

1.5 eq of carbonyl diimidazole were dissolved in a minimum amount (c = 0.5-1 M) of CH₂C1₂ then 1 eq of 2-aminobenzothiazole were added to the solution. A white precipitate appeared. The suspension was stirred for 15 hours at room temperature. 1.2 eq of N-cyclohexyl-N-(3,3 -diphenyl-propyl)-N-methyl-ethane- 1,2-diamine in CH₂Cl₂ were then added. The solution, which has become clear again, was stirred for 3 hours at room temperature. A sodium bicarbonate solution was added and the aqueous phase was extracted with dichloromethane. The organic phase was washed with brine, dried over MgS0₄, filtered and concentrated. The crude product was subjected to chromatography over silica gel (EtOAc/ MeOH: 95 /5) to obtain the desired urea.

**¹H NMR** (400 MHz, DMSO): δ 7.82 (d, 1H, aromatic H), 7.52 (d, 1H, aromatic H), 7.35 (m, 4H, aromatic H), 7.30 (m, 5H, aromatic H), 7.16 (m, 3H, aromatic H), 3.99 (t, 1H, CH), 3.39 (m, 2H, CH₂), 3.24 (m, 2H, CH₂), 2.62 (m, 2H, CH₂), 2.32 (m, 5H, NCH₃+CH₂), 1.78 (m, 2H, CH₂), 1.69 (m, 2H, CH₂), 1.52 (m, 2H, CH₂), 1.15 (m, 4H, 2xCH₂), 1.02 (m, 1 H, CH).

**MS (ES⁺):** 351.2⁺, 527.15⁺ (M+H)⁺

### EXAMPLE 164

### 1-[2-(Cyclobexyl-methyl-amino)-ethyl]-1-(3,3-diphenyl-propyl)-3-(4-phenyl-thiazol-2-yl)-urea

Method D of Example i 62was used to prepare the above product of formula:

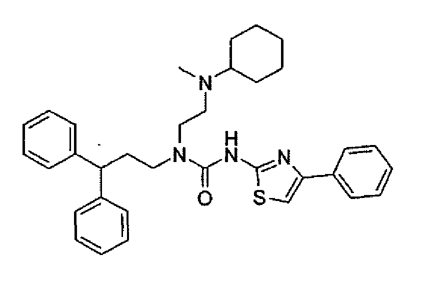

**¹H NMR** (400 MHz, DMSO): δ 7.88 (d, 2H, aromatic H), 7.38 (m, 6H, aromatic H), 7.30 (m, 6H, aromatic H), 7.18 (m, 2H, aromatic H), 3.99 (t, 1H, CH), 3.38 (m, 2H, CH₂), 3.22 (m, 2H, CH₂), 2.65 (m, 2H, CH₂), 2.32 (m, 5H, NCH₃+CH₂), 1.90 (m, 2H, CH₂), 1.55 (m, 2H, CH₂),1.52 (m, 2H, CH₂), 1.20 (m, 4H, 2xCH₂), 1.02 (m, 1H, CH). **MS (ES⁺):** 351.2+, 553.2⁺ (M+H)⁺

### EXAMPLE 165

### 4-(2-[3-Benzothiazol-2-yl-1-(3,3-diphenyl-propyl)-ureido]-ethylcarbamoyl)-piperidine-1-carboxylic acid tert-butyl ester

### Method E: Amide formation

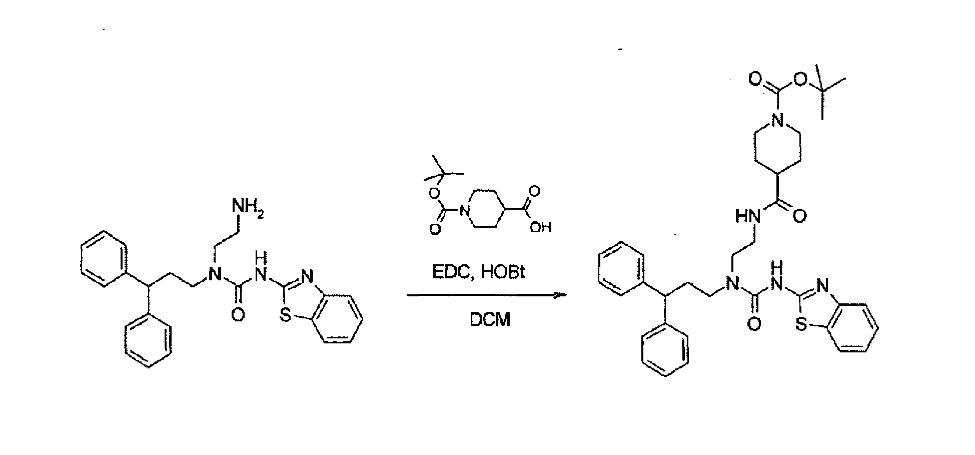

100 mg of 1-(2-amino-ethyl)-3-benzothiazol-2-yl-1-(3,3-diphenyl-propyl)-urea (0.232 mmol, 1 eq) and 58.5 mg of piperidine-1,4-dicarboxylic acid mono-tert-butyl ester (0.255 mmol, 1.1 eq) were dissolved in 5 mL of dry DCM. 34.5 mg of HOBt (0.255 mmol, 1.1 eq) and 49 mg of EDC.HCl (0.255 mmol, 1.1 eq) were added to the solution. The reaction mixture was stirred for 16 h at room temperature, under argon, before addition of water. The aqueous phase was extracted several times with DCM, the organics were washed with brine, dried over MgSO₄, filtered then concentrated. The crude product was purified with flash chromatography over silica gel (DCMI MeOH) to give 121 mg of the desired amide (81% yield).

**¹H NMR** (400 MHz, DMSO): δ 7.85 (m, 2H, NH+aromatic H), 7.53 (m, 1H, aromatic H), 7.35 (m, 5H, aromatic H), 7.30 (m, 4H, aromatic H), 7.18 (m, 3H, aromatic H), 3.99 (t, 1H, CH), 3.82 (m, 2H, CH₂), 3.39 (m, 2H, CH₂), 3.24 (m, 2H, CH₂), 3.i8 (m, 2H, CH₂), 2.60 (m, 2H, CH₂), 2.30 (m, 2H, CH₇), 2.12 (m, it, CH), 1.48 (m, 2H, CH₂), 1.37 (s, 9H, 3xCH₃),1.23 (m, 2H, CH₂).

MS (ES): 466.2⁺, 642.2⁺ (M+H)⁺

### EXAMPLE 166

### Piperidine-4-carboxylic acid {2-[3-benzothiazol-2-yl-1-(3,3-diphenyl-propyl)-ureido]-ethyl}-amide

Method B of Example 160 was used to prepare the above product of formula:

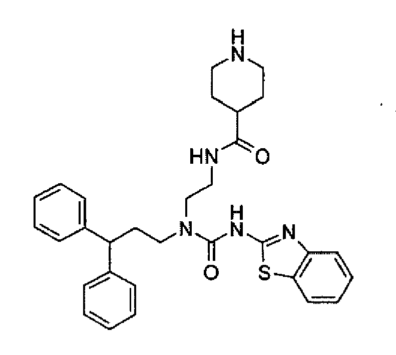

**¹H NMR** (400 MHz, DMSO): δ 7.89 (m, 1H, NH), 7.78 (d, 1H, aromatic H), 7.49 (d, 1H, aromatic H), 7.35 (m, 5H, aromatic H), 7.30 (m, 4H, aromatic H), 7.18 (m, 3H, aromatic H), 3.99 (t, 1H, CH), 3.39 (m, 2H, CH₂), 3.26 (m, 2H, CH₂), 3.18 (m, 2H, CH₂), 3.12 (m, 2H, CH₂),2.42 (m, 2H, CH₂),2.30 (m, 2H, CH₂), 2.09 (m, 1H, CH), 1.49 (m, 2H, CH₂), 1.38 (m, 2H, CH₂).

MS (ES⁺): 366.2⁺, 542.2⁺ (M+H)⁺

### Metitod F: Sulfonamide formation

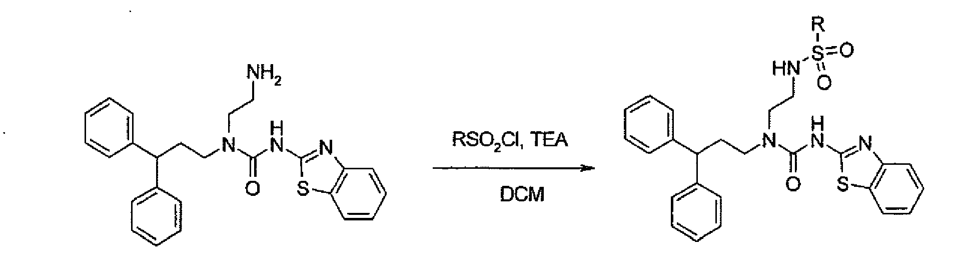

1 eq of 1-(2-Amino-ethyl)-3-benzothiazol-2-yl-l-(3,3-diphenyl-propyl)-urea was diluted in 10 mL of dry DCM. 1.5 eq of triethylamine followed by 1.2 eq of sulfonylchloride derivative were then added to the solution. The reaction mixture was stirred for 5 h at room temperature, under argon, before addition of water. The aqueous phase was extracted several times with DCM, the organics were washed with brine, dried over MgSO₄, filtered then concentrated. The crude product was purified by flash chromatography over silica gel to give the desired sulfonamide derivative.

### EXAMPLE 167

### N-{2-[3-Benzothiazol-2-yl-1-(3,3-diphenyl-propyl)-ureido]-ethyl}-methanesulfonamide

Method F was used to prepare the above product of formula:

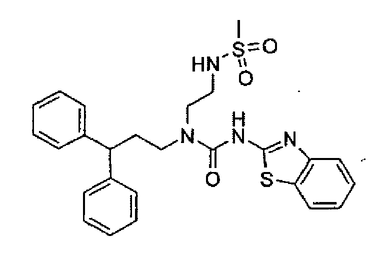

**¹H NMR** (400 MHz, DMSO): δ 7.89 (m, 1H, aromatic H), 7.62 (m, 1H, aromatic H), 7.35 (m, 5H, aromatic H), 7.30 (m, 4H, aromatic H), 7.18 (m, 2H, aromatic H), 7.08 (m, 1H, aromatic H), 4.01 (m, 1H, CH), 3.40 (m, 4H, 2xCH₂), 3.09 (m, 2H, CH₂), 2.86 (s, 3H, CH₃), 2.35 (m, 2H, CH₂).

### EXAMPLE 168

### Propane-1-sulfonic acid 12-13-benzothiazol-2-yl-l-(3,3-diphenyl-propyl)-ureido]-ethyl}-amide

Method F was used to prepare the above product of formula:

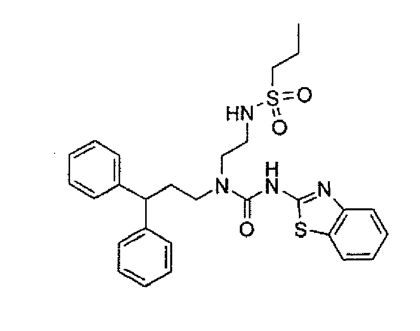

**¹H NMR** (400 MHz, DMSO): δ 7.89 (m, 1H, aromatic H), 7.63 (m, 1H, aromatic H), 7.35 (m, 5H, aromatic H), 7.30 (m, 4H, aromatic H), 7.18 (m, 2H, aromatic H), 7.08 (m, 1H, aromatic H), 4.01 (m, 1H, CH), 3.40 (m, 4H, 2xCH₂), 3.05 (m, 2H, CH₂), 2.92 (m, 2H, CH₂),2.35 (m, 2H, CH₂), 1.62 (m, 2H, CH₂), 0.92 (t, 3H, CH₃).

**MS (ES⁺):** 361.1⁺, 537.1⁺ (M+H)⁺

### EXAMPLE 169

### N-{2-[3-Benzothiazol-2-yl-1-(3,3-diphenyl-propyl)-ureido]-ethyl}-4-cyano-benzenesulfonamide

Method F was used to prepare the above product of formula:

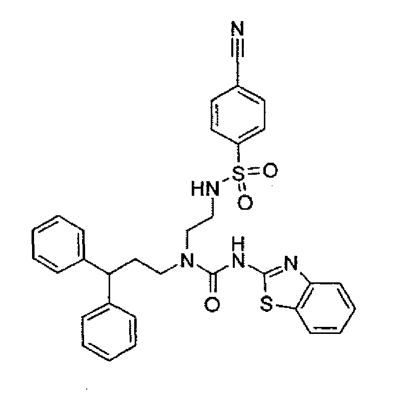

**¹H NMR** (400 MHz, DMSO): δ 8.00 (d, 2H, aromatic H), 7.89 (d, 2H, aromatic H), 7.79 (m, 1H, aromatic H), 7.49 (m, 1H, aromatic H), 7.30 (m, 9H, aromatic H), 7.18 (m, 3H, aromatic H), 3.98 (m, 1H, CH), 3.36 (m, 2H, CH₂), 3.21 (m, 2H, CH₂), 2.91 (m, 2H, CH₂), 2.26 (m, 2H, CH₂).

**MS (ES⁺)**: 420.1⁺, 596.0⁺ (M+H)⁺

### EXAMPLE 170

### N-(4-{2-[3-Benzothiazol-2-yl-1-(3,3-diphenylpropyl)-ureido]-ethylsulfamoyl}-phenyl)-acetamide

Method F was used to prepare the above product of formula:

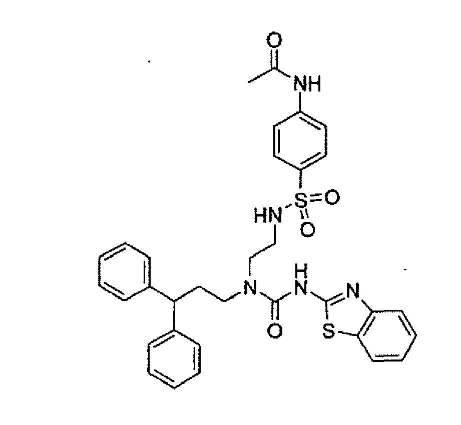

**¹H NMR** (400 MHz, DMSO): δ 7.88 (m, 1H, aromatic H), 7.74 (d, 2H, aromatic H), 7.68 (d, 2H, aromatic H), 7.55 (m, 1H, aromatic H), 7.30 (m, 9H, aromatic H), 7.18 (m, 3H, aromatic H), 3.98 (m, 1H, CH), 3.28 (m, 4H, 2xCH₂),2.82 (m, 2H, CH₂),2.28 (m, 2H, CH₂), 2.08 (s, 3H, CH₃).

**MS (ES⁺):** 452.1⁺,628.1⁺(M+H)⁺

### EXAMPLE 171

### 2-(2-[3-Benzothiazol-2-yl-1-(3,3-diphenyl-propyl)-ureido]-etbylsulfamoyl)-benzoic acid methyl ester

Method F was used to prepare the above product of formula:

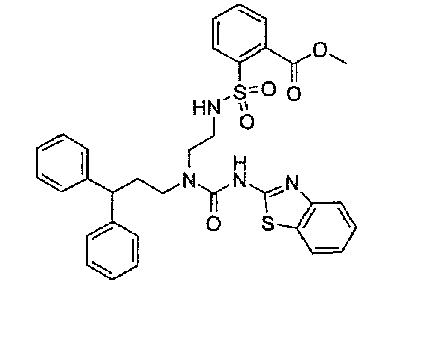

**¹H NMR** (400 MHz, DMSO): δ 8.30 (d, 1H, aromatic H), 8.05 (m, 3H, aromatic H), 7.85 (m, 1H, aromatic H), 7.63 (m, 1H, aromatic H), 7.30 (m, 1H, aromatic H), 7.20 (m, 8H, aromatic H), 7.18 (m, 3H, aromatic H), 3.99 (m, 1H, CH), 3.89 (m, 3H, CH₃), 3.62 (m,2H, CH₂),3.31 (m, 2H, CH₂),3.25 (m, 2H, CH₂),2.26 (m, 2H, CH₂).

**MS (ES⁺):** 453.1⁺, 629.1⁺ (M+H)⁺

### EXAMPLE 172

### N-{2-[3-Benzothiazol-2-yl-1-(3,3-diphenylpropyl)-ureido]-ethyl}-4-methoxy-benzenesulfonamide

Method F was used to prepare the above product of formula:

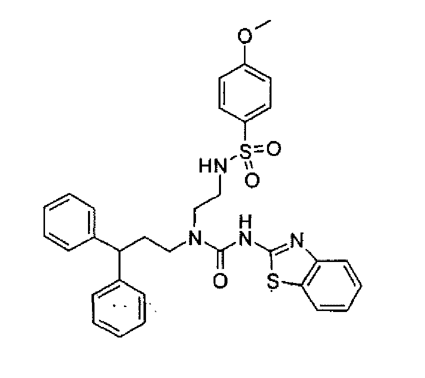

**¹H NMR** (400 MHz, DMSO): δ 7.78 (m, 1H, aromatic H), 7.69 (d, 2H, aromatic H), 7.51 (m, 2H, aromatic H), 7.30 (m, 8H, aromatic H), 7.18 (m, 2H, aromatic H), 7.05 (d, 2H, aromatic H), 3.98 (m, 1H, CH), 3.80 (s, 3H, OCH₃), 3.31 (m, 2H, OCH2), 3.23 (m, 2H, CH₂), 2.81 (m, 2H, CH₂), 2.28 (m, 2H, CH₂).

**MS (ES⁺):** 425.1⁺, 601.1⁺ (M+H)⁺

### EXAMPLE 173

### N-(2-[3-Benzothiazol-2-yl-l-(3,3-diphenylpropyl)-ureido]-ethyl)-4-trifluoromethyl-benzenesulfonamide

Method F was used to prepare the above product of formula:

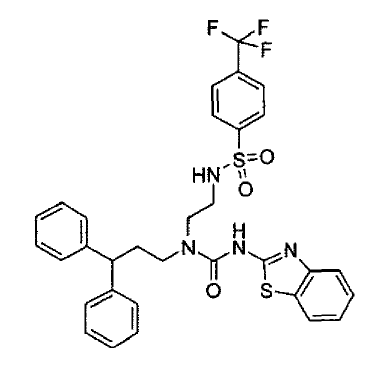

**¹H NMR** (400 MHz, DMSO): δ 7.95 (m, 5H, aromatic H), 7.62 (d, 1H, aromatic H), 7.30 (m, 9H, aromatic H), 7.18 (m, 3H, aromatic H), 3.98 (m, 1H, CH), 3.42 (m, 2H, CH₂), 3.25 (m, 2H, CH₂), 2.90 (m, 2H, CH₂), 2.28 (m, 2H, CH₂).

**MS (ES⁺):** 463.1⁺, 639.05⁺ (M+H)⁺

### EXAMPLE 174

### N, N-dimethylamino-sulfonyl-{2-[3-benzothiazol-2-yl-1-(3,3-diphenyl-propyl)-ureido]-ethyl}-amide

Method F was used to prepare the above product of formula:

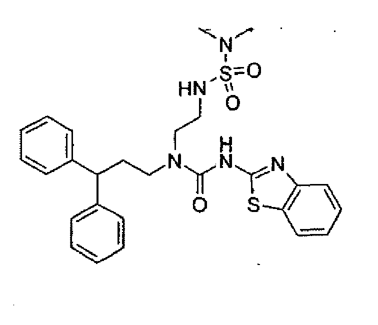

**¹H NMR** (400 MHz, DMSO): δ 7.89 (m, 1H, aromatic H), 7.62 (m, 1H, aromatic H), 7.35 (m, 4H, aromatic H), 7.29 (m, 4H, aromatic H), 7.18 (m, 4H, aromatic H), 3.98 (m, 1H, CH), 3.31 (m, 4H, 2xCH₂), 3.02 (m, 2H, CH₂), 2.60 (m, 6H, N(CH₃)₂), 2.32 (m, 2H, CH₂). **MS (ES⁺)**: 362.1⁺, 538.1⁺ (M+H)⁺

### EXAMPLE 175

### 3-Benzotbiazol-2-yl-1-(3_{J}-diphenylpropyl)-1-(2-bydroxy-etbyl)-urea

### Method G:

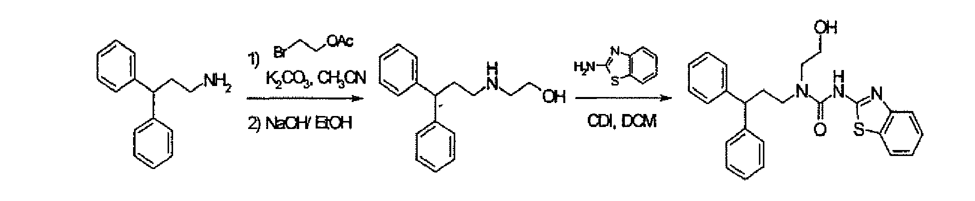

10 g of 3,3-diphenylpropylamine (47.4 mmol, 1 eq) were diluted in 200 mL of CH₃CN at room temperature, under argon. 9.8 g of K₂CO₃ (71.08 mmol, 1.5 eq) then 7.9 g of acetic acid 2-bromo-ethyl ester (47.4 mmol, 1 eq) were added slowly. The mixture was heated to reflux and stirred for 4 h. The insoluble was filtered and washed several times with EtOAc. The filtrate was then concentrated and purified by flash chromatography over silica gel (DCM/ MeOH: 95/5) to give 6 g of the desired secondary amine, acetic acid 2-(3,3-diphenyl-propylamino)-ethyl ester, which was submitted to the next step.

6 g of acetic acid 2-(3,3-diphenyl-propylamino)-ethyl ester were (20 mmol, 1 eq) solubilized in 100 mL of EtOH, then 20 mL of 2 N NaOH solution (40.4 mmol, 2 eq) were added to the solution. The mixture was stirred for 2 h at room temperature before adding 1 N HCl solution until pH = 7, and evaporating EtOH. The aqueous phase was extracted with EtOAc, then the organic phases were washed with water, brine, dried over MgSO₄, filtered and concentrated. The crude product, 2-(3,3-diphenyl-propylamino)-ethanol, was submitted to the next step without any purification.

396 mg of carbonyl diimidazole (2.44 mmol, 1.5 eq) were dissolved in 4 mL of DCMthen 245 mg (1.63 mmol, 1 eq) of 2-aminobenzothiazole were added to the solution. A white precipitate appeared. The suspension was stirred for 15 hours at room temperature. 500 mg (1.96 mmol, 1.2 eq) of 2-(3,3-diphenyl-propylamino)-ethanol in DCM were then added. The solution, which has become clear again, was stirred for 5 hours at room temperature. A sodium bicarbonate solution was added and the aqueous phase was extracted with dichloromethane. The organic phase was washed with brine, dried over MgSO₄ filtered and concentrated. The crude product was subjected to chromatography over silica gel (EtOAc/ MeOH: 95/5) to obtain 500 mg of the desired urea (71% yield).

**¹H NMR** (400 MHz, DMSO): δ 7.82 (m, 1H, aromatic H), 7.55 (m, 1H, aromatic H), 7.35 5 (m, 5H, aromatic H), 7.29 (m, 4H, aromatic H), 7.18 (m, 3H, aromatic H), 3.99 (m, 1H, CH), 3.52 (m, 2H, CH₂), 3.41 (m, 2H, CH₂), 3.30 (m, 2H, CH₂), 2.32 (m, 2H, CH₂).

MS (ES⁺): 256.1⁺, 432.1⁺ (M+H)⁺_{.}

### EXAMPLE 176

### 1-(5-cbloro-4-(4-(methylsulfonamido)phenyl)thiazol-2-yl)-3-(3,3-diphenylpropyl)urea-(3)

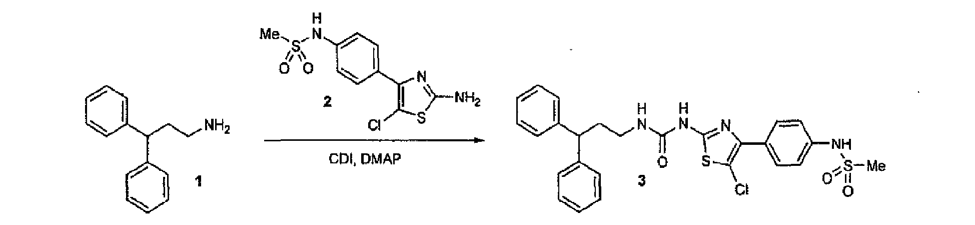

To a mixture of N-(4-(2-amino-5-chlorothiazol-4-yl)phenyl)methanesulfonamide **2** (0.103 g, 0.34 mmol), DMAP (0.056 g, 0.46 mmol), and CDI (0.085 g, 0.52 mmol) was added DMF (0.5 mL). The reaction mixture was heated to 40 °C for 20 h and 3,3-diphenylpropan-1-amine **1** (0.072 g, 0.34 mmol) was added. The reaction mixture was heated to 40 °C for 2 d. Direct purification by flash column chromatography on silica gel (eluted with 30% to 70% EtOAc in hexanes) gave 1-(5-chloro-4-(4-(methylsulfonamido)-phenyl)thiazol-2-yl)-3-(3,3-diphenylpropyl)urea 3 (0.047 g, 25% yield) as a white solid. ¹H NMR (400 MHz, CDCl₃) δ 2.23 (q, *J* = 6.8 Hz, 2H), 3.04 (q, *J* = 6.6 Hz, 2H), 3.04 (s, 3H), 3.99 (t, *J* =*.* 7.6 Hz, 1H), 6.58 (s br, 1H), 7.17 (t, *J* = 5.5 Hz, 2H), 7.27-7.34 (m, 10H), 7.84 (d, *J* = 8.8 Hz, 2H), 9.95 (s br, 1H), 10.81 (s br, 1H).

Mass spectrum: calculated for C₂₆H₂₅CIN₄O₃S₂ 540.1; found 541.2 (M++ 1)

### EXAMPLE 177

### In vitro Measurements

The activities of the compounds of the present invention on calcium receptors were measured in accordance with the method described hereinbelow.

Human Ca²⁺ receptor cDNA was subcloned into the mammalian expression vector PECE as described in Ellis, L et al. (1986) Cell vol. 45, 721-732. The luciferase reporter was subcloned into the mammalian expression vector pGL3basic (Promega). Resistance to neomycin (pSV2-neo) and resistance to puromycin (pSG5-puro) were used as selection markers. All these plasmids were simultaneously transfected into CHO cells by calcium phosphate precipitation. Transfected cells were grown in F12 medium containing 7.5% foetal bovine serum, 100U/ml penicillin and 100 ug/ml streptomycin (as 1% Pen-Strep, BioWithaker), neomycin (750)µg/ml) and puromycin (5µg/ml). Neomycin and puromycin resistant colonies were subcloned and assayed for activation against a range of calcium concentration. Clone 8-5-5 was used to assess the effects of compounds on [Ca²⁺]_{¡}. This stably transfected cell line was termed ET8-5-5.

For measurements of [Ca²⁺]ᵢ, the cells were recovered from tissue culture flasks by brief treatment with Trypsin-EDTA (Invitrogen; containing 0.53mM EDTA•4Na in HBSS) and then seeded in culture-treated 96-well plates (Greiner) at SOk cells per well in the growth media (same as above, except neomycin 400µg/ml). Cells were grown in 37°C TC incubator for 24h. The culture medium was then removed and replaced with F12 medium, 1% Pen-Strep for an overnight foetal bovine serum starvation in 37°C TC incubator. Then the starvation medium was removed and replaced with a test buffer (20 mM HEPES pH 7.4, 125 mM NaCI, 5 mM KCl, 1 mM MgCl₂, 5.5 mM Glucose, 2g/l lysosyme and 0.3 mM CaCl₂) supplemented with a range of test compound concentrations crossed against a super-added range of eaCh concentrations. The cells were incubated with the test compounds for 5h in 37°C TC incubator. Then the test buffer was discarded, and cells were added with the substrate for Luciferase from SteadyLite Kit (Perkin-Elmer). The luminescence was recorded.

The compounds of Examples 1 to 176 were tested according to this procedure described above and all were found to have an EC₅₀ of 10 µM or less. Examples of those activities were shown in Table 1.

**Table 1**

| **Example** | **Activity*** |
|---|---|
| **63** | ++ |
| **64** | +++ |
| **65** | + |
| **66** | + |
| **171** | ++ |
| **172** | ++ |
| **173** | ++ |

| | |
|---|---|
| * EC50 < 00nM = +++; 100nM < EC50 < 1µM = ++; 1µM < EC50 < 10µM = + | |

### EXAMPLE 178

### In vitro Measurements

The activities of the compounds of the present invention on calcium receptors were measured. In one embodiment, the measurement was performed in accordance with the method described in Example 4 ofNemeth et al., PCT/US95/13704 (International Publication No. WO96/12697) herein incorporated by reference.

A 4.0-kb *Not*l-HindIII fragment of the human parathyroid cell Ca²⁺ receptor (hPCaR) cDNA was subcloned into the mammalian expression vector pCEP4 (Invitrogen) containing the hygromycin-resistant gene as a selectable marker. This plasmid was transfected into HEK 293 cells by calcium phosphate precipitation. Transfected cells were grown in Dulbecco's modified Eagle's medium containing 10% fetal bovine serum and hygromycin (200 µg/mL). Hygromycin-resistant colonies were subcloned and assayed for hPCaR mRNA by solution hybridization using a ³²P-labeled RNA probe complementary to the (4.0 kb) hPCaR sequence (Garrett, et al., J. Biol. Chem. 270, I29I9-12925 (1995)). Clone 7 was used to assess the effects of compounds on [Ca²⁺]_{¡}. This stably transfected cell line was termed HEK 293 4.0-7. For measurements of [Ca²⁺]ᵢ, the cells were recovered from tissue culture flasks by brief treatment with Versene (Invitrogen; Containing 0.2 gIL EDTA•4Na in phosphate-buffered saline) and then seeded in collagen coated 384-well plates (BD Biosciences) at 20K cells per well in the growth media (same as above). Cells were grown in 37c TC incubator overnight. Then, the media was discarded and cells were loaded with 1x dye from Ca2+ Assay Kit I (BD Biosciences) in parathyroid cell buffer (126mM NaCl, 4mM KCl, 1mM MgSO₄, 0.7mM K₂HPO₄/KH₂PO₄, 20mM HEPES·NaOH (pH 7.45)) containing 0.5% BSA and 1mM CaCl₂. Cells were loaded at room temperature for 90 minutes. Each test compound was added to the cells and the fluorescence was recorded by using excitation and emission wavelengths of 485 and 530 nm, respectively.

The compounds of Examples 67 to 175 were tested according to this procedure described and were shown in Table 2

**Table 2**

| **Example** | **Activity*** | **Example** | **Activity*** | **Example** | **Activity*** |
|---|---|---|---|---|---|
| **67** | +++ | **103** | ++ | **138** | +++ |
| **68** | +++ | **104** | ++ | **139** | +++ |
| **69** | +++ | **105** | ++ | **140** | +++ |
| **70** | +++ | **106** | ++ | **141** | +++ |
| **71** | +++ | **107** | ++ | **142** | +++ |
| **72** | +++ | **108** | ++ | **143** | +++ |
| **73** | +++ | **109** | ++ | **144** | +++ |
| **74** | +++ | **110** | ++ | **145** | +++ |
| **75** | +++ | **111** | ++ | **146** | +++ |
| **76** | +++ | **112** | ++ | **147** | +++ |
| **77** | +++ | **113** | ++ | **148** | ++ |
| **78** | +++ | **114** | ++ | **149** | ++ |
| **79** | +++ | **115** | ++ | **150** | + |
| **80** | +++ | **116** | ++ | **151** | ++ |
| **81** | +++ | **117** | ++ | **152** | + |
| **82** | +++ | **118** | ++ | **153** | ++ |
| **83** | +++ | **119** | ++ | **154** | ++ |
| **84** | +++ | **120** | + | **155** | + |
| **85** | +++ | **121** | + | **156** | + |
| **86** | +++ | **122** | + | **157** | +++ |
| **87** | +++ | **123** | + | **158** | ++ |
| **89** | +++ | **124** | +++ | **159** | ++ |
| **90** | ++ | **125** | + | **160** | +++ |
| **91** | ++ | **126** | + | **161** | ++ |
| **92** | ++ | **127** | +++ | **162** | +++ |
| **93** | ++ | **128** | +++ | **163** | ++ |
| **94** | ++ | **129** | +++ | **164** | ++ |
| **95** | ++ | **130** | +++ | **165** | ++ |
| **96** | ++ | **131** | +++ | **166** | +++ |
| **97** | ++ | **132** | +++ | **167** | ++ |
| **98** | ++ | **133** | +++ | **168** | + |
| **99** | ++ | **134** | +++ | **169** | + |
| **100** | ++ | **135** | +++ | **170** | ++ |
| **101** | ++ | **136** | +++ | **174** | ++ |
| **102** | ++ | **137** | +++ | **175** | ++ |
| | | | | **176** | ++ |

| | | | | | |
|---|---|---|---|---|---|
| *EC50 <100nM = +++; 100nM < EC50 < 1 >M = ++; 1µM < EC50 < 5 >M = + | | | | | |

### EXAMPLE 17 9

### In vivo Measurements

Male Sprague-Dawley rats weighing 250-400g were given free access to food and water. Unanesthetized rats were gavaged with an 18-gauge balled needle at a volume between 0.5 and 1ml. Compounds were formulated in 20% captisol in water at pH 7.0 or 2% hydroxypropyl methylcellulose (HPMC)/1% Tween 80/5% Captisol in water pH 2.0. Calcimimetics were administered at various doses covering the following range 0.03-30 mg/kg in 20% captisol. Vehicle-treated rats received one of the above two vehicles at the maximum volume (0.5-lml) used for the calcimimetics. Each rat was bled at time 0 (pre-calcimimetic or vehicle administration) and at various times (l, 2, 4, 8 and 24 h) after oral gavage of calcimimetic or vehicle.

For measurements of blood-ionized Ca²⁺ levels, blood (50 µl) was collected from the orbital sinus of anesthetized rats (3% isoflurane in O₂) with heparinized capillary tubes. Blood samples were analyzed within seconds of collection using a Rapidlab 348 Blood Gas Analyzer (Bayer HealthCare LLC Diagnostic Division; Tarrytown, NY).

For measurements of serum PTH, phosphorus, a nonheparinized capillary tube was inserted into the orbital sinus and blood (0.5 ml) was collected into SST (clot activator) brand blood tubes. Blood samples were allowed to clot for 15-30 min and centrifuged (3000 rpm; Sorvall RT 600B) at 4°C. Serum was removed and stored below 0°C until assayed. Serum PTH levels were quantified according to the vendor's instructions using rat PTH immunoradiometric assay kits (Immutopics, San Clement, CA) or rat bioactive intact PTH elisa kit (Immutopics, San Clemente, CA). Serum phosphorus levels were determined using a blood chemistry analyzer (AU 400; Olympus, Melville, NY).

## Claims

1. A compound of formula (1): 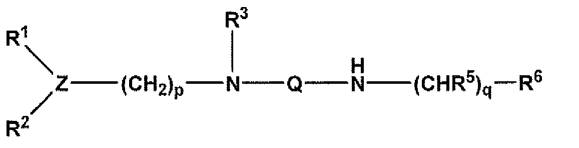 wherein:
Z is >CH-, >C=CH- or >N-,
R¹ and R² are the same or different, and each represents an aryl group, a heteroaryl group, or Z, R¹ and R² form a fused ring structure of formula: 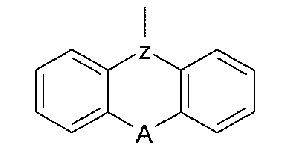 in which A represents a single bond, a methylene group, a dimethylene group, oxygen, nitrogen or sulphur, said sulphur optionally being in the sulphoxide or sulphone forms,
wherein each of R¹ and R², or said fused ring structure formed thereby, is optionally substituted by at least one substituent selected from the group ***c***
wherein the group c consists of: halogen atoms, hydroxyl, carboxyl, linear and branched alkyl, hydroxyalkyl, haloalkyl, alkylthio, alkenyl, and alkynyl groups; linear and branched alkoxyl groups; linear and branched thioalkyl groups; hydroxycarbonylalkyl; alkylcarbonyl; alkoxycarbonylalkyl; alkoxycarbonyl; trifluoromethyl; trifluoromethoxyl; -CN; -NO₂; sulphonamido groups; alkylsulphonyl groups optionally in the sulphoxide or sulphone forms; wherein any alkyl component has from 1 to 6 carbon atoms, and any alkenyl or alkynyl components have from 2 to 6 carbon atoms,
and wherein, when there is more than one substituent, then each said substituent is the same or different,
R³ represents -AlkNR⁷R⁸, wherein Alk is a straight or branched chain C₁₋₆ alkylene group,
R⁷ and R⁸, which may be the same or different, each represents: a hydrogen atom; an alkylsulphonyl group; alkylamino- or dialkylamino- sulphonyl; a linear or branched alkyl group containing from 1 to 6 carbon atoms and optionally substituted by at least one of a phenyl group, a halogen atom, a hydroxyl group, a carboxyl group, an alkoxycarbonyl group, or an alkoxy group containing from 1 to 6 carbon atoms; an alkylaminoalkyl or dialkylaminoalkyl group wherein each alkyl group contains from 1 to 6 carbon atoms; an amino group; an aminocarbonyl group; an alkylamino group; a saturated or unsaturated cycle, optionally spaced from the N to which the cycle is linked by an ―SO₂- group, -C(O)-group, -C(O)O- group, -C(O)NH- group or an Alk group as defined, and containing 0, l, 2, or 3 heteroatoms and having 5, 6, or 7 ring atoms, or 3-7 ring atoms when the cycle is a carbocycle, said cycle being optionally substituted by at least one substituent selected from the group **'*b*'** defined below,
and wherein, when there is more than one substituent, said substituent is the same or different,
Q represents >C=O or >C=S,
R⁵ represents a hydrogen atom or an alkyl, alkoxy, hydroxyalkyl, alkylthio, or thioalkyl group wherein any alkyl part contains from 1 to 4 carbon atoms,
p is l,2or3,
_{d}is0, 1 or 2,
R⁶ represents an aryl or heteroaryl ring, two linked rings each being selected from aryl or heteroaryl rings, or a fused double or triple ring system comprising at least two rings each being selected from aryl or heteroaryl rings, and wherein said ring or rings forming R⁶ are optionally substituted by at least one substituent selected from the group ***a***,
wherein the group a consists of: halogen atoms; hydroxyl; carboxyl; aldehyde groups; aryl groups; linear and branched alkyl, alkenyl, alkynyl, hydroxyalkyl, hydroxyalkenyl, hydroxyalkynyl, haloalkyl, haloalkenyl, and haloalkynyl groups; linear and branched alkoxyl groups; linear and branched thioalkyl groups; heteroaryl groups; saturated or unsaturated heterocycyl groups; aralkoxy groups; aryloxy groups; alkoxycarbonyl; aralkoxycarbonyl; aryloxycarbonyl; heteroaralkoxy groups; heteroaryloxy groups; heteroaralkoxycarbonyl; heteroaryloxycarbonyl; hydroxycarbonylalkyl; alkoxycarbonylalkyl;
aralkoxycarbonylalkyl; aryloxycarbonylalkyl; heteroaralkoxycarbonylalkyl; heteroaryloxycarbonylalkyl; perfluoroalkyl; perfluoroallcoxy; -CN; -NO₂; acyl; amino, alkylamino, aralkylamino, arylamino, dialkylamino, diaralkylamino, diarylamino, heteroaralkylamino, heteroarylamino, diheteroaralkylamino, diheteroarylamino, alkylsulphonylamino, haloalkylsulphonylamino, acylamino, and diacylamino groups; alkoxycarbonylamino, aralkoxycarbonylamino, aryloxycarbonylamino, heteroaralkoxycarbonylamino, heteroaryloxycarbonylamino, alkylcarbonylamino, heteroaralkylearbonyl amino, heteroarylcarbonylamino, alkylanninocarbonyloxy, aralkylaminocarbonyloxy, and arylaminocarbonyloxy groups; alkyl groups substituted with an amino, alkylamino, aminoalkylamino, alkylaminoalkylamino, aralkylamino, arylamino, aryloxy, arylthio, heteroaralkylamino, heteroarylamino, heteroaryloxy, heteroarylthio, heterocycyloxy, heterocycylthio, dialkylamino, diaralkylamino, diarylamino, diheteroaralkylamino, diheteroarylamino, acylamino, trifluoromethylcarbonylamino, fluoroalkyl-carbonylamino, diacylamino group; a carbamoyl group optionally substituted by an alkyl, alkylsulphonamide, sulphonamide, alkylsulphonyl, sulphonyl, aminoalkyl, or alkylaminoalkyl group; a sulphonamide group optionally substituted by an alkyl, acyl, alkoxycarbonyl, carbamoyl, alkylcarbamoyl, or carbamoyl further substituted by a carboxylic acid, aminoalkyl, or alkylaminoalkyl group; alkyl-, aralkyl-, aryl- heteroaralkyl-, and heteroaryl- amido groups; alkylthio, arylthio, aralkylthio, heteroarylthio and heteroaralkylthio and the oxidised sulphoxide and sulphone forms thereof; sulphonyl, alkylsulphonyl, haloalkylsulphonyl, arylsulphonyl, aralkylsulphonyl, and heteroaralkylsulphonyl groups; alkylsulphonamide, haloalkylsulphonamide, di(alkylsulphonyl)amino, aralkylsulphonamide, di(aralkylsulphonyl)amino, arylsulphonamide, di(arylsulphonyl)amino, heteroaralkylsulphonamide, di(heteroaralkylsulphonyl)amino, heteroarylsulphonamide, and di(heteroarylsulphonyl)amino; and saturated and unsaturated heterocyclyl groups, said aryl, heteroaryl and heterocyclyl groups being mono- or bi- cyclic and being optionally substituted by one or more substituents, which may be the same or different, selected from the group ***b***,
wherein the group ***b*** consists of: keto when substituting a saturated or
partially unsaturated heterocycle, halogen atoms; hydroxyl; carboxyl; aldehyde groups; linear and branched alkyl, alkenyl, alkynyl, hydroxyalkyl, hydroxyalkenyl, hydroxyalkynyl, haloalkyl, haloalkenyl, and haloalkynyl groups; linear and branched alkoxyl groups; linear and branched thioalkyl groups; alkoxycarbonyl; hydroxycarbonylalkyl; alkoxycarbonylalkyl; perfluoroalkyl; perfluoroalkoxy; -CN; acyl; amino, alkylamino, dialkylamino, acylamino, and diacylamino groups; alkyl groups substituted with an amino, alkylamino, dialkylamino, acylamino, or diacylamino group; CONH₂; alkylamido groups; alkylthio and the oxidised sulphoxide and sulphone forms thereof; sulphonyl, alkylsulphonyl groups; and sulphonamide, alkylsulphonamide, and di(alkylsulphonyl)amino groups
wherein two groups a, where present, optionally form a fused carbocycle or heterocycle with the ring on which they are located, and are optionally substituted with a keto or a substituent selected from group ***b***, as defined, wherein, in groups ***a*** and ***b***, any alkyl components contain from 1 to 6 carbon atoms, and any alkenyl or alkynyl components contain from 2 to 6 carbon atoms, and are optionally substituted by at least one halogen atom or hydroxyl group, and wherein any aryl component is optionally a heteroaryl group,
and salts and esters thereof.

2. A compound according to claim 1, wherein each of R¹ and R², or said fused ring structure formed thereby, is optionally substituted by at least one substituent selected from the group consisting of: fluorine and chlorine atoms, hydroxyl groups, linear or branched alkoxy groups containing from 1 to 5 carbon atoms, linear or branched alkyl groups containing from 1 to 5 carbon atoms, trifluoromethyl and trifluoromethoxy groups, and - CN groups,
and wherein, when there is more than one substituent, then each said substituent is the same or different.

3. A compound according to any preceding claim, wherein each R¹ and R² is substituted with a substituent selected from: hydrogen; chlorine atoms; hydroxyl groups; carboxyl groups; linear and branched alkyl and hydroxyalkyl, groups; linear and branched alkoxyl groups; alkoxycarbonyl groups; hydroxycarbonylalkyl groups; alkoxycarbonylalkyl groups; trifluoromethyl groups; trifluoromethoxy groups; -CN groups; alkylthio groups; alkylsulphonyl groups; and sulphonamide groups.

4. A compound according to claim 1, wherein R¹ and R² are each phenyl.

5. A compound according to any preceding claim, wherein R₆ is a monocyclic aryl or a 5 or 6 membered heteroaryl ring.

6. A compound according to claim 4, wherein R⁶ is selected from the group consisting of: phenyl, naphthyl, benzothiazolyl, fluorenyl, benzazolyl, benzoxazolyl, thienyl, thiazolyl, isothiazolyl, furyl, oxazolyl, isoxazolyl, imidazolyl, triazolyl, indolyl, pyrrolyl, quinolyl, pyridinyl, pyrazolyl, pyrazinyl, pyrimidinyl, pyridazinyl, pyrrolyl, furanyl, 1,2,3-triazolyl, 1,2,4-triazolyl, tetrazolyl, 1,2,4-triazinyl, 1,3,5-triazinyl, benzofuranyl, benzothiazyl, benzimidazolyl, indazolyl, tetraquinolinyl, tetrahydroisoquinolinyl, isoquinolinyl, indolyl, carbazolyl, indolinyl, alpha- or beta-carbolinyl, and benzothienyl groups.

7. A compound according to any of claims 1 to 6, wherein one of R⁷ and R⁸ represents a hydrogen atom or a methyl group, and the other represents an optionally substituted cycle.

8. A compound according to claim 7, wherein the cycle is a six-membered cycle.

9. A compound according to claim 8, wherein the cycle is cyclohexyl or phenyl.

10. A compound according to claim 8, wherein the cycle is piperidinyl or piperazinyl.

11. A compound according to claim 7, wherein the cycle is a five-membered cycle.

12. A compound according to any of claims 1 to 6, wherein one oft7 and R⁸ represents a hydrogen atom and the other represents a hydrogen atom or an optionally substituted alkyl group.

13. A compound according to claim 12, wherein one of R⁷ and R⁸ represents an alkyl group substituted by one or two substituents selected from: alkoxy, carboxyl, amino, alkylamino, dialkylamino, and aromatic groups.

14. A compound according to any preceding claim, wherein Z is >CH-.

15. A compound according to any preceding claim, wherein q is 0.

16. A compound according to any preceding claim, wherein Q is >C=O.

17. A compound as defined in any preceding claim for use in therapy.

18. A pharmaceutically acceptable composition comprising a compound as defined in any of claims 1 to16.

19. A compound as defined in any of claims 1 to 16 for use in the prevention or treatment of cancers neurodegenerative diseases, bone and articular metabolism diseases, abnormal calcium homeostasis, hyperplasia and parathyroid adenoma, intestinal malabsorption, biliary lithiasis and renal lithiasis, hyperparathyroidism" ionised serum calcium level reduction during the treatment of hypercalcaemia, and cardiovascular diseases.
